# EUROPEAN PATENT APPLICATION

(11) **EP 1 277 473 A1**
(43) Date of publication of application: **22.01.2003**
(21) Application number: 01919858.9
(22) Date of filing: 10.04.2001
(51) Int. Cl.: A61K 35/78, A61K 31/216, A61K 31/7048, A61K 31/192, A61K 31/12, A61P 43/00, A61P 25/00, A23L 2/00, A23L 1/30, A23K 1/16

(54) **REMEDIES**

(30) Priority: 10.04.2000 JP 2000108602; 06.10.2000 JP 2000308522; 26.01.2001 JP 2000019167
(71) Applicant: TAKARA BIO INC., Otsu-shi, Shiga 520-2193 (JP)
(72) Inventor: OHNOGI, Hiromu, Muko-shi Kyoto 617-0002 (JP); SHIRAGA, Masahiro, Otsu-shi Shiga 520-2134 (JP); KOBAYASHI, Eiji, Otsu-shi, Shiga 520-2153 (JP); NISHIMURA, Kaori, Otsu-shi Shiga 520-0853 (JP); LI, Tuo-Ping c/o Central Research Laboratories, Otsu-shi, Shiga 520-2193 (JP); SAGAWA, Hiroaki, Kusatsu-shi Shiga 525-0025 (JP); KATO, Ikunoshin, Uji-shi Kyoto 611-0028 (JP)
(74) Representative: VOSSIUS & PARTNER
(86) International application number: JP0103074
(87) International publication number: WO01076614

(57) **Abstract**

The present invention provides a composition for enhancing growth factor production, comprising a plant-derived enhancer for growth factor production; a therapeutic agent or prophylactic agent for a disease requiring enhancement of growth factor production and a food, beverage or feed for enhancing growth factor production, each comprising the above composition. In addition, the present invention relates to a functional food, beverage or feed excellent in health enhancement.

## Description

### TECHNICAL FIELD

The present invention relates to a composition for enhancing growth factor production comprising a plant-derived enhancer for growth factor production; a therapeutic agent or prophylactic agent for a disease requiring enhancement of growth factor production, comprising the composition; and a food, beverage or feed for enhancing growth factor production. In addition, the present invention relates to a functional food, beverage or feed excellent in health enhancement.

### BACKGROUND ART

As pharmacological actions owned by a plant belonging to Umbelliferae such as *Angelica keiskei* koidz., prophylactic effect for hypertension, antibacterial action, anti-ulcerative action, suppressive action for gastric acid secretion, anti-cancerous effect, prophylactic effect for cancer and the like have been known.

As pharmacological actions owned by a plant belonging to Compositae such as *Carthamus tinctorius,* anti-inflammatory activity, anti-edematous effect, anti-bacterial activity and the like have been known.

As pharmacological actions owned by a plant belonging to Liliaceae such as onion, action for lowering cholesterol, antibacterial action, anti-fungal action, action for lowering blood sugar level, anti-asthmatic action, anti-inflammatory action, inhibitory action for aldose reductase and the like have been known.

As pharmacological actions owned by a plant belonging to Ginkgoaceae such as *Ginkgo biloba*, action for accelerating blood circulation in the brain, effect for disappearance of blotch, suppressive action for platelet coagulation, action for lowering serum cholesterol and the like have been known.

As pharmacological actions owned by a plant belonging to Gramineae such as bamboo, antibacterial action, anti-fungal action, action for lowering blood pressure, antioxidating activity and the like have been known. Also, rice bran has been known for its action for lowering cholesterol.

As pharmacological actions owned by a plant belonging to Rosaceae such as rose, anti-inflammatory action, anti-allergic action, suppressive action for cancer cell proliferation, antibacterial action, and the like have been known. Also, plum has been known for its action for lowering cholesterol and prophylactic action for diabetes.

As pharmacological actions owned by a plant belonging to Moraceae such as *Humulus lupulus*, antibacterial action, anti-tumor action, sedative action and the like have been known.

As pharmacological actions owned by a plant belonging to Zingiberaceae such as *Curcuma zedoaeia Roscoe*, antibacterial action, secretory action of bile acids, anti-ulcerative activity, anti-tumor activity and the like have been known.

As pharmacological actions owned by a plant belonging to Leguminosae such as soybean, action for lowering cholesterol, antioxidating action and the like have been known.

As pharmacological actions owned by a plant belonging to Tiliaceae such as mulukhiya, action for lowering blood pressure, immunopotentiaing action and the like have been known.

As pharmacological actions owned by a plant belonging to Cruciferae such as broccoli, anti-cancerous effect has been known.

However, enhancing action for growth factor production owned by these plant components, especially enhancing action for hepatocyte growth factor (HGF) production and enhancing action for nerve growth factor (NGF) production, has not yet been known.

By the way, a liver subjected to partial hepatectomy quickly regenerates and regains its original size. Although the substance of the factor for hepatic regeneration has been unknown for many years, hepatocyte growth factor (HGF) has been found in plasma of a patient suffering from fulminant hepatitis, and isolated and purified from the plasma of this patient (Gohda, E. et al.: *J. Clin. Invest.,* **88** 414-419, 1988). Further, human HGF cDNA has been also cloned, and the primary structure for HGF has been also elucidated (Miyakawa, K. et al.: *Biochem. Biophys. Res. Commun.*, **163** 967-973, 1989). In addition, it has been elucidated that scatter factor (SF) for facilitating motility of cells, and a tumor cell disorder factor, tumor cytotoxic factor (TCF) are identical substances to HGF (Weidner, K. M. et al.: *Proc. Natl. Acad. Sci. USA*, **88** 7001-7005, 1991; Shima, N. et al.: *Biochem. Biophys. Res. Commun.*, **180** 1151*-1158,1991).*

HGF accelerates growth of many of epithelial cells, such as changioepithelial cells, renal tubule epithelial cells, and gastric mucosa cells, as well as hepatocytes, and induces morphological formations as seen in facilitation of motility of epithelial cells, vascularization or luminal formation of epithelial cells, so that HGF is a multi-functional active substance exhibiting a wide variety of physiological activity. In other words, in various organs, HGF induces morphological formations such as proliferation acceleration and facilitation of motility of epithelial cells, or vascularization during the recovery of the disorder of the organ, and the like.

HGF exhibits growing action for hepatocytes, accelerating action for protein synthesis, ameliorating action for cholestasia, and further preventing action for renal disorder caused by drugs and the like. mRNA of HGF is synthesized even in the brain, the kidney, the lungs, and the like. HGF is a mesoblast growth factor that accelerates growth of many of epithelial cells, such as changioepithelial cells, renal tubule epithelial cells, and gastric mucosa cells. In addition, it induces morphological formations such as proliferation acceleration and facilitation of motility of epithelial cells, or vascularization during the recovery of the disorder, so that HGF is a multi-functional active substance exhibiting a wide variety of physiological activity. Further, HGF also has actions for protection, proliferation acceleration, and recovery of disorder of nerve cells, and the like. Therefore, by enhancing the production of HGF, it has been expected to treat or prevent hepatic disorders such as hepatitis, severe hepatitis, fulminant hepatitis, cirrhosis, and cholestasia in the liver, renal disorders caused by drugs and the like, gastrointestinal disorders, vascular disorders, chronic nephritis, pneumonia, wound, diabetes, cancer, and the like.

Since HGF has the various actions mentioned above, the HGF itself is expected to be used as a therapeutic agent for hepatic disorders such as hepatitis, severe hepatitis, fulminant hepatitis, cirrhosis, and cholestasia in the liver, renal disorders caused by drugs and the like, gastrointestinal disorders, vascular disorders, chronic nephritis, pneumonia, wound, diabetes, cancer, and the like. However, the HGF itself has not yet been used as a therapeutic agent for actual use. Further, although a method of introducing a gene of HGF by gene therapy has been tried, it is far from being actually used because of adverse actions resulting from HGF actions caused in an unnecessary timing and location. As described above, if HGF can be arbitrarily enhanced without being externally administered, HGF is thought to be effective for treatment and prophylaxis of a disease requiring enhancement for HGF production. However, this has not yet been actually used.

Nerve cells play a principal role for sustaining psychoactivities of human being such as intellectual functions, memory, emotions and behaviors. It has been thought that the differentiation, survival and exhibition of functions of the nerve cells which are the foundations of these psychoactivities need a neurotrophic factor specific for each nerve cell. Among the neurotrophic factors, one of which existence and function have been firstly elucidated is a nerve growth factor (hereinafter simply referred to as "NGF"), and currently, there have been found a brain-derived-neurotrophic factor, neurotrophin-3, neurotrophin-4/5, and the like.

NGF is a neurotrophic factor of a large cellular cholinergic nerve cell of basal portion of the forebrain, so that its association with Alzheimer's dementia has been remarked [*Pharmacia*, Vol.22, No.2, 147-151 (1986), *Ronen Seishin Igaku (Senile Psychiatry),* Vol.3, No.6, 751-758 (1986)]. Alzheimer's dementia refers to a disease that gives a pathological finding such as senile plaque or Alzheimer's fibrillar changes, which are accompanied by a clinical picture such as developmental disability, manic state, tonic seizures of lower limbs, or epileptic seizure, and is one disease of senile dementia. The Alzheimer's dementia tends to be increasing in recent aging society, so that a larger societal interest has been drawn thereto. However, there has not yet been found a method for ameliorating or treating such symptoms.

In the brain of a patient with Alzheimer's dementia, there has been found a dramatic denaturation, a drastic lowering of the activity of choline acetyl transferase (CAT), in the basal portion of the forebrain centering about Meynert's basal nuclei [*Annu. Rev. Neurosci.,* Vol.3, 77 (1980)]. In the studies of a rat brain in 1985, there has been elucidated that NGF is a neurotrophic factor at this site of the brain [*EMBO J.*, Vol.4, 1389 (1985)], so that the association of NGF with this disease has been remarked. In addition, there have been elucidated that in the striate body of the brain of a patient with Huntington's chorea, there are remarkable detachment of GABAergic nerve cell as well as detachment of cholinergic nerve cell, so that NGF also acts on the endogenous cholinergic nerve cell of the striate body [*Science*, Vol.234, 1341 (1986)], addressing a possibility that this disease is associated with NGF. The effects of NGF have been studied with an animal such as a rat which can serve as a model for various nerve diseases. There has been reported that the degeneration of the nerve cell can be stopped in a rat if NGF is intracerebrally administered before the degeneration becomes remarkable, and that the lowering of CAT activity is also prevented [*J*. *Neurosci*., Vol.6, 2155 (1986), *Brain Res*., Vol.293, 305 (1985), *Science*, Vol.235, 214 (1986), *Proc. Natl*. *Acad*. *Sci. USA*, Vol.83, 9231 (1986)]. Also, it has been proven that NGF is biosynthesized in the peripheral sympathetic nerve-dominant tissues and in the brain, and that each of fibroblasts or astroglia which are interstitial cells for peripheral tissues or brain tissues plays an important role for the NGF biosynthesis [*J*. *Biol*. *Chem*., Vol.259, 1259 (1984), *Biochem. Biophys. Res*. *Commun*., Vol.136, 57 (1986)]. In addition, it has been elucidated that antigenicity, molecular weight, isoelectric point and biological activity of the fibroblast-producing or astroglia-producing NGF are the same as NGF of conventionally well studied submandibular gland. At the same time, it has been found that a catecholamine such as norepinephrine, epinephrine or dopamine shows enhancing action for NGF production by a test of adding various neurotransmitters to a culture medium of fibroblasts (L-M cells) and astroglia [*J*. *Biol. Chem*., Vol.201, 6039 (1986)].

There has been expected that NGF can be used as a therapeutic agent for stopping degeneration in a nerve disease in which a site at which NGF acts as a neurotrophic factor is degenerated. In addition, once the cranial nerve cells are degenerated by cebrovascular disorders, cerebral tumor, cerebral apicitus, head injury, nerve degenerative disease, intoxication with an anesthetic, or the like, the degenerated cranial nerve cells would never recover during the life time, whereby various disorders such as emotional disorders and behavioral abnormality are consequently caused in addition to lowering in the intellectual functions and memory disabilities. On the other hand, nerve fiber shows plasticity, that is, when the nerve fiber is damaged, budding takes place from its surrounding healthy fibers, so that a new synapsis is formed in place of the damaged synapsis. Therefore, it has been expected that NGF can be used as a therapeutic agent for promoting restoration and regeneration of nerve functions at this stage.

However, when NGF is applied to a treatment of various nerve diseases, NGF must reach in very close vicinity of nerve cell that requires NGF, and NGF must be transmitted to lesion site of the cranial cell in a case of a disease in the central nervous system. However, NGF cannot be transmitted into the brain through the blood system. This is because the vascular endothelial cells in the brain are bound to each other by adhesion bonding (referred to as brain blood barrier), so that there is a limitation in the transport of a substance other than water, gas or an oil-soluble substance from blood to a brain tissue, whereby a protein (including NGF), which is polymeric substance, cannot pass through the brain blood barrier. There is a too large risk involved in the introduction of NGF directly into the brain by a surgical means, even if the introduction is conducted by the current techniques.

On the other hand, there has been developed a substance for enhancing NGF production, not a direct administration of NGF. Most of the compounds, however, have various problems such that the compounds have strong toxicity, or the compounds have effective concentration very closely approximating concentration at which toxicity is shown, or the compounds have severe adverse actions against nervous system such as nerve excitation action. Therefore, these compounds have not yet been actually used.

As described above, although it is thought that various diseases associated with growth factor can be treated or prevented by enhancing the growth factor, there have not been known substances, means, and the like capable of appropriately enhancing growth factor production as desired without showing toxicity or adverse actions.

### DISCLOSURE OF INVENTION

An object of the present invention is to provide a composition for enhancing growth factor production, comprising as an effective ingredient an enhancer for growth factor production, which is derived from fruit, seed, seed coat, flower, leaf, stem, root and/or root stem of a plant, for instance, a plant belonging to Umbelliferae, Compositae, Liliaceae, Ginkgoaceae, Gramineae, Rosaceae, Moraceae, Leguminosae, Tiliaceae, Cruciferae and Zingiberaceae; and a medicament, a food, a beverage or a feed, utilizing physiological actions of the enhancer for growth factor production.

Summarizing the present invention, a first invention of the present invention relates to a composition for enhancing growth factor production, characterized in that the composition comprises as an effective ingredient a plant-derived enhancer for growth factor production. In the first invention of the present invention, the plant is preferably one or more plants selected from the group consisting of plants belonging to Umbelliferae, Compositae, Liliaceae, Ginkgoaceae, Gramineae, Rosaceae, Moraceae, Leguminosae, Tiliaceae, Cruciferae and Zingiberaceae. In addition, the plant-derived enhancer for growth factor production is preferably one or more compounds selected from the group consisting of chlorogenic acid, dicaffeoyl-quinic acid, caffeoyl-quinic acid, isoorientin, safflomin A, guaianolide, xanthoangelol, 3'-O-β-D-glucopyranoyl khellactone, 7-O-β-D-glucopyranosyloxy-8-prenylcoumarin, caffeic acid methyl ester, caffeic acid ethyl ester, 8-carboxyl-3-hydroxy-5-methoxyl-2-dimethylchroman, 7-β-D-glucopyranosyloxy-6-prenylcoumarin, 4'-O-angeloyl-3'-O-[6-O-(β-D-glucopyranosyl)-β-D-glucopyranosyl]-khellactone, isoxanthohumol, xanthohumol B, xanthohumol D, and xanthohumol. Further, there is also encompassed a composition comprising a plant-derived extract or a fraction obtained from the plant-derived extract. Also, it is especially preferable that the composition can contain the plant-derived enhancer for growth factor production in a high concentration and/or at a high purity. Also, the growth factor is preferably a hepatocyte growth factor or a nerve growth factor.

A second invention of the present invention relates to a therapeutic agent or prophylactic agent for a disease requiring enhancement of growth factor production, characterized in that the agent comprises the composition of the first invention of the present invention.

A third invention of the present invention relates to a food, beverage or feed for enhancing growth factor production, characterized in that the food, beverage or feed comprises the composition of the first invention of the present invention.

A fourth invention of the present invention relates to a food, beverage or feed, characterized in that the food, beverage or feed comprises a plant-derived enhancer for growth factor production in a high concentration and/or at a high purity.

The plant-derived enhancer for growth factor production is preferably one or more compounds selected from the group consisting of chlorogenic acid, dicaffeoyl-quinic acid, caffeoyl-quinic acid, isoorientin, safflomin A, guaianolide, xanthoangelol, 3'-O-β-D-glucopyranoyl khellactone, 7-O-β-D-glucopyranosyloxy-8-prenylcoumarin, caffeic acid methyl ester, caffeic acid ethyl ester, 8-carboxyl-3-hydroxy-5-methoxyl-2-dimethylchroman, 7-β-D-glucopyranosyloxy-6-prenylcoumarin, 4'-O-angeloyl-3'-O-[6-O-(β-D-glucopyranosyl)-β-D-glucopyranosyl]-khellactone, isoxanthohumol, xanthohumol B, xanthohumol D, and xanthohumol.

A fifth invention of the present invention relates to a food, beverage or feed, characterized in that the food, beverage or feed comprises the composition of the first invention of the present invention in a high concentration and/or at a high purity.

The composition of the present invention is preferably a composition comprising one or more compounds selected from the group consisting of chlorogenic acid, dicaffeoyl-quinic acid, caffeoyl-quinic acid, isoorientin, safflomin A, guaianolide, xanthoangelol, 3'-O-β-D-glucopyranoyl khellactone, 7-O-β-D-glucopyranosyloxy-8-prenylcoumarin, caffeic acid methyl ester, caffeic acid ethyl ester, 8-carboxyl-3-hydroxy-5-methoxyl-2-dimethylchroman, 7-β-D-glucopyranosyloxy-6-prenylcoumarin, 4'-O-angeloyl-3'-O-[6-O-(β-D-glucopyranosyl)-β-D-glucopyranosyl]-khellactone, isoxanthohumol, xanthohumol B, xanthohumol D, and xanthohumol.

A sixth invention of the present invention relates to a food, beverage or feed for enhancing nerve growth factor production, comprising xanthohumol in an amount of 0.00007% by weight or more.

A seventh invention of the present invention relates to a food, beverage or feed for enhancing growth factor production, comprising a food, beverage or feed comprising an enhancer for growth factor production, or a treated product thereof. A preferred embodiment thereof is a feed, beverage or feed comprising a beer. The beer includes, beers, low-malt beer, nonalcoholic beer beverages, concentrates thereof, dilutions thereof and a beverage containing an extract from *Humulus lupulus.*

An eighth invention of the present invention relates to a food, beverage or feed for enhancing growth factor production, comprising an extract from *Humulus lupulus*. It is preferable that the extract from *Humulus lupulus* is contained in an amount of 0.0001% by weight or more in the food, beverage or feed.

Nineth to eleventh inventions of the present invention relate to a composition for enhancing growth factor production, a therapeutic agent or prophylactic agent for a disease requiring enhancement of growth factor production, and a food, beverage or feed for enhancing growth factor production, characterized in that each of them comprises a guaianolide as an effective ingredient.

### BRIEF DESCRIPTION OF THE DRAWINGS

Figure 1 is a chart (upper) showing ¹H-NMR spectrum of the compound (1) and a chart (lower) showing ¹H-NMR spectrum of the chlorogenic acid preparation.
Figure 2 is a graph showing the correlation between the concentration of chlorogenic acid contained in extraction fractions 1 to 3 derived from *Angelica keiskei* koidz. and the enhancing activity for HGF production of each fraction.
Figure 3 is a graph showing the correlation between the concentration of chlorogenic acid preparation and the enhancing activity for HGF production.
Figure 4 is a chart showing ¹H-NMR spectrum of the compound (2).
Figure 5 is a chart showing MS spectrum of the chloroform-extracted fraction A-a-2 of *Chrysanthemum morifolium.*
Figure 6 is a chart showing ¹H-NMR spectrum of the chloroform-extracted fraction A-a-2 of *Chrysanthemum morifolium.*
Figure 7 is a chart showing MS spectrum of the chloroform-extracted fraction A-b-1 of *Chrysanthemum morifolium.*
Figure 8 is a chart showing IR spectrum of the chloroform-extracted fraction A-b-1 of *Chrysanthemum morifolium.*
Figure 9 is a chart showing ¹H-NMR spectrum of the chloroform-extracted fraction A-b-1 of *Chrysanthemum morifolium*.
Figure 10 is a chart showing ¹³C-NMR spectrum of the chloroform-extracted fraction A-b-1 of *Chrysanthemum morifolium*.
Figure 11 is a chart showing mass spectrum of the fraction, including the peak at 7.82 minutes in the extraction fraction of *Angelica keiskei* koidz.
Figure 12 is a chart showing IR spectrum of the fraction, including the peak at 7.82 minutes in the extraction fraction of *Angelica keiskei* koidz.
Figure 13 is a chart showing ¹H-NMR spectrum of the fraction, including the peak at 7.82 minutes in the extraction fraction of *Angelica keiskei* koidz.
Figure 14 is a chart showing ¹³C-NMR spectrum of the fraction, including the peak at 7.82 minutes in the extraction fraction of *Angelica keiskei* koidz.
Figure 15 is a chart showing mass spectrum of the fraction, including the peak at 11.09 minutes in the extraction fraction of *Angelica keiskei* koidz.
Figure 16 is a chart showing IR spectrum of the fraction, including the peak at 11.09 minutes in the extraction fraction of *Angelica keiskei* koidz.
Figure 17 is a chart showing ¹H-NMR spectrum of the fraction, including the peak at 11.09 minutes in the extraction fraction of *Angelica keiskei* koidz.
Figure 18 is a chart showing ¹³C-NMR spectrum of the fraction, including the peak at 11.09 minutes in the extraction fraction of *Angelica keiskei* koidz.
Figure 19 is a chart showing FAB-MS spectrum of the fraction 10 from root portions of *Angelica keiskei* koidz.
Figure 20 is a chart showing ¹H-NMR spectrum of the fraction 10 from root portions of *Angelica keiskei* koidz.
Figure 21 is a chart showing FAB-MS spectrum of the fraction 13-2 from root portions of *Angelica keiskei* koidz.
Figure 22 is a chart showing ¹H-NMR spectrum of the fraction 13-2 from root portions of *Angelica keiskei* koidz.
Figure 23 is a chart showing ¹³C-NMR spectrum of the fraction 13-2 from root portions of *Angelica keiskei* koidz.
Figure 24 is a chart showing FAB-MS spectrum of the fraction 18-3 from root portions of *Angelica keiskei* koidz.
Figure 25 is a chart showing ¹H-NMR spectrum of the fraction 18-3 from root portions of *Angelica keiskei* koidz.
Figure 26 is a chart showing FAB-MS spectrum of the fraction 18-4 from root portions of *Angelica keiskei* koidz.
Figure 27 is a chart showing ¹H-NMR spectrum of the fraction 18-4 from root portions of *Angelica keiskei* koidz.
Figure 28 is a chart showing ¹³C-NMR spectrum of the fraction 18-4 from root portions of *Angelica keiskei* koidz.
Figure 29 is a chart showing FAB-MS spectrum of the fraction 19-, 20-5 from root portions of *Angelica keiskei* koidz.
Figure 30 is a chart showing ¹H-NMR spectrum of the fraction 19-, 20-5 from root portions of *Angelica keiskei* koidz.
Figure 31 is a chart showing ¹³C-NMR spectrum of the fraction 19-, 20-5 from root portions of *Angelica keiskei* koidz.
Figure 32 is a chart showing FAB-MS spectrum of the fraction 19-, 20-6 from root portions of *Angelica keiskei* koidz.
Figure 33 is a chart showing ¹H-NMR spectrum of the fraction 19-, 20-6 from root portions of *Angelica keiskei* koidz.
Figure 34 is a chart showing ¹³C-NMR spectrum of the fraction 19-, 20-6 from root portions of *Angelica keiskei* koidz.
Figure 35 is a chart showing FAB-MS spectrum of the fraction 28 from root portions of *Angelica keiskei* koidz.
Figure 36 is a chart showing ¹H-NMR spectrum of the fraction 28 from root portions of *Angelica keiskei* koidz.
Figure 37 is a chart showing FAB-MS spectrum of the fraction A-5 from xanthohumol fraction.
Figure 38 is a chart showing ¹H-NMR spectrum of the fraction A-5 from xanthohumol fraction.
Figure 39 is a chart showing ¹H-NMR spectrum of the xanthohumol fraction derived from *Humulus lupulus.*

### BEST MODE FOR CARRYING OUT THE INVENTION

In the present invention, there is provided a composition for enhancing growth factor production, characterized in that the composition comprises as an effective ingredient a plant-derived enhancer for growth factor production. The existence of the substance showing enhancing action for growth factor production in a plant has been found for the first time in the present invention. Any of the substances listed as the effective ingredients in the present specification can be used alone or in admixture of two or more kinds in the present invention.

The plant-derived enhancer for growth factor production according to the present invention is not particularly limited, as long as the enhancer is a substance which can be obtained by the process for preparing an enhancer described below. Especially, a substance having an enhancing action for growth factor production, which is derived from one or more plants selected from the group consisting of plants belonging to Umbelliferae, Compositae, Liliaceae, Ginkgoaceae, Gramineae, Rosaceae, Moraceae, Leguminosae, Tiliaceae, Cruciferae and Zingiberaceae, is preferable as the enhancer.

Examples of the plant belonging to Umbelliferae include, for instance, *Angelica keiskei* koidz., *Angelica pubescens, Daucus, Oenanthe, Cryptotaenia, Apium,* and the like. Examples of the plant belonging to Compositae include, for instance, *Chrysanthemum, Taraxacum, Helianthus, Cirsium yozoense, Chrysanthemum coronarium, Arctium, Petasites,* butterbar, *Artemisia L.,* and the like, and as *Artemisia L.,* KWANGHWA MUGWORT can be preferably used. Examples of the plant belonging to Liliaceae include, for instance, onion, scallion, garlic, *Tulipa, Lilium,* and the like. Examples of the plant belonging to Ginkgoaceae include, for instance, *Ginkgo biloba.* Examples of the plant belonging to Gramineae include, for instance, *Phyllestachys pubescens, Phyllestachys bambusoides, Phyllestachys nigra, Bambusa, Sasa, Oryza, Hordeum, Phragmites, Zoysia, Panicum*, and the like. Grain crops and processed products thereof such as rice bran and wheat bran can also be used. Examples of the plant belonging to Rosaceae include, for instance, *Prunus donarium, Prunus yedoensis, Prunus Mume, Prunus Persia, Prunus domestica, Prunus amygdalus, Malum, Rosa, Rubus, Eriobotrya, Rosa rugasa, Sedum erythrosticum,* and the like. Examples of the plant belonging to Moraceae include, for instance, *Morus, Ficus, Humulus lupulus, Cudrania tricuspidata, Cudrania, Artocarpus, Ficus elastica*, and the like. Examples of the plant belonging to Zingiberaceae include, for instance, *Zingiber officinale, Curcuma, Zingiber mioga, Zingiber zerumbet,* and the like, and as *Curcuma*, especially *Curcuma zedoaeia Roscoe* can be preferably used. Examples of the plant belonging to Tiliaceae include, for instance, mulukhiya, and the like. Examples of the plant belonging to Legminosae include, for instance, soybeans, *azuki* beans, kidney beans, green beans, and the like. Examples of the plant belonging to Cruciferae include, for instance, cabbage, broccoli, cauliflower, a Chinese cabbage, rape blossoms, and the like.

In addition, in the present invention, the plant can be used in any form, for instance, in the form of fruit, seed, seed coat, flower, leaf, stem, root, and/or root stem, or processed products thereof, including rice bran, wheat bran, soybean embryo, seed coat of soybean, and soyameal, or a plant itself, skin portion thereof or portions thereof other than the skin removed.

In addition, the plant-derived enhancer for growth factor production in the present invention is not particularly limited, as long as the enhancer is a plant-derived substance having an activity for enhancing growth factor production. From the viewpoint of exhibiting the desired effects of the present invention, preferred examples include one or more compounds selected from the group consisting of chlorogenic acid, dicaffeoyl-quinic acid, caffeoyl-quinic acid, isoorientin, safflomin A, guaianolide, xanthoangelol, 3'-O-β-D-glucopyranoyl khellactone, 7-O-β-D-glucopyranosyloxy-8-prenylcoumarin, caffeic acid methyl ester, caffeic acid ethyl ester, 8-carboxyl-3-hydroxy-5-methoxyl-2-dimethylchroman, 7-β-D-glucopyranosyloxy-6-prenylcoumarin, 4'-O-angeloyl-3'-O-[6-O-(β-D-glucopyranosyl)-β-D-glucopyranosyl]-khellactone, isoxanthohumol, xanthohumol B, xanthohumol D, and xanthohumol. Also, the exhibition of the enhancing action for growth factor production of the substance used as an effective ingredient in the present invention can be evaluated by the method disclosed in the present invention (for instance, a method described in item (1) of Example 4 and that described in Example 13). The enhancer is not particularly limited as long as the enhancer is a plant-derived substance which shows enhancing action for growth factor production. As a matter of course, the enhancer can be those other than the compounds preferred as the effective ingredients exemplified above. In the present invention, the preferred caffeoyl-quinic acid is exemplified by 5-caffeoyl-quinic acid and 4-caffeoyl-quinic acid. Also, in the present invention, the preferred guaianolide is exemplified by 2-oxo-8-angeloyloxy-guaia-3(4),11(13)-dien-12,6-olide or 3,4-epoxy-8-angeloyloxy-guaia-1(10),11(13)-dien-12,6-olide. In the present invention, the term "plant-derived" means those obtained from plant raw materials.

In addition, one embodiment of the composition for enhancing growth factor production of the present invention is exemplified by the composition comprising a plant-derived extract or a fraction obtained from the plant-derived extract, comprising an enhancer for growth factor production. The composition may be the above-mentioned extract or the fraction itself. The extraction and purification from a plant can be carried out by the following known method. For instance, fruit, seed, seed coat, flower, leaf, stem, root and/or rhizome or the like, of the raw material plant is collected in an appropriate timing, and thereafter used directly or subjected to a drying process such as an ordinary air-drying and then powdered as desired, to give a raw material for extraction. Also, the raw material may be aged under various conditions, and the aged raw material may be used. Also, processed product of a plant, for instance, rice bran, wheat bran, soybean embryo, seed coat of soybean, soyameal, or the like may be used. When the raw material is a squeezed juice or sap of a plant, the raw material can be directly used as a raw material for extraction. In the present invention, the term "plant-derived extract" refers to a substance obtained from a plant by subjecting a plant to an extraction procedure using an extraction solvent.

The enhancer for growth factor production can be prepared from a plant as follows by a known extraction method. For instance, the raw material is powdered or cut into thin pieces, and thereafter extracted in a batch process or continuous process using a solvent. The extraction solvent includes hydrophilic or lipophilic solvents such as water, chloroform, alcohols such as ethanol, methanol and isopropyl alcohol, ketones such as acetone and methyl ethyl ketone, methyl acetate, and ethyl acetate, which can be used alone or appropriately as a mixed solution as desired. Usually, the extraction method is preferably carried out with water, an ethanol-containing water, or ethanol. The amount of the extraction solvent may be appropriately determined, and the extraction solvent may be used in an amount of preferably from 1 to 100 times the amount of the raw material. The extraction temperature may be also appropriately determined according to its purpose. In the case of a water extraction, usually the extraction temperature is preferably from 4° to 130°C, more preferably from 25° to 100°C. In addition, when ethanol is contained in the solvent, the extraction temperature is preferably within the range of from 4° to 60°C. The extraction time may be also determined in consideration of the extraction efficiency. Usually, the raw material, the extraction solvent, and the extraction temperature are preferably set in consideration of the extraction efficiency so that the extraction time is preferably from several minutes to several days, more preferably from 5 minutes to 3 hours. The extraction procedure may be carried out with stirring or allowing the mixture to stand still, and the extraction procedure may be optionally repeated several times. By the above procedure, the enhancer for growth factor production is obtained as an extract containing the enhancer. The extract may be optionally subjected to such a treatment as filtration, centrifugation, concentration, ultrafiltration, or molecular sieving, whereby an extract in which the desired enhancer for growth factor production is concentrated can be prepared. The enhancing activity for growth factor production of the extract or concentrated extract can be conveniently assayed by the method described in item (1) of Example 4 or the method described in Example 13.

In addition, in the present invention, the fraction obtained from a plant extract refers to an extract obtained by fractionating a plant-derived extract by a known method, or a fraction containing a single substance, obtained by repeating the fractionation procedure for plural times, and the fractions are encompassed in the present invention, as long as they have enhancing action for growth factor production. The above-mentioned fractionation means include extraction, separation by precipitation, column chromatography, thin-layer chromatography, and the like. The enhancer for growth factor production can also be isolated by further proceeding the purification of the resulting fraction using the enhancing activity for growth factor production as an index.

In addition, extracts obtained by processing a plant into a tealeaf form by a known method, and extracting with the tealeaves, or fractions obtained from the plant-derived extract can be also used as the extracts or fractions obtained from the plant-derived extracts of the present invention, as long as they have enhancing action for growth factor production. Also, these extracts or fractions obtained from the plant-derived extracts can be used in an admixture of two or more kinds. Incidentally, in the present invention, extracts obtained by different extraction methods from the same plant or fractions obtained from the plant-derived extracts can be used in an admixture of two or more kinds.

The process for preparing a composition for enhancing growth factor production other than the plant-derived extracts or fractions obtained from the plant-derived extracts comprises, for instance, drying a plant, and powdering the dried product, whereby the composition in the powdery form can be obtained.

It is especially preferable that the plant-derived composition for enhancing growth factor production in the present invention comprises the enhancer for growth factor production in a high concentration and/or at high purity, as compared to that of the plant itself. Here, the term "high concentration" means that the weight of the enhancer for growth factor production per unit weight of the composition is greater than that of the enhancer for growth factor production per unit weight of the raw material plant. In addition, the term "high purity" means that the content ratio of the enhancer for growth factor production of the composition is higher than that of the raw material plant.

In addition, the present invention provides a food, beverage or feed, comprising an enhancer for growth factor production in a high concentration and/or at a high purity. This means that the enhancer for growth factor production is contained in the food, beverage or feed of the present invention in a high concentration and/or at a high purity, as compared to that of a conventional food, beverage or feed.

Here, the present invention also encompasses an enhancing agent for growth factor production containing the composition as an effective ingredient.

In the present invention, the shape of the plant-derived composition for enhancing growth factor production is not particularly limited as long as the composition contains the plant-derived enhancer for growth factor production, and may be any forms of powder, solid or liquid. Also, the above composition can be granulated by a known method to give a granular solid product to be used as the composition for enhancing growth factor production of the present invention. The granulation method is not particularly limited, and examples thereof include tumbling granulation, agitation granulation, fluidized bed granulation, airflow granulation, extruding granulation, compression molding granulation, disintegration granulation, spray granulation, spray drying granulation, and the like. The powdery composition is dissolved in a liquid such as water or an alcohol, to make the composition in a liquid form, which can be used as the composition for enhancing growth factor production of the present invention.

In the effective ingredient of the present invention, toxicity is not particularly recognized as described below, and there is no concern for generation of adverse actions, so that the enhancement of the growth factor production can be carried out safely and appropriately. Therefore, the therapeutic agent, the prophylactic agent, the food, the beverage or the feed of the present invention, each comprising the effective ingredient, is effective for treatment or prevention of a disease requiring the enhancement of growth factor production.

The growth factor in the present invention is not particularly limited, as long as the growth factor has activity for accelerating the cell growth. The growth factor is exemplified by hepatocyte growth factor (HGF), nerve growth factor (NGF), neurotrophic factor, epidermal growth factor, milk-derived growth factor, fibroblast growth factor, brain-derived fibroblast growth factor, acidic fibroblast growth factor, platelet-derived growth factor, platelet basic protein, connective tissue-activating peptide, insulin-like growth factor (IGF), colony-stimulating factor, erythropoietin, thrombopoietin, T cell growth factor, interleukins (for instance, interleukins 2, 3, 4, 5, 7, 9, 11 and 15), B cell growth factor, cartilage-derived factor, cartilage-derived growth factor, bone-derived growth factor, skeletal growth factor, epithelial cell growth factor, epithelial cell-derived growth factor, oculus-derived growth factor, testis-derived growth factor, Sertoli's cell-derived growth factor, mammotropic factor, spinal cord-derived growth factor, macrophage-derived growth factor, mesodermal growth factor, transforming growth factor-α, transforming growth factor-β, heparin-binding EGF-like growth factor, amphyllegrin, SDGF, betacellulin, epiregulin, neuregulin 1, 2 and 3, vascular endotherial growth factor, neurotrophin, BDNF, NT-3, NT-4, NT-5, NT-6, NT-7, glial cell line-derived neurotrophic factor, stem cell factor, midkine, pleiotrophin, ephrin, angiopoietin, activin, tumor necrosis factor, interferons, and the like. According to the present invention, the enhancement for production of nerve growth factor (NGF) or hepatocyte growth factor (HGF) can be especially suitably carried out.

HGF is a factor for hepatocyte regeneration, and is further a factor for facilitating motility of cells, as well as a tumor cytotoxic factor. HGF accelerates growth of many of epithelial cells, such as changioepithelial cells, renal tubule epithelial cells, and gastric mucosa cells, as well as hepatocytes. In addition, HGF exhibits remarkably a wide variety of physiological activity, for instance, HGF induces morphological formations as seen in facilitation of motility of epithelial cells, vascularization or luminal formation of epithelial cells. Therefore, by enhancing the production of HGF, hepatic disorders such as hepatitis, severe hepatitis, fulminant hepatitis, cirrhosis, and cholestasia in the liver, renal disorders caused by drugs and the like, gastrointestinal disorders, vascular disorders, chronic nephritis, pneumonia, wound, diabetes, cancer, and the like can be treated or prevented.

On the other hand, NGF is an endogenous growth factor for maintaining viability and functions of nerve cells, elongating nerve cells in accordance with a concentration gradient of NGF, or the like. By enhancing the production of NGF, the treatment or prevention of senile dementia such as Alzheimer's disease, peripheral nerve disorder, cerebrovascular disorder, cerebral tumor, cerebral apicitis, nerve degenerative disease caused by head injury, diseases requiring recovery and regeneration of nerve functions, caused by intoxication with an anesthetic, and the like can be carried out. In addition, it is useful in the treatment or prevention of amyotrophic lateral sclerosis, drug-induced peripheral nerve disorder, diabetic peripheral nerve disorder, Parkinson's disease, sensory nerve disorder, retinitis pigmentosa, macular dystrophy, and the like.

The disease requiring the enhancement of growth factor production in the present invention is not particularly limited, as long as the treatment or prevention can be carried out by administering the therapeutic agent, the prophylactic agent or the like of the present invention. The disease is exemplified by hepatic disorders such as hepatitis, severe hepatitis, fulminant hepatitis, cirrhosis, and cholestasia in the liver, renal disorders caused by drugs and the like, gastrointestinal disorders, vascular disorders, chronic nephritis, pneumonia, wound, diabetes, cancer, dementia, nerve disorders, peripheral neural disease, cerebral ischemia, diabetic neuropathy, and the like.

The therapeutic agent or prophylactic agent of the present invention for a disease requiring the enhancement for growth factor production, characterized in that the therapeutic agent or prophylactic agent comprises the plant-derived composition for enhancing growth factor production used in the present invention, can be appropriately prepared by using the plant-derived composition for growth factor production.

The therapeutic agent or prophylactic agent of the present invention may be formed into a preparation by combining the plant-derived composition for enhancing growth factor production used in the present invention with a known pharmaceutical vehicle. Alternatively, the agent can be formed into a preparation by combining the enhancer for growth factor production with a known pharmaceutical vehicle.

The above preparation is generally manufactured by formulating the plant-derived composition for enhancing growth factor production used in the present invention with a pharmacologically acceptable liquid or solid vehicle, and optionally adding thereto a solvent, a dispersant, an emulsifier, a buffer, a stabilizer, an excipient, a binder, a disintegrant, a lubricant, or the like, thereby being usually made into a solid agent such as a tablet, a granule, a powder, a fine powder, and a capsule, or a liquid agent such as a common liquid agent, a suspension agent or an emulsion agent. In addition, there can be also prepared a dry product which can be made liquid by adding an appropriate vehicle before use, and an external preparation.

The pharmaceutical vehicle can be selected depending upon the above-mentioned administration form and preparation form of the therapeutic agent or prophylactic agent. In the case of an orally administered preparation, there can be utilized, for instance, starch, lactose, saccharose, mannitol, carboxymethyl cellulose, cornstarch, an inorganic salt or the like. In addition, during the preparation of the orally administered preparation, a binder, a disintegrant, a surfactant, a lubricant, a fluidity accelerator, a flavor, a colorant, a perfume, and the like can be further formulated.

On the other hand, a non-orally administered preparation can be prepared by dissolving or suspending the plant-derived composition for enhancing growth factor production, which is the effective ingredient of the present invention, in a diluent such as distilled water for injection, physiological saline, an aqueous solution of glucose, vegetable oil for injection, sesame oil, peanut oil, soybean oil, corn oil, propylene glycol or polyethylene glycol, by a conventional method, and adding a microbicide, a stabilizer, an osmotic regulator, a soothing agent, or the like as necessary.

The therapeutic agent or prophylactic agent of the present invention is administered via an administration route appropriate for each of the preparation form. The administration route is not limited to specific one. The agent can be administered internally or externally (or topically) or by injection. The injection can be administered, for instance, intravenously, intramuscularly, subcutaneously, intracutaneously, or the like. External preparations include a suppository.

The dose for the therapeutic agent or prophylactic agent of the present invention is changeable and properly set depending upon its preparation form, administration method, purpose of use, age, body weight, symptom or the like of the patient to which the agent is applied, or the like. The dose for the agent is such that the amount of the plant-derived composition for enhancing growth factor production used in the present invention contained in the preparation is preferably from 0.001 to 2000 mg/kg, preferably from 0.01 to 200 mg/kg, per day for adult. In addition, the dose for the agent is such that the amount of the plant-derived enhancer for growth factor production contained in the preparation is preferably from 0.0001 to 2000 mg/kg, more preferably from 0.001 to 200 mg/kg, still more preferably from 0.01 to 20 mg/kg per day for adult.

As a matter of course, the dose varies depending upon various conditions, so that an amount smaller than the dose mentioned above may be sufficient, or an amount exceeding the dose range may be required. The agent of the present invention can be directly orally administered, or the agent can be added to any foodstuffs to take it on a daily basis. Also, the plant-derived composition for enhancing growth factor production used in the present invention may be used as a raw material of foodstuffs for enhancing growth factor production.

In addition, in another embodiment of the present invention, there can provided an enhancing agent for growth factor production comprising an enhancer for growth factor production as an effective ingredient according to the present invention. The enhancing agent may the above-mentioned effective ingredient itself, or a composition comprising the above-mentioned effective ingredient. The enhancing agent for growth factor production can be produced by formulating the above-mentioned effective ingredient with other ingredients which can be used for the same application as the effective ingredient, and forming into a form of reagent usually used according to the above-mentioned process for producing the therapeutic agent or prophylactic agent. The content of the above-mentioned effective ingredient in the enhancing agent is not particularly limited, as long as the content is in an amount so that the desired effects of the present invention can be exhibited in consideration of administration form, administration purpose or the like of the enhancing agent. Also, the amount of the enhancing agent used is not particularly limited, as long as the desired effects of the present invention can be exhibited. Especially in the case where the enhancing agent is administered to a living body, the enhancing agent is preferably used in an amount so that the effective ingredient can be administered within the dose range of the effective ingredient in the above-mentioned therapeutic agent or prophylactic agent. The enhancing agent for growth factor production is useful for enhancement of growth factor production, especially useful for enhancement of the production of HGF or NGF in a case of a disease requiring enhancement for HGF or NGF production. In addition, the enhancing agent is also useful for functional studies of growth factor and screening of drugs for growth factor-associated diseases.

Further, in a still another embodiment of the present invention, there can provided a method for enhancing growth factor production, comprising administering the above-mentioned effective ingredient according to the present invention to an animal. This method can be carried out by administering the above-mentioned effective ingredient, preferably as the above-mentioned enhancing agent for growth factor production, to an animal that is predicted to require or requires enhancement of growth factor production, whereby the growth factor production is enhanced. The administration method, dose, or the like of the effective ingredient may be similar to that of the above-mentioned enhancing agent for growth factor production. In the method for enhancing growth factor production, the therapeutic agent or prophylactic agent, or the food, beverage or feed described below of the present invention can be used. In addition, the term "animal" includes a mammal such as human, dogs, cats, *Bos, Porcus, Equus,* and the like, among which the method is preferably used for human. The method for enhancing growth factor production is useful for, for instance, the enhancement of growth factor production in a case of treatment or prevention of a disease requiring enhancement for growth factor production. In addition, the method is also useful for functional studies of growth factor and screening of drugs for growth factor-associated diseases.

The food, beverage or feed of the present invention comprises the plant-derived composition for enhancing growth factor production, or the plant-derived enhancer for growth factor production used in the present invention. Since the food, beverage or feed has enhancing action for growth factor production, the food, beverage or feed is very useful in amelioration or prevention of symptoms for a disease requiring enhancement for growth factor production, which is sensitive to the plant-derived composition for enhancing growth factor production or the like used in the present invention, or in amelioration of physical condition of an organism as described below.

Here, in this embodiment, the term "comprise or comprising" includes the meanings of containing, adding and diluting. The term "containing" refers to an embodiment of containing the effective ingredient used in the present invention in the food, beverage or feed; the term "adding" refers to an embodiment of adding the effective ingredient used in the present invention to a raw material for the food, beverage or feed; and the term "diluting" refers to an embodiment of adding a raw material for the food, beverage or feed to the effective ingredient used in the present invention.

The method for preparing the food or beverage of the present invention is not particularly limited. For instance, formulation, cooking, processing, and the like can be carried out in accordance with those generally employed for foods or beverages, and the food or beverage can be prepared by the general methods for preparing a food or beverage, as long as the resulting food or beverage contain the plant-derived composition for enhancing growth factor production or the plant-derived enhancer for growth factor production, which has an enhancing action for growth factor production. Also, the present invention encompasses a food or beverage for enhancing growth factor production, obtained by mixing a plant in a food or beverage, heating the mixture, or allowing the mixture to stand, and thereafter removing the plant-derived extraction residue as desired. In addition, a food or beverage having enhancing action for growth factor production, comprising the food or beverage comprising an enhancer for growth factor production, or a processed product thereof is also the food or beverage of the present invention. The processed product includes, for instance, concentrates or dilutions of a beverage having enhancing action for growth factor production.

An embodiment thereof is preferably a food, beverage or feed for enhancing growth factor production, comprising a beer. The beer includes, beers, low-malt beer, nonalcoholic beer beverages, concentrates thereof, dilutions thereof and a beverage containing an extract from *Humulus lupulus.*

The food or beverage of the present invention is not particularly limited. The food or beverage includes, for instance, processed agricultural and forest products, processed stock raising products, processed marine products and the like, including processed grain products such as processed wheat products, processed starch products, processed premix products, noodles, macaronis, bread, bean jam, buckwheat noodles, wheat-gluten bread, rice noodle, *fen-tiao*, and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, boiled fish paste, tubular roll of boiled fish paste, cake of ground fish, deep-fried patty of fish paste, fish ball, sinew, fish meat ham and sausage, dried bonito, products of processed fish egg, marine cans, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks, rice snacks, and whiskey-containing bonbon; alcohol beverages such as *sake,* Chinese liquor, wine, whiskey, Japanese distilled liquor *(shochu),* vodka, brandy, gin, rum, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, binned or pouched foods such as rice topped cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste *(miso)* soup, precooked foods, precooked beverages, and precooked soup; frozen foods such as *sukiyaki*, pot-steamed hotchpotch, split and grilled eel, hamburger steak, *shao-mai*, dumpling stuffed with minced pork, various sticks, and fruit cocktails; solid foods; liquid foods (soups); spices; and the like.

The content of the above-mentioned plant-derived effective ingredient having enhancing action for growth factor production in the food or beverage of the present invention is not particularly limited, and the content can be appropriately selected from the viewpoints of sensory ability and exhibition of activity. The content of the effective ingredient in the food is, for instance, preferably 0.000001% by weight or more, more preferably from 0.00001 to 100% by weight, still more preferably from 0.0003 to 90% by weight, or the content in the beverage is, for instance, preferably 0.000001% by weight or more, more preferably from 0.00001 to 100% by weight, still more preferably from 0.0003 to 90% by weight. Also, the food or beverage of the present invention may be taken such that the effective ingredient contained therein is in an amount of preferably from 0.0001 to 100 mg/kg body weight, more preferably from 0.001 to 10 mg/kg body weight, still more preferably from 0.01 to 1 mg/kg body weight, per day for adult.

In addition, according to the present invention, there is provided a food or beverage comprising the above-mentioned composition for enhancing growth factor production in a high concentration and/or at a high purity. Here, the phrase "comprising the above-mentioned composition for enhancing growth factor production in a high concentration and/or at a high purity" means that the enhancer for growth factor production derived from the composition for enhancing growth factor is contained in the food or beverage of this embodiment of the present invention at a level of a high concentration or high purity, or that the composition for enhancing growth factor production is contained therein at a level comparable to the containment of the enhancer for growth factor production in a high concentration and/or at a high purity, from the viewpoint of exhibiting the enhancing action for growth factor production.

In the production of the food or beverage, there may be used as a composition for enhancing growth factor production, for instance, a plant-derived extract comprising an enhancer for growth factor production, for instance, an extract selected from an extract from a plant belonging to Umbelliferae, a plant belonging to Compositae, a plant belonging to Liliaceae, a plant belonging to Ginkgoaceae, a plant belonging to Gramineae, a plant belonging to Rosaceae, a plant belonging to Moraceae, a plant belonging to Leguminosae, a plant belonging to Tiliaceae, a plant belonging to Cruciferae and a plant belonging to Zingiberaceae. As the effective ingredient, there may be preferably used a composition comprising one or more compounds selected from the group consisting of chlorogenic acid, dicaffeoyl-quinic acid, caffeoyl-quinic acid, isoorientin, safflomin A, guaianolide, xanthoangelol, 3'-O-β-D-glucopyranoyl khellactone, 7-O-β-D-glucopyranosyloxy-8-prenylcoumarin, caffeic acid methyl ester, caffeic acid ethyl ester, 8-carboxyl-3-hydroxy-5-methoxyl-2-dimethylchroman, 7-β-D-glucopyranosyloxy-6-prenylcoumarin, 4'-O-angeloyl-3'-O-[6-O-(β-D-glucopyranosyl)-β-D-glucopyranosyl]-khellactone, isoxanthohumol, xanthohumol B, xanthohumol D, and xanthohumol, for instance, a plant-derived extract.

The present invention also provides a food or beverage for enhancing health condition, comprising the plant-derived enhancer for growth factor production in a high concentration and/or at a high purity. By the daily intake of the above food or beverage, the growth factor production in a living body is enhanced, so that health condition can be maintained or enhanced.

In other words, in a certain embodiment of the present invention, there is provided a food or beverage, characterized in that the food or beverage comprises a plant-derived enhancer for growth factor production in a high concentration and/or at a high purity.

In the embodiment of the present invention, the plant-derived enhancer for growth factor production is preferably one or more compounds selected from the group consisting of chlorogenic acid, dicaffeoyl-quinic acid, caffeoyl-quinic acid, isoorientin, safflomin A, guaianolide, xanthoangelol, 3'-O-β-D-glucopyranoyl khellactone, 7-O-β-D-glucopyranosyloxy-8-prenylcoumarin, caffeic acid methyl ester, caffeic acid ethyl ester, 8-carboxyl-3-hydroxy-5-methoxyl-2-dimethylchroman, 7-β-D-glucopyranosyloxy-6-prenylcoumarin, 4'-O-angeloyl-3'-O-[6-O-(β-D-glucopyranosyl)-β-D-glucopyranosyl]-khellactone, isoxanthohumol, xanthohumol B, xanthohumol D, and xanthohumol. There is provided a food or beverage comprising the above compound in a high concentration and/or at a high purity.

For instance, in the present invention, when a bamboo extract is used as a composition containing an enhancer for HGF production, it is suitable that the extract is contained in the food or beverage in an amount of preferably 0.00001% by weight or more as calculated on a dry weight basis. The bamboo extract can be prepared by, for instance, keeping bamboo blades in hot water. In addition, the isoorientin content in the extract is determined, and its effective amount may be contained in the food or beverage. It is suitable that the isoorientin content is such that isoorientin is contained in the food or beverage in an amount of preferably 0.01% by weight or more.

In the present invention, when an onion extract is used as a composition containing an enhancer for HGF production, for instance, a hot-water extract of onionskin, for instance, a product obtained by heat-treating 5 g of onionskin in 100 ml of water at 100°C for 15 minutes, may be contained in the food or beverage in an amount of preferably 1% by weight or more as calculated on a dry weight basis.

In the present invention, when a biloba extract is used as a composition containing an enhancer for HGF production, for instance, a hot-water extract of biloba tealeaves, for instance, a product obtained by heat-treating 3 g of biloba tealeaves in 200 ml of water at 100°C for 15 minutes, may be contained in the food or beverage in an amount of preferably 5% by weight or more as calculated on a dry weight basis.

In the present invention, when a biloba extract is used as a composition containing an enhancer for HGF production, for instance, a hot-water extract of biloba tealeaves, for instance, a product obtained by heat-treating 3 g of biloba tealeaves in 200 ml of water at 100°C for 15 minutes, may be contained in the food or beverage in an amount of preferably 5% by weight or more as calculated on a dry weight basis.

In the present invention, when an *Artemisia L*. extract is used as a composition containing an enhancer for HGF production, it is suitable that the extract is contained in the food or beverage in an amount of preferably 0.0001% by weight or more as calculated as a dry extract. The extract may be prepared by, for instance, extracting 10 g of *Artemisia L.* with 150 ml of water at 60°C for 1 hour. In addition, when 3,5-dicaffeoyl-quinic acid in the *Artemisia L.* extract is used as an enhancer for HGF production, it is suitable that 3,5-dicaffeoyl-quinic acid is contained in the food or beverage in an amount of preferably 0.0005% by weight or more.

Also, when chlorogenic acid in the *Artemisia L.* extract is used as an enhancer for HGF production, it is suitable that chlorogenic acid is contained in the food or beverage in an amount of preferably 0.001% by weight or more.

In the present invention, when chlorogenic acid derived from *Angelica keiskei* koidz. is used as an enhancer for HGF production, it is suitable that chlorogenic acid is contained in the food or beverage in an amount of preferably 0.001% by weight or more.

In the present invention, when a Chrysanthemum flower extract is used as a composition containing an enhancer for HGF production, for instance, a hot-water extract of Chrysanthemum flower, for instance, a product obtained by heat-treating 10 g of the Chrysanthemum flower in 100 ml of water at 60°C for 2 hours, may be contained in the food or beverage in an amount of preferably 1% by weight or more as calculated on a dry weight basis.

In the present invention, when a *Curcuma zedoaeia Roscoe* extract is used as a composition containing an enhancer for HGF production, for instance, a hot-water extract of *Curcuma zedoaeia Roscoe,* for instance, a product obtained by heat-treating 20 g of *Curcuma zedoaeia Roscoe* in 100 ml of water at 60°C for 2 hours, may be contained in the food or beverage in an amount of preferably 0.001% by weight or more as calculated on a dry weight basis.

In the present invention, when a plum extract is used as a composition containing an enhancer for HGF production, for instance, an ethanol extract of plum, for instance, a product obtained by extraction treatment of 60 g of plum with 100 ml of ethanol, may be contained in the food or beverage in an amount of preferably 0.1% by weight or more as calculated on a dry weight basis. In addition, when 5-caffeoyl-quinic acid or 4-caffeoyl-quinic acid in the extract is used as an enhancer for HGF production, it is suitable that 5-caffeoyl-quinic acid or 4-caffeoyl-quinic acid is contained in the food or beverage in an amount of preferably 0.01% by weight or more.

In the present invention, when a broccoli extract is used as a composition containing an enhancer for HGF production, for instance, a 50% ethanol extract of broccoli, for instance, a product obtained by extraction treatment of 100 g of the broccoli with 100 ml of a 50% ethanol, may be contained in the food or beverage in an amount of preferably 0.2% by weight or more as calculated on a dry weight basis.

In the present invention, when a *Chrysanthemum coronarium* extract is used as a composition containing an enhancer for HGF production, for instance, a hot water extract of *Chrysanthemum coronarium*, for instance, a product obtained by washing 10 g of the *Chrysanthemum coronarium* powder with ethanol, and extracting washed powder with 100 ml of water at 60°C for 1 hour may be contained in the food or beverage in an amount of preferably 0.1% by weight or more as calculated on a dry weight basis.

In addition, it is suitable that for instance, an ethanol extract of *Chrysanthemum coronarium*, for instance, an extract obtained by homogenizing 50 g of *Chrysanthemum coronarium* with one liter of a 80% ethanol, is contained in the food or beverage in an amount of 0.1% by weight or more. In addition, when 3,5-dicaffeoyl-quinic acid in the extract is contained, 3,5-dicaffeoyl-quinic acid is contained in the food or beverage in an amount of preferably 0.0005% by weight or more.

When a beer, for instance, a beer, a low-malt beer or a nonalcoholic beer beverage, is used as a composition containing an enhancer for HGF production, it is suitable that the beer is contained in the food or beverage in an amount of preferably 2% by weight or more as calculated on a dry weight basis.

In the present invention, when a soybean extract is used as a composition containing an enhancer for HGF production, there may be used, for instance, a hot water extract of a soybean-derived substance, for instance, an extract obtained by subjecting each of soybean embryo, seed coat of soybean, or soyameal to ethanol washing, and thereafter extracting the washed product with water at 60°C for 2 hours. Further, there can be used each product obtained by fractionating this extract into an ethanol-soluble fraction and an ethanol-insoluble fraction.

For instance, 10 g of thinly cut pieces of soybean embryo are washed with ethanol and then extracted with 200 ml of water at 60°C for 2 hours. The 2.5-fold amount of ethanol is added to the resulting extract, so that the extract can be separated into an ethanol-soluble fraction and an ethanol-insoluble fraction. It is suitable that the ethanol-soluble fraction is contained in the food or beverage in an amount of preferably 0.02% by weight or more, as calculated on a dry basis, and that the ethanol-insoluble fraction is contained in the food or beverage in an amount of preferably 0.01% by weight or more.

For instance, 10 g of thinly cut pieces of seed coat of soybean are washed with ethanol and then extracted with 70 ml of water at 60°C for 2 hours. The 2.5-fold amount of ethanol is added to the resulting extract, so that the extract can be separated into an ethanol-soluble fraction and an ethanol-insoluble fraction. It is suitable that the ethanol-soluble fraction is contained in the food or beverage in an amount of preferably 0.01% by weight or more, as calculated on a dry basis, and that the ethanol-insoluble fraction is contained in the food or beverage in an amount of preferably 0.003% by weight or more.

For instance, 10 g of thinly cut pieces of soyameal are washed with ethanol and then extracted with 100 ml of water at 60°C for 2 hours. The 2.5-fold amount of ethanol is added to the resulting extract, so that the extract can be separated into an ethanol-soluble fraction and an ethanol-insoluble fraction. It is suitable that the ethanol-soluble fraction is contained in the food or beverage in an amount of preferably 0.01% by weight or more, as calculated on a dry basis, and that the ethanol-insoluble fraction is contained in the food or beverage in an amount of preferably 0.005% by weight or more.

In the present invention, when a mulukhiya extract is used as a composition containing an enhancer for HGF production, there can be used, for instance, a hot water extract of mulukhiya leaves, for instance, an extract obtained by subjecting 10 g of mulukhiya powder to ethanol washing, subjecting the washed product to extraction treatment with 80 ml of water at 60°C for 2 hours, and adding the 2.5-fold amount of ethanol to the resulting extract, thereby separating the extract into an ethanol-soluble fraction and an ethanol-insoluble fraction. It is suitable that the ethanol-soluble fraction is contained in the food or beverage in an amount of preferably 0.0002% by weight or more as calculated on a dry basis, and that the ethanol-insoluble fraction is contained in the food or beverage in an amount of preferably 0.0004% by weight or more.

In the present invention, when a rice bran extract is used as a composition containing an enhancer for HGF production, there can be used, for instance, a hot water extract of rice bran, for instance, an extract obtained by subjecting 10 g of rice bran to ethanol washing, subjecting the washed product to extraction treatment with 100 ml of water at 60°C for 2 hours, and adding the 2.5-fold amount of ethanol to the resulting extract, thereby separating the extract into an ethanol-soluble fraction and an ethanol-insoluble fraction. It is suitable that the ethanol-soluble fraction is contained in the food or beverage in an amount of preferably 0.1% by weight or more as calculated on a dry basis.

In the present invention, when a *Carthamus tinctorius* pigment is used as a composition containing an enhancer for NGF production, it is suitable that the pigment is contained in the food or beverage in an amount of preferably 0.1% by weight or more.

In the present invention, when safflomin A derived from *Carthamus tinctorius* is used as an enhancer for NGF production, it is suitable that the safflomin A is contained in the food or beverage in an amount of preferably 0.3% by weight or more.

In the present invention, when a Chrysanthemum flower extract is used as a composition containing an enhancer for NGF production, for instance, a hot-water extract of Chrysanthemum flower , for instance, a product obtained by heat-treating 10 g of the Chrysanthemum flower in 100 ml of water at 60°C for 2 hours, may be contained in the food or beverage in an amount of preferably 2% by weight or more as calculated on a dry weight basis. Also, in the present invention, when a guaianolide derived from Chrysanthemum flower is used as an enhancer for NGF production, it is suitable that the guaianolide is contained in the food or beverage in an amount of preferably 0.002% by weight or more.

In the present invention, when an extract from *Angelica keiskei* koidz. is used as a composition containing an enhancer for NGF production, for instance, a hot-water extract of Chrysanthemum flower, for instance, a product obtained by heat-treating 10 g of leaf and stem portions of *Angelica keiskei* koidz. in 200 ml of water at 60°C for 2 hours, may be contained in the food or beverage in an amount of preferably 0.04% by weight or more as calculated on a dry weight basis. In the present invention, when an extract from root portions of *Angelica keiskei* koidz. is used as a composition containing an enhancer for NGF production, it is suitable that, for instance, a product obtained by heat-treating 10 g of root portions of *Angelica keiskei* koidz. in 200 ml of water at 60°C for 2 hours, is contained in the food or beverage in an amount of preferably 0.06% by weight or more as calculated on a dry weight basis.

In the present invention, when xanthoangelol derived from *Angelica keiskei* koidz. is used as an enhancer for NGF production, it is suitable that the xanthoangelol is contained in the food or beverage in an amount of preferably 0.001% by weight or more.

In the present invention, when 3'-O-β-D-glucopyranoyl khellactone derived from *Angelica keiskei* koidz. is used as an enhancer for NGF production, it is suitable that 3'-O-β-D-glucopyranoyl khellactone is contained in the food or beverage in an amount of preferably 0.005% by weight or more.

In the present invention, when 7-O-β-D-glucopyranosyloxy-8-prenylcoumarin derived from *Angelica keiskei* koidz. is used as an enhancer for NGF production, it is suitable that 7-O-β-D-glucopyranosyloxy-8-prenylcoumarin is contained in the food or beverage in an amount of preferably 0.003% by weight or more.

In the present invention, when caffeic acid methyl ester derived from *Angelica keiskei* koidz. is used as an enhancer for NGF production, it is suitable that caffeic acid methyl ester is contained in the food or beverage in an amount of preferably 0.002% by weight or more.

In the present invention, when 8-carboxyl-3-hydroxy-5-methoxyl-2-dimethylchroman derived from *Angelica keiskei* koidz. is used as an enhancer for NGF production, it is suitable that 8-carboxyl-3-hydroxy-5-methoxyl-2-dimethylchroman is contained in the food or beverage in an amount of preferably 0.0003% by weight or more.

In the present invention, when 7-β-D-glucopyranosyloxy-6-prenylcoumarin derived from *Angelica keiskei* koidz. is used as an enhancer for NGF production, it is suitable that 7-β-D-glucopyranosyloxy-6-prenylcoumarin is contained in the food or beverage in an amount of preferably 0.03% by weight or more.

In the present invention, when 4'-O-angeloyl-3'-O-[6-O-(β-D-glucopyranosyl)-β-D-glucopyranosyl]-khellactone derived from *Angelica keiskei* koidz. is used as an enhancer for NGF production, it is suitable that 4'-O-angeloyl-3'-O-[6-O-(β-D-glucopyranosyl)-β-D-glucopyranosyl]-khellactone is contained in the food or beverage in an amount of preferably 0.04% by weight or more.

In the present invention, when caffeic acid ethyl ester derived from *Angelica keiskei* koidz. is used as an enhancer for NGF production, it is suitable that caffeic acid ethyl ester is contained in the food or beverage in an amount of preferably 0.04% by weight or more.

In the present invention, when an onionskin extract is used as an enhancer for NGF production, it is suitable that, for instance, an ethanol extract of onionskin, for instance, an extract obtained by extracting 25 g of onionskin with 500 ml of ethanol, is contained in the food or beverage in an amount of preferably 0.01% by weight or more as calculated on a dry weight basis.

In the present invention, when a biloba leaf extract is used as a composition containing an enhancer for NGF production, it is suitable that, for instance, a hot water extract of biloba tealeaves, for instance, an extract obtained by extracting 3 g of biloba tealeaves with 200 ml of water at 100°C for 1 hour, is contained in the food or beverage in an amount of preferably 0.07% by weight or more as calculated on a dry weight basis.

In the present invention, when a *Rosa* flower extract is used as an enhancer for NGF production, it is suitable that, for instance, a hot water extract of *Rosa* flower, for instance, an extract obtained by extracting 2 g of *Rosa* flower with 40 ml of water at 60°C for 1 hour, is contained in the food or beverage in an amount of preferably 0.008% by weight or more as calculated on a dry weight basis.

In the present invention, when xanthohumol derived from *Humulus lupulus* is used as an enhancer for NGF production, it is suitable that xanthohumol is contained in the food or beverage in an amount of preferably 0.0003% by weight or more.

In the present invention, when an extract derived from *Humulus lupulus* is used as a composition containing an enhancer for NGF production, it is suitable that the extract is contained in the food or beverage in an amount of preferably 0.0001% by weight or more, more preferably 0.001% by weight or more as calculated on a dry basis.

In the present invention, when xanthohumol B or xanthohumol D is used as an enhancer for NGF production, it is suitable that each is contained in the food or beverage in an amount of preferably 0.002% by weight or more.

In the present invention, when isoxanthohumol is used as an enhancer for NGF production, it is suitable that isoxanthohumol is contained in the food or beverage in an amount of preferably 0.008% by weight or more in each case.

In the present invention, when a beer, for instance, a beer, a low-malt beer or a nonalcoholic beer beverage, is used as a composition containing an enhancer for NGF production, it is suitable that the beer is contained in the food or beverage in an amount of preferably 2% by weight or more as calculated on a dry basis.

In the present invention, when an extract derived from *Humulus lupulus* is used as a composition containing an enhancer for NGF production, it is suitable that the extract derived from *Humulus lupulus* is contained so that isoxanthohumol is contained in the food or beverage in an amount of preferably 0.000004% by weight or more, and that xanthohumol is contained in an amount of preferably 0.00013% by weight or more.

In the present invention, when an extract derived from *Humulus lupulus* is used as a composition containing an enhancer for NGF production, for instance, an ethanol extract derived from *Humulus lupulus*, for instance, an extract obtained by extracting 50 g of powder of dry product of *Humulus lupulus* with one liter of ethanol, may be contained in the food or beverage in an amount of preferably 0.0001% by weight or more, more preferably 0.001% by weight or more.

In the present invention, when a *Curcuma zedoaeia Roscoe* extract is used as a composition containing an enhancer for NGF production, it is suitable that for instance, a hot-water extract of *Curcuma zedoaeia Roscoe,* for instance, a product obtained by heat-treating 5 g of *Curcuma zedoaeia Roscoe* in 25 ml of water at 60°C for 2 hours, is contained in the food or beverage in an amount of preferably 0.06% by weight or more as calculated on a dry weight basis.

In the present invention, when a soybean extract is used as a composition containing an enhancer for NGF production, there may be used, for instance, a hot water extract of a soybean-derived substance, for instance, an extract obtained by subjecting each of soybean embryo, seed coat of soybean, or soyameal to ethanol washing, and thereafter extracting the washed product with water at 60°C for 2 hours. Further, there can be used a product obtained by fractionating this extract into an ethanol-soluble fraction and an ethanol-insoluble fraction.

For instance, 10 g of thinly cut pieces of soybean embryo are washed with ethanol and then extracted with 200 ml of water at 60°C for 2 hours. The 2.5-fold amount of ethanol is added to the resulting extract, so that the extract can be separated into an ethanol-soluble fraction and an ethanol-insoluble fraction. It is suitable that the ethanol-soluble fraction is contained in the food or beverage in an amount of preferably 0.5% by weight or more as calculated on a dry weight basis, and that the ethanol-insoluble fraction is contained in the food or beverage in an amount of preferably 0.3% by weight or more.

For instance, 10 g of thinly cut pieces of seed coat of soybean are washed with ethanol and then extracted with 70 ml of water at 60°C for 2 hours. The 2.5-fold amount of ethanol is added to the resulting extract, so that the extract can be separated into an ethanol-soluble fraction and an ethanol-insoluble fraction. It is suitable that the ethanol-soluble fraction is contained in the food or beverage in an amount of preferably 0.3% by weight or more as calculated on a dry weight basis, and that the ethanol-insoluble fraction is contained in the food or beverage in an amount of preferably 0.06% by weight or more.

For instance, 10 g of thinly cut pieces of soyameal are washed with ethanol and then extracted with 100 ml of water at 60°C for 2 hours. The 2.5-fold amount of ethanol is added to the resulting extract, so that the extract can be separated into an ethanol-soluble fraction and an ethanol-insoluble fraction. It is suitable that the ethanol-soluble fraction is contained in the food or beverage in an amount of preferably 0.3% by weight or more as calculated on a dry weight basis, and that the ethanol-insoluble fraction is contained in the food or beverage in an amount of preferably 0.006% by weight or more.

In the present invention, when a mulukhiya extract is used as a composition containing an enhancer for NGF production, there can be used, for instance, a hot water extract of mulukhiya leaves, for instance, an extract obtained by subjecting 10 g of mulukhiya powder to ethanol washing, subjecting the washed product to extraction treatment with 80 ml of water at 60°C for 2 hours, and adding the 2.5-fold amount of ethanol to the resulting extract, thereby separating the extract into an ethanol-soluble fraction and an ethanol-insoluble fraction. It is suitable that the ethanol-soluble fraction is contained in the food or beverage in an amount of preferably 0.05% by weight or more, as calculated on a dry weight basis, and that the ethanol-insoluble fraction is contained in the food or beverage in an amount of preferably 0.0005% by weight or more.

In the present invention, when a rice bran extract is used as a composition containing an enhancer for NGF production, there can be used, for instance, a hot water extract of rice bran, for instance, an extract obtained by subjecting 10 g of rice bran to ethanol washing, subjecting the washed product to extraction treatment with 100 ml of water at 60°C for 2 hours, and adding the 2.5-fold amount of ethanol to the resulting extract, thereby separating the extract into an ethanol-soluble fraction and an ethanol-insoluble fraction. It is suitable that the ethanol-soluble fraction is contained in the food or beverage in an amount of 0.05% by weight or more, as calculated on a dry weight basis, and that the ethanol-insoluble fraction is contained in the food or beverage in an amount of 0.05% by weight or more.

For instance, the food or beverage for enhancing HGF production of the present invention can be produced by known methods as follows.
(1) An alcohol-containing beverage is prepared by extracting oolong tealeaves with an extraction solvent, and mixing the extract with ethyl alcohol and other ingredients. For instance, an alcohol-containing beverage in which the extract is formulated so as to make a 100-fold dilution of the extract can be prepared.
(2) Bamboo blade powder is extracted with an extraction solvent such as water, and a lyophilized product of the extract is prepared. An alcohol-containing beverage is prepared by the lyophilized product in the same manner as in item (1) above. For instance, by formulating the lyophilized product so as to have a concentration of 0.1 µg/ml, an alcohol-containing beverage containing 100 µg/ml isoorientin can be prepared.
(3) Onionskin is extracted with an extraction solvent such as water, and an alcohol-containing beverage is prepared by the extract in the same manner as in item (1) above. For instance, an alcohol-containing beverage in which the extract is formulated so as to make a 100-fold dilution of the extract can be prepared.
(4) Biloba tealeaves are extracted with an extraction solvent such as water (for instance, extracted at 100°C for 15 minutes), and a lyophilized product of the extract is prepared. An alcohol-containing beverage is prepared by the lyophilized product in the same manner as in item (1) above. For instance, an alcohol-containing beverage in which the lyophilized product is formulated so as to make a 100-fold dilution of the lyophilized product can be prepared.
(5) A dry product of *Artemisia L.* is extracted with an extraction solvent such as water (for instance, extracted at 60°C for 1 hour), and an alcohol-containing beverage is prepared by the extract in the same manner as in item (1) above. For instance, an alcohol-containing beverage in which 3,5-dicaffeoyl-quinic acid obtained from the extract is formulated so as to have a concentration of 5 µg/ml can be prepared. Also, an alcohol-containing beverage in which chlorogenic acid obtained from the extract is formulated so as to have a concentration of 40 µg/ml can be prepared.
(6) A dry product of *Angelica keiskei* koidz. (leaf stem portions) is extracted with an extraction solvent such as water (for instance, extracted at 60°C for 1 hour), and an alcohol-containing beverage is prepared by the extract in the same manner as in item (1) above. For instance, an alcohol-containing beverage in which chlorogenic acid obtained from the extract is formulated so as to have a concentration of 10 µg/ml can be prepared.
(7) A dry product of Chrysanthemum flower is extracted with an extraction solvent such as water (for instance, extracted at 60°C for 2 hours), and an alcohol-containing beverage is prepared by the extract in the same manner as in item (1) above. For instance, an alcohol-containing beverage in which the extract is formulated so as to make a 100-fold dilution of the extract can be prepared.
(8) A dry product of *Curcuma zedoaeia Roscoe* is extracted with an extraction solvent such as water (for instance, extracted at 60°C for 2 hours), and a lyophilized product of the extract is prepared. An alcohol-containing beverage is prepared by the lyophilized product in the same manner as in item (1) above. For instance, an alcohol-containing beverage in which the lyophilized product is formulated so as to have a concentration of 10 µg/ml can be prepared.
(9) Plum is homogenized with an extraction solvent such as ethanol, and the homogenate is filtered to obtain its filtrate. An alcohol-containing beverage is prepared by the resulting extract in the same manner as in item (1) above. For instance, an alcohol-containing beverage in which the extract is formulated so as to make a 1000-fold dilution of the extract can be prepared. In addition, an alcohol-containing beverage in which 5-caffeoyl-quinic acid and 4-caffeoyl-quinic acid obtained from the extract are formulated so as to have a concentration of 100 µg/ml each can be prepared.
(10) Broccoli is homogenized with an extraction solvent such as a 50% aqueous ethanol solution, the homogenate is filtered to obtain its filtrate, and the filtrate is concentrated. An alcohol-containing beverage is prepared by the resulting concentrate in the same manner as in item (1) above. For instance, an alcohol-containing beverage in which the concentrate is formulated so as to make a 1000-fold dilution of the concentrate can be prepared.
(11) A lyophilized product of *Chrysanthemum coronarium* is powdered, the powder is washed with an organic solvent such as ethanol, and thereafter the washed powder is extracted with an extraction solvent such as water (for instance, extracted at 60°C for 1 hour). An alcohol-containing beverage is prepared by the extract in the same manner as in item (1) above. For instance, an alcohol-containing beverage in which the extract is formulated so as to make a 1000-fold dilution of the extract can be prepared.
(12) *Chrysanthemum coronarium* is homogenized with an extraction solvent such as a 80% ethanol, the homogenate is filtered to obtain its filtrate, and the filtrate is concentrated. An alcohol-containing beverage is prepared by the resulting concentrate in the same manner as in item (1) above. For instance, an alcohol-containing beverage in which the concentrate is formulated so as to make a 1000-fold dilution of the concentrate can be prepared. In addition, an alcohol-containing beverage in which 3,5-dicaffeoyl-quinic acid obtained from the extract is formulated so as to have a concentration of 10 µg/ml can be prepared.
(13) A beer is concentrated, and an alcohol-containing beverage is prepared by the concentrate in the same manner as in item (1) above. For instance, an alcohol-containing beverage in which the concentrate is formulated so as to make a 100-fold dilution of the concentrate can be prepared.
(14) A nonalcoholic beer beverage is concentrated, and an alcohol-containing beverage is prepared by the concentrate in the same manner as in item (1) above. For instance, an alcohol-containing beverage in which the concentrate is formulated so as to make a 100-fold dilution of the concentrate can be prepared.
(15) Thinly cut pieces of soybean embryo are washed with an organic solvent such as ethanol, and thereafter the washed powder is extracted with an extraction solvent such as water (for instance, extracted at 60°C for 2 hours), to give an extract. The extract is filtered to obtain its filtrate, and thereafter an organic solvent, such as a 2.5-fold amount of ethanol, is added to the filtrate to separate into an organic solvent-soluble fraction and an organic solvent-insoluble fraction, and a dry product of each fraction is prepared. An alcohol-containing beverage is prepared by the dry product in the same manner as in item (1) above. For instance, an alcohol-containing beverage in which the dry product of an ethanol-soluble fraction is formulated so as to have a concentration of 200 µg/ml can be prepared. Also, an alcohol-containing beverage in which the dry product of an ethanol-insoluble fraction is formulated so as to have a concentration of 130 µg/ml can be prepared.
(16) Thinly cut pieces of seed coat of soybean are washed with an organic solvent such as ethanol, and thereafter the washed powder is extracted with an extraction solvent such as water (for instance, extracted at 60°C for 2 hours), to give an extract. The extract is filtered to obtain its filtrate, and thereafter an organic solvent, such as a 2.5-fold amount of ethanol, is added to the filtrate to separate into an organic solvent-soluble fraction and an organic solvent-insoluble fraction, and a dry product of each fraction is prepared. An alcohol-containing beverage is prepared by the dry product in the same manner as in item (1) above. For instance, an alcohol-containing beverage in which the dry product of an ethanol-soluble fraction is formulated so as to have a concentration of 130 µg/ml can be prepared. Also, an alcohol-containing beverage in which the dry product of an ethanol-insoluble fraction is formulated so as to have a concentration of 30 µg/ml can be prepared.
(17) Thinly cut pieces of soyameal are washed with an organic solvent such as ethanol, and thereafter the washed powder is extracted with an extraction solvent such as water (for instance, extracted at 60°C for 2 hours), to give an extract. The extract is filtered to obtain its filtrate, and thereafter an organic solvent, such as a 2.5-fold amount of ethanol, is added to the filtrate to separate into an organic solvent-soluble fraction and an organic solvent-insoluble fraction, and a dry product of each fraction is prepared. An alcohol-containing beverage is prepared by the dry product in the same manner as in item (1) above. For instance, an alcohol-containing beverage in which the dry product of an ethanol-soluble fraction is formulated so as to have a concentration of 100 µg/ml can be prepared. Also, an alcohol-containing beverage in which the dry product of an ethanol-insoluble fraction is formulated so as to have a concentration of 60 µg/ml can be prepared.
(18) A mulukhiya leaf powder is washed with an organic solvent such as ethanol, and thereafter the washed powder is extracted with an extraction solvent such as water (for instance, extracted at 60°C for 2 hours), to give an extract. The extract is filtered to obtain its filtrate, and thereafter an organic solvent, such as a 2.5-fold amount of ethanol, is added to the filtrate to separate into an organic solvent-soluble fraction and an organic solvent-insoluble fraction, and a dry product of each fraction is prepared. An alcohol-containing beverage is prepared by the dry product in the same manner as in item (1) above. For instance, an alcohol-containing beverage in which the dry product of an ethanol-soluble fraction is formulated so as to have a concentration of 2 µg/ml can be prepared. Also, an alcohol-containing beverage in which the dry product of an ethanol-insoluble fraction is formulated so as to have a concentration of 4 µg/ml can be prepared.
(19) Rice bran is washed with an organic solvent such as ethanol, and thereafter the washed powder is extracted with an extraction solvent such as water (for instance, extracted at 60°C for 2 hours), to give an extract. The extract is filtered to obtain its filtrate, and thereafter an organic solvent such as a 2.5-fold amount of ethanol is added to the filtrate to obtain an organic solvent-soluble fraction, and a dry product thereof is prepared. An alcohol-containing beverage is prepared by the dry product in the same manner as in item (1) above. For instance, an alcohol-containing beverage in which the dry product of an ethanol-soluble fraction is formulated so as to have a concentration of 1 mg/ml can be prepared.
(20) Each of the alcohol-containing beverages described in items (1) to (19) above is subjected to carbonation by a conventional method, whereby a carbonated type beverage can be prepared. In addition, for each beverage, a nonalcoholic type beverage in which the effective ingredient is contained in an amount of 20-folds the beverage described above can be prepared. These beverages containing an enhancer for growth factor production in a high concentration and/or at a high purity are preferable because they can show high enhancing action for HGF production.

In addition, for instance, the food or beverage for enhancing NGF production of the present invention can be produced by known methods as follows.
(1) An alcohol-containing beverage is prepared by mixing grapefruit fruit juice with ethyl alcohol and other ingredients. For instance, an alcohol-containing beverage in which the grapefruit fruit juice is formulated so as to make a 6-fold dilution of the fruit juice can be prepared.
(2) An alcohol-containing beverage is prepared by using a *Carthamus tinctorius* pigment in the same manner as in item (1) above. For instance, an alcohol-containing beverage containing safflomin A in a high concentration, in which the pigment is formulated so as to have a concentration of 1.25 mg/ml, can be prepared.
   A dry product of *Carthamus tinctorius* is extracted with an extraction solvent such as water (for instance, extracted at 60°C for 2 hours), a lyophilized product of the extract is prepared, and an alcohol-containing beverage is prepared by the lyophilized product in the same manner as in item (1) above. For instance, an alcohol-containing beverage in which the lyophilized product is formulated so that the concentration of safflomin A contained in the lyophilized product is 3 mg/ml can be prepared.
(3) A dry product of Chrysanthemum flower is extracted with an extraction solvent such as water (for instance, extracted at 60°C for 2 hours), a lyophilized product of the extract is prepared, and an alcohol-containing beverage is prepared by the lyophilized product in the same manner as in item (1) above. For instance, an alcohol-containing beverage in which the lyophilized product is formulated so as to have a concentration of 20 mg/ml can be prepared. In addition, an alcohol-containing beverage in which the lyophilized product is formulated so that the concentration of guaianolide contained in the lyophilized product is 20 µg/ml can be prepared.
(4) A dry product of *Angelica keiskei* koidz. (leaf stem portions) is extracted with an extraction solvent such as water (for instance, extracted at 60°C for 2 hours), a lyophilized product of the extract is prepared, and an alcohol-containing beverage is prepared by the lyophilized product in the same manner as in item (1) above. For instance, an alcohol-containing beverage in which the lyophilized product is formulated so as to have a concentration of 400 µg/ml can be prepared.
   In addition, a dry product of *Angelica keiskei* koidz. (root portions) is extracted with an extraction solvent such as water (for instance, extracted at 60°C for 2 hours), a lyophilized product of the extract is prepared, and an alcohol-containing beverage is prepared by the lyophilized product in the same manner as in item (1) above. For instance, an alcohol-containing beverage in which the lyophilized product is formulated so as to have a concentration of 600 µg/ml can be prepared.
   Also, an alcohol-containing beverage in which the lyophilized product is formulated so that the concentration of xanthoangelol contained in the lyophilized product is 10 µg/ml can be prepared.
   An alcohol-containing beverage in which the lyophilized product is formulated so that the concentration of 3'-O-β-D-glucopyranoyl khellactone contained in the lyophilized product is 50 µg/ml can be prepared.
   An alcohol-containing beverage in which the lyophilized product is formulated so that the concentration of 7-O-β-D-glucopyranosyloxy-8-prenylcoumarin contained in the lyophilized product is 30 µg/ml can be prepared.
   An alcohol-containing beverage in which the lyophilized product is formulated so that the concentration of caffeic acid methyl ester contained in the lyophilized product is 20 µg/ml can be prepared.
   An alcohol-containing beverage in which the lyophilized product is formulated so that the concentration of 8-carboxyl-3-hydroxy-5-methoxyl-2-dimethylchroman contained in the lyophilized product is 3 µg/ml can be prepared.
   An alcohol-containing beverage in which the lyophilized product is formulated so that the concentration of 7-β-D-glucopyranosyloxy-6-prenylcoumarin contained in the lyophilized product is 300 µg/ml can be prepared.
   An alcohol-containing beverage in which the lyophilized product is formulated so that the concentration of 4'-O-angeloyl-3'-O-[6-O-(β-D-glucopyranosyl)-β-D-glucopyranosyl]-khellactone contained in the lyophilized product is 400 µg/ml can be prepared.
   An alcohol-containing beverage in which the lyophilized product is formulated so that the concentration of caffeic acid ethyl ester contained in the lyophilized product is 30 µg/ml can be prepared.
(5) Onionskin is extracted with an extraction solvent such as a 95% by volume ethyl alcohol, a lyophilized product of the extract is prepared, and an alcohol-containing beverage is prepared by using the lyophilized product in the same manner as in item (1) above. For instance, an alcohol-containing beverage in which the lyophilized product is formulated so as to have a concentration of 100 µg/ml can be prepared.
(6) Biloba tealeaves are extracted with an extraction solvent such as water (for instance, extracted at 100°C for 1 hour), and a lyophilized product of the extract is prepared. An alcohol-containing beverage is prepared by the lyophilized product in the same manner as in item (1) above. For instance, an alcohol-containing beverage in which the lyophilized product is formulated so as to have a concentration of 700 µg/ml can be prepared.
(7) *Rosa* flower is extracted with an extraction solvent such as water (for instance, extracted at 60°C for 1 hour), and a lyophilized product of the extract is prepared. An alcohol-containing beverage is prepared by the lyophilized product in the same manner as in item (1) above. For instance, an alcohol-containing beverage in which the lyophilized product is formulated so as to have a concentration of 80 µg/ml can be prepared.
(8) An alcohol-containing beverage is prepared by using an extract derived from *Humulus lupulus* in accordance with a conventional method in the same manner as in item (1) above. For instance, an alcohol-containing beverage in which the extract derived from *Humulus lupulus* is formulated so that the concentration of xanthohumol contained in the extract derived from *Humulus lupulus* is 3 µg/ml can be prepared.
(9) An alcohol-containing beverage is prepared by using an extract derived from *Humulus lupulus* in accordance with a conventional method in the same manner as in item (1) above. For instance, an alcohol-containing beverage in which the extract derived from *Humulus lupulus* is formulated so as to have a concentration of 20 µg/ml on a dry weight basis can be prepared.
(10) An alcohol-containing beverage is prepared by using an extract derived from *Humulus lupulus* in accordance with a conventional method in the same manner as in item (1) above. For instance, an alcohol-containing beverage in which the extract derived from *Humulus lupulus* is formulated so that the concentration of both xanthohumol B and xanthohumol D contained in the total extract derived from *Humulus lupulus* is 20 µg/ml can be prepared.
(11) An alcohol-containing beverage is prepared by using an extract derived from *Humulus lupulus* in accordance with a conventional method in the same manner as in item (1) above. For instance, an alcohol-containing beverage in which the extract derived from *Humulus lupulus* is formulated so that the concentration of isoxanthohumol contained in the extract derived from *Humulus lupulus* is 80 µg/ml can be prepared.
(12) A beer is concentrated, and an alcohol-containing beverage is prepared by using the concentrate in the same manner as in item (1) above. For instance, an alcohol-containing beverage in which the concentrate is formulated so as to make a 10-fold dilution of the concentrate can be prepared.
(13) A nonalcoholic beer beverage is concentrated, and an alcohol-containing beverage is prepared by using the concentrate in the same manner as in item (1) above. For instance, an alcohol-containing beverage in which the concentrate is formulated so as to make a 10-fold dilution of the concentrate can be prepared.
(14) An alcohol-containing beverage is prepared by using an extract derived from *Humulus lupulus* in accordance with a conventional method in the same manner as in item (1) above. For instance, an alcohol-containing beverage in which the extract derived from *Humulus lupulus* is formulated so that the concentration of xanthohumol contained in the extract derived from *Humulus lupulus* is 0.04 µg/ml, and that the concentration of isoxanthohumol is 1.3 µg/ml can be prepared.
   Also, an alcohol-containing beverage in which the extract derived from *Humulus lupulus* is formulated so that the concentration of xanthohumol contained in the extract derived from *Humulus lupulus* is 0.9 µg/ml, and that the concentration of isoxanthohumol is 4 µg/ml can be prepared.
(15) A dry product of *Humulus lupulus* is powdered, the resulting powder is extracted with an extraction solvent such as ethanol (for instance, extracted at 60°C for 1 hour), and the extract is concentrated. An alcohol-containing beverage is prepared by using the concentrate in the same manner as in item (1) above. For instance, an alcohol-containing beverage in which the concentrate is formulated so as to have a concentration of 20 µg/ml on a dry weight basis can be prepared.
(16) Thinly cut pieces of *Curcuma zedoaeia Roscoe* are extracted with an extraction solvent such as water (for instance, extracted at 60°C for 2 hours), and a lyophilized product of the extract is prepared. An alcohol-containing beverage is prepared by the lyophilized product in the same manner as in item (1) above. For instance, an alcohol-containing beverage in which the lyophilized product is formulated so as have a concentration of 600 µg/ml can be prepared.
(17) Thinly cut pieces of soybean embryo are washed with an organic solvent such as ethanol, and thereafter the washed powder is extracted with an extraction solvent such as water (for instance, extracted at 60°C for 2 hours), to give an extract. The extract is filtered to obtain its filtrate, and thereafter an organic solvent, such as a 2.5-fold amount of ethanol, is added to the filtrate to separate into an organic solvent-soluble fraction and an organic solvent-insoluble fraction, and a dry product of each fraction is prepared. An alcohol-containing beverage is prepared by using the dry product in the same manner as in item (1) above. For instance, an alcohol-containing beverage in which the dry product of an ethanol-soluble fraction is formulated so as to have a concentration of 5 mg/ml can be prepared. Also, an alcohol-containing beverage in which the dry product of an ethanol-insoluble fraction is formulated so as to have a concentration of 3 mg/ml can be prepared.
(18) Thinly cut pieces of seed coat of soybean are washed with an organic solvent such as ethanol, and thereafter the washed powder is extracted with an extraction solvent such as water (for instance, extracted at 60°C for 2 hours), to give an extract. The extract is filtered to obtain its filtrate, and thereafter an organic solvent, such as a 2.5-fold amount of ethanol, is added to the filtrate to separate into an organic solvent-soluble fraction and an organic solvent-insoluble fraction, and a dry product of each fraction is prepared. An alcohol-containing beverage is prepared by using the dry product in the same manner as in item (1) above. For instance, an alcohol-containing beverage in which the dry product of an ethanol-soluble fraction is formulated so as to have a concentration of 3 mg/ml can be prepared. Also, an alcohol-containing beverage in which the dry product of an ethanol-insoluble fraction is formulated so as to have a concentration of 600 µg/ml can be prepared.
(19) Thinly cut pieces of soyameal are washed with an organic solvent such as ethanol, and thereafter the washed powder is extracted with an extraction solvent such as water (for instance, extracted at 60°C for 2 hours), to give an extract. The extract is filtered to obtain its filtrate, and thereafter an organic solvent, such as a 2.5-fold amount of ethanol, is added to the filtrate to separate into an organic solvent-soluble fraction and an organic solvent-insoluble fraction, and a dry product of each fraction is prepared. An alcohol-containing beverage is prepared by using the dry product in the same manner as in item (1) above. For instance, an alcohol-containing beverage in which the dry product of an ethanol-soluble fraction is formulated so as to have a concentration of 3 mg/ml can be prepared. Also, an alcohol-containing beverage in which the dry product of an ethanol-insoluble fraction is formulated so as to have a concentration of 60 µg/ml can be prepared.
(20) Mulukhiya leaf powder is washed with an organic solvent such as ethanol, and thereafter the washed powder is extracted with an extraction solvent such as water (for instance, extracted at 60°C for 2 hours), to give an extract. The extract is filtered to obtain its filtrate, and thereafter an organic solvent, such as a 2.5-fold amount of ethanol, is added to the filtrate to separate into an organic solvent-soluble fraction and an organic solvent-insoluble fraction, and a dry product of each fraction is prepared. An alcohol-containing beverage is prepared by using the dry product in the same manner as in item (1) above. For instance, an alcohol-containing beverage in which the dry product of an ethanol-soluble fraction is formulated so as to have a concentration of 500 µg/ml can be prepared. Also, an alcohol-containing beverage in which the dry product of an ethanol-insoluble fraction is formulated so as to have a concentration of 5 µg/ml can be prepared.
(21) Rice bran is washed with an organic solvent such as ethanol, and thereafter the washed powder is extracted with an extraction solvent such as water (for instance, extracted at 60°C for 2 hours), to give an extract. The extract is filtered to obtain its filtrate, and thereafter an organic solvent such as a 2.5-fold amount of ethanol is added to the filtrate to separate into an organic solvent-soluble fraction and an organic solvent-insoluble fraction, and a dry product of each fraction is prepared. An alcohol-containing beverage is prepared by using the dry product in the same manner as in item (1) above. For instance, an alcohol-containing beverage in which the dry product of an ethanol-soluble fraction is formulated so as to have a concentration of 500 µg/ml can be prepared. Also, an alcohol-containing beverage in which the dry product of an ethanol-insoluble fraction is formulated so as to have a concentration of 500 µg/ml can be prepared.
(22) Each of the alcohol-containing beverages described in items (1) to (21) above is subjected to carbonation by a conventional method, whereby a carbonated type beverage can be prepared. In addition, for each beverage, a nonalcoholic type beverage in which the effective ingredient is contained in an amount of 20-folds the beverage described above can be prepared. These beverages containing an enhancer for growth factor production in a high concentration and/or at a high purity are preferable because they can show high enhancing action for NGF production.

In sum, according to the present invention, there is provided a food or beverage for enhancing HGF production or a food or beverage for enhancing NGF production, comprising as an effective ingredient a plant-derived enhancer for HGF production or enhancer for NGF production.

According to the present invention, there is provided a food or beverage comprising an effective amount of an enhancer for NGF production, for instance, a food or beverage for enhancing NGF production, comprising an extract derived from *Humulus lupulus*, in which the content of xanthohumol in the food or beverage is preferably 0.00004% by weight or more and/or the content of isoxanthohumol is preferably 0.00013% by weight or more. Such a food or beverage is very useful for ameliorating symptoms and preventing diseases such as a disease requiring NGF production, including, for instance, Parkinson's disease, and senile dementia such as Alzheimer's disease and cerebrovascular dementia.

In addition, according to the present invention, there is provided an alcohol-containing beverage comprising a plant-derived effective ingredient having enhancing action for growth factor production in a high concentration. In the present specification, the term alcohol-containing beverage refers to a beverage containing ethyl alcohol. The ethyl alcohol content in the alcohol-containing beverage is, for instance, 0.1 to 50% by volume. In addition, there is also provided a food such as whiskey-containing bonbon using the alcohol-containing beverage of the present invention.

Preferred examples of the alcohol-containing beverage include an alcohol-containing beverage containing a plant-derived effective ingredient having enhancing action for growth factor production in a high concentration and/or at a high purity, including for instance, *sake,* artificially produced *sake,* Chinese liquor, baijiu, wine, whiskey, Japanese distilled liquor *(shochu),* vodka, brandy, gin, rum, beer, low-malt beer, refreshing alcoholic beverages, sour liquor, cocktail, fruit liquor, liqueur, and the like, containing a plant-derived effective ingredient having enhancing action for growth factor production.

Usually, the ethyl alcohol concentration of the above-mentioned alcohol-containing beverage is preferably from 0.1 to 12% by volume, more preferably from 0.5 to 8% by volume, still more preferably from 1 to 6% by volume. The alcohol used is not particularly limited. For instance, each of a brewing alcohol, spirit such as rum, vodka or gin, whiskey, brandy, Japanese distilled liquor (*shochu*), or the like can be used alone or in combination.

In addition, the alcohol-containing beverage can be prepared by mixing the plant-derived effective ingredient having enhancing action for growth factor production with ethyl alcohol and other ingredients as desired. Alternatively, the alcohol-containing beverage of the present invention can be also prepared by immersing a desired plant in an alcohol-containing beverage having an ethyl alcohol concentration within the above-mentioned desired range, thereby extracting an effective ingredient having enhancing action for growth factor production from the plant into the beverage.

The alcohol-containing beverage of the present invention is a beverage for enhancing growth factor production. In the preparation of the above beverage of the present invention, raw materials and methods conventionally used in the preparation can be used. There can be used as raw materials, for instance, any drinkable and edible substances including food materials and food additives such as sweeteners, food colorings, food souring, seasonings, emulsifiers; functional food materials such as vitamin enrichments and dietary fibers; spices, flavors, thickeners, and minerals.

The pH of the alcohol-containing beverage of the present invention can be selected at a useful range in which a food is taken, and the pH can be selected within the range of 2 to 7. In the refreshing alcoholic beverage of the present invention, the presence or absence of carbonation is optional. When carbonated, the gas volume of the manufactured product is preferably 1 or more, more preferably within the range of from 1.3 to 3. In addition, in the alcohol-containing beverage of the present invention, fruit juice, vegetable juice, sarcocarp, or vegetable pieces can be optionally added.

Also, according to the present invention, there is provided a food or beverage for enhancing growth factor production, comprising xanthohumol in an amount of 0.0003% by weight or more in the food or beverage.

Xanthohumol used in the present invention is contained in beers and nonalcoholic beers which are conventional beverages containing extracts derived from *Humulus lupulus*. However, the xanthohumol content in a beer manufactured by the company A is 0.73 µg/100 ml, a beer manufactured by the company B is 2.1 µg/100 ml, a beer manufactured by the company C is 0.70 µg/100 ml, so that none of the beers fall under the xanthohumol content range for the beverage as defined in the present invention of 0.00007% by weight or more.

The method for producing the food or beverage of the present invention in which xanthohumol is contained in an amount of 0.00007% by weight or more is not particularly limited. For instance, an extract derived from *Humulus lupulus* containing xanthohumol may be added to a beer. Also, a nonalcoholic beer may be used as a raw material. Also, an extract derived from *Humulus lupulus* containing xanthohumol may be used for a raw material for preparing a refreshing alcohol beverage, a luxury beverage or the like. The use *of Humulus lupulus* itself in place of an extract derived from *Humulus lupulus* is as a matter of course within the scope of the present invention. An extract derived from *Humulus lupulus* itself can be also used as a composition for enhancing growth factor production of the present invention.

The xanthohumol content of the food or beverage of the present invention may be determined by the physiological effects and sensory results. The xanthohumol may be contained in an amount of preferably 0.00007% by weight or more, and it is preferable that the content in the food or beverage is usually within the range of from 0.0001 to 0.001% by weight.

Also, according to the present invention, there is provided a food or beverage for enhancing growth factor production, comprising as an effective ingredient a food, beverage or processed product thereof comprising an enhancer for growth factor production. The food or beverage comprising an enhancer for growth factor production is exemplified by a beer. The beer is exemplified by beers, low-malt beer, nonalcoholic beer beverages, concentrates thereof, dilutions thereof and a beverage containing an extract from *Humulus lupulus.* For instance, a beer or low-malt beer may be used as a beverage for enhancing growth factor production, or as a raw material for a food or beverage. Also, a processed product of a beer, for instance, a concentrate or a dilution may be used as a raw material for the food or beverage.

Further, the present invention relates to a food or beverage for enhancing growth factor production, comprising an extract from *Humulus lupulus.* As the extract from *Humulus lupulus*, a commercially available extract from *Humulus lupulus* can be used. Usually, the extract may be contained in the food or beverage in an amount of preferably 0.0001% by weight or more, more preferably from 0.002 to 0.02% by weight. In any case, the enhancing activity for growth factor production in the extract from *Humulus lupulus* is assayed by the method disclosed according to the present invention (for instance, those disclosed in item (1) of Example 4, and in Example 13), and the content of the extract from *Humulus lupulus* is determined from the viewpoints of sensory and physiological activity, to prepare a food or beverage of the present invention.

According to the present invention, there is provided a food or beverage for enhancing growth factor production, comprising an extract from *Humulus lupulus*, in which xanthohumol is contained in an amount of preferably 0.00000006% by weight or more, and isoxanthohumol is contained in an amount of preferably 0.000005% by weight or more.

In addition, according to the present invention, there is provided a concentrated-type food or beverage for enhancing growth factor production, comprising an extract from *Humulus lupulus,* in which xanthohumol is contained in an amount of preferably 0.0000032% by weight or more, and isoxanthohumol is contained in an amount of preferably 0.00013% by weight or more.

Further, according to the present invention, there is provided a food or beverage, comprising an extract from *Humulus lupulus*, in which xanthohumol is contained in an amount of preferably 0.00007% by weight or more, and isoxanthohumol is contained in an amount of preferably 0.0004% by weight or more, wherein the food or beverage has a highly active enhancing action for growth factor production.

The food or beverage of the present invention does not have any particular limitation on its shape, as long as the food or beverage comprises the plant-derived composition for enhancing growth factor production having enhancing action for growth factor production in an amount necessary for exhibiting its physiological functions. Such shapes also include orally taken shapes such as tablets, granules and capsules.

In addition, the present invention provides a feed for an organism having enhancing action for growth factor production, comprising as an effective ingredient an enhancer for growth factor production. In the same manner as the above-described food or beverage, the feed includes a feed for enhancing growth factor production, comprising the above-mentioned composition for enhancing growth factor production; a feed characterized in that the feed comprises the above-mentioned enhancer for growth factor production in a high concentration and/or at a high purity; a feed comprising the above-mentioned composition for enhancing growth factor production in a high concentration and/or at a high purity; a feed for enhancing nerve growth factor production, comprising xanthohumol in an amount of 0.00007% by weight or more; a feed for enhancing growth factor production, comprising a feed or a processed product thereof comprising the above-mentioned enhancer for growth factor production (preferably an embodiment containing a beer); and a feed for enhancing growth factor production, comprising an extract from *Humulus lupulus.* The enhancer for growth factor production used, like in the above-mentioned food or beverage, is preferably one or more compounds selected from the group consisting of chlorogenic acid, dicaffeoyl-quinic acid, caffeoyl-quinic acid, isoorientin, safflomin A, guaianolide, xanthoangelol, 3'-O-β-D-glucopyranoyl khellactone, 7-O-β-D-glucopyranosyloxy-8-prenylcoumarin, caffeic acid methyl ester, caffeic acid ethyl ester, 8-carboxyl-3-hydroxy-5-methoxyl-2-dimethylchroman, 7-β-D-glucopyranosyloxy-6-prenylcoumarin, 4'-O-angeloyl-3'-O-[6-O-(β-D-glucopyranosyl)-β-D-glucopyranosyl]-khellactone, isoxanthohumol, xanthohumol B, xanthohumol D, and xanthohumol.

In another embodiment, the present invention also provides a method of feeding an organism, characterized by administering the above-mentioned effective ingredient to the organism. In still another embodiment, the present invention provides an organism feeding agent characterized in that the organism feeding agent comprises the above-mentioned effective ingredient.

In these inventions, the organism includes, for instance, culturing or breeding animals, pet animals, and the like. The culturing or breeding animal is exemplified by cattle, experimental animals, poultry, pisces, crustaceae or shellfish. The feed is exemplified by a feed for sustenance of and/or improvement in physical conditions. The organism feeding agent includes immersion agents, feed additives, and beverage additives.

According to these inventions, the same effects can be expected to be exhibited as those of the above-mentioned therapeutic agent or prophylactic agent of the present invention, on the basis of the enhancing action for growth factor production of the above-mentioned effective ingredient used in the present invention, in the organism exemplified above for applying these. In other words, there can be expected exhibition of therapeutic or prophylactic effect for a disease requiring enhancing action for growth factor production in the organism.

The above-mentioned effective ingredient used in the present invention is usually administered in an amount of preferably from 0.01 to 2000 mg per 1 kg of the body weight of the subject organism per day. The administration can be made by adding and mixing the effective ingredient in a raw material for an artificially formulated feed, or mixing the effective ingredient with a powder raw material for an artificially formulated feed, and thereafter further adding and mixing the resulting mixture with other raw materials, wherein the artificially formulated feed is applied to a subject organism. In addition, the content of the above-mentioned effective ingredient in the feed is not particularly limited. The content of the effective ingredient can be appropriately set in accordance with its purposes, and an appropriate proportion in the feed is from 0.001 to 15% by weight.

The artificially formulated feed includes feeds using as raw materials animal-derived raw materials such as fish meal, casein, and squid meal; plant-derived raw materials such as soybean grounds, flour, and starch; microorganism raw materials such as yeasts for feed; animal fats and oils such as cod-liver oil and squid-liver oil; vegetable fats and oils such as soybean oil and rapeseed oil; and other raw materials such as vitamins, minerals, amino acids, and antioxidants; and the like. In addition, feeds for fish such as fish minced meat are also included.

The method for preparing the feed of the present invention is not particularly limited. In addition, the formulation may be in accordance with those of general feeds, as long as the effective ingredient according to the present invention having enhancing action for growth factor production is contained in the feed produced.

Also, the above-mentioned effective ingredient having enhancing activity for growth factor production according to the present invention can be administered by directly adding the above-mentioned effective ingredient to water, seawater, or the like in a pool, a water tank, a water reservoir, or a feeding range, and immersing a subject organism into the resulting solution. The immersion method is especially effective when the amount of intake of the feed of the subject organism is lowered. The concentration of the effective ingredient according to the present invention having enhancing action for growth factor production in water or seawater is not particularly limited, and the effective ingredient may be used in accordance with its purposes. It is appropriate that the concentration is preferably from 0.00001 to 1% by weight.

Also, a beverage comprising the above-mentioned effective ingredient according the present invention, having enhancing action for growth factor production may be given to a subject organism as a feeding drink. The concentration of the effective ingredient used in the present invention having enhancing action for growth factor production in the beverage is not particularly limited, and the effective ingredient may be used in accordance with its purposes. It is appropriate that the concentration is preferably from 0.0001 to 1% by weight. The organism feeding agent, for instance, an immersion agent, a feed additive, or a beverage additive comprising the above-mentioned effective ingredient according to the present invention having enhancing action for growth factor production may be prepared by known formulation and preparation method. The content of the effective ingredient in the organism feeding agent is not particularly limited, so long as the desired effects of the present invention can be obtained.

The organism to which the present invention can be applied is not limited. The culturing or breeding animals include cattle such as *Equus, Bos, Porcus, Ovis, Copra, Camelus,* and *Lama*; experimental animals such as mice, rats, guinea pigs, and rabbits; poultry such as *Chrysolophus,* ducks, *Meleagris*, and *Struthioniformes*; pisces such as *Pagrus, Oplegnathidae, Paralichthys*, plaice, *Seriola*, young *Seriola*, amberjack, *Thunna, Caranx delicatissimus, Plecoglossus, Salmo•Oncorhynchus, Fugu, Anguilla, Misguirus,* and *Parasilurus*; Crustaceae such as *Penaidae*, black tiger shrimp, *Penaeus roentalis*, and *Portulus trituberculatus*; and shellfish such as abalones (*awabi*), turban shells, scallops, and oysters; and the pet animals includes dogs, cats, and the like, so that the feed can be widely applied to animals on land and in water.

By allowing a subject organism to take the feed comprising the above-mentioned effective ingredient used in the present invention having enhancing action for growth factor production, or immersing a subject organism into a solution containing the above-mentioned effective ingredient used in the present invention having enhancing action for growth factor production, the physical conditions of the cattle, experimental animals, poultry, pisces, Caustacea, shellfish, pet animals or the like can be well sustained and ameliorated.

As described above, according to the present invention, there are provided various medicaments, foods, beverages, feeds and the like, having enhancing action for growth factor production, among which a composition for enhancing growth factor production, a therapeutic agent or prophylactic agent, and a food, beverage or feed, each comprising a guaianolide as an enhancer for growth factor production are especially preferable, from the viewpoints of exhibiting the desired effects of the present invention.

Further, as a still another embodiment, the present invention provides use of the above-mentioned effective ingredient according to the present invention in the preparation of a therapeutic agent or prophylactic agent for a disease requiring enhancement of growth factor production, an enhancing agent for growth factor production, or a food, beverage or feed for enhancing growth factor production. The use embodiments include use embodiments of the above-mentioned effective ingredient in the preparation of the therapeutic agent or prophylactic agent, the enhancing agent for growth factor production, or the food, beverage or feed for enhancing growth factor production of the present invention mentioned above. For instance, as the use of the above-mentioned effective ingredient in the preparation of a therapeutic agent or prophylactic agent for a disease requiring enhancement of growth factor production, or an enhancing agent for growth factor production, there are exemplified the use in the preparation of a solid agent such as a tablet, a granule, a powder, a fine powder, and a capsule, a liquid agent such as a common liquid agent, a suspension agent, or an emulsion agent, or a dry product which can be liquefied by adding an appropriate vehicle before use.

There are found no cases of death when the enhancer for growth factor production used as an effective ingredient in the present invention is orally administered to a rat in a single dose at 1g/kg of the body weight.

The present invention will be more concretely described by means of the examples, without by no means limiting the scope of the present invention thereto. Unless specified otherwise, "%" in the examples means "% by weight."

### Example 1

(1) The procedures of suspending 6.7 g of powder of bamboo blade, prepared by lyophilizing a piece of bamboo blade and thereafter powdering the lyophilized product, in 100 ml of chloroform, and filtering the suspension to collect an insoluble fraction were repeated thrice. Thereafter, the procedures of suspending the insoluble fraction in 100 ml of ethanol, and filtering the suspension to collect an insoluble fraction were repeated thrice. The ethanol was completely removed from the insoluble fraction obtained by the above procedures, and the residue was suspended in 100 ml of distilled water. This suspension was incubated at 60°C for 1 hour, and thereafter the suspension was filtered. A 2.5-fold amount of ethanol was added to the filtrate, and the mixture was cooled to -20°C, and thereafter centrifuged at a low temperature to give precipitates. The precipitates were dissolved in distilled water, and the solution was lyophilized to give bamboo blade extract containing a powdery saccharide.
(2) Three-hundred microliters of a 10 mg/ml aqueous solution of the bamboo blade extract prepared in item (1) of Example 1 was placed in Microcon 3000 Cut (manufactured by Amicon), and centrifuged at 10000 rpm for 90 minutes, to prepare a filter-pass fraction (lower layer fraction). Further, the fraction remaining in the upper layer was re-dissolved in 300 µl of distilled water, to prepare an upper layer fraction.

### Example 2

Five grams of brownish onionskin was suspended in 100 ml of distilled water, and incubated at 100°C for 15 minutes. The onionskin was removed, to give 5% onionskin extract.

### Example 3

Three grams of biloba tea leaves (100% Ginkgo, Biloba Tea: trade name: GINGOTON, Gingoton, Inc., Gardena, OA 90248 USA) were suspended in 200 ml of distilled water, and the mixture was incubated at 100°C for 15 minutes. Thereafter, the biloba tea leaves were removed by filtration, to give supernatant. This supernatant was lyophilized, and thereafter dissolved in 8 ml of distilled water, to give biloba tea leaf extract.

### Example 4

(1) The enhancing activity for HGF production of each of the bamboo blade extract (sample (1)) prepared in item (1) of Example 1, and the lower layer fraction (sample (2)) and the upper layer fraction (sample (3)) prepared in item (2) of Example 1 was assayed. Five-hundred microliters each of MRC-5 cells (CCL 171: manufactured by DAINIPPON PHARMACEUTICAL CO., LTD., code. 02-021) suspended in a DME medium containing 10% fetal bovine serum so as to have a concentration of 1 × 10⁵ cells/ml were added into each well of a 48-well cell culture plate. The cells were cultured at 37°C for 24 hours in the presence of 5% CO₂, and then the medium was exchanged for a DME medium containing 1% fetal bovine serum. Thereafter, the sample was added to the medium, and the cells were further cultured for 24 hours. The medium was then collected, and by using Quantikine Human Hepatocyte Growth Factor (HGF) ELISA Kit (manufactured by Funakoshi, Code. RS-0641-00), the amount of HGF in the medium was determined. The amount of HGF produced was expressed, assuming that the value of the negative control is 100%. The sample (1) was added so as to have a final concentration of 0.001, 0.01, 0.1 or 1 µg/ml, and the sample (2) and the sample (3) were each added so as to have a final concentration of 1, 10 or 100 µg/ml. As the negative control, distilled water was added in the same volume as that of the sample. The amount of HGF produced was expressed, assuming that the value of the negative control is 100%. The results for the sample (1) are shown in Table 1, and the results for the sample (2) and the sample (3) are shown in Table 2. Each experiment was carried out twice, and its average value was taken. As shown in Tables 1 and 2, each of these samples enhanced HGF production. Especially the sample (1)-added and sample (3)-added groups strongly increased the amount of HGF production. From the above, it was shown that the bamboo blade extract has an enhancing activity for HGF production. Further, since the upper layer fraction showed an enhancing activity for HGF production, the polymeric substances having a molecular weight larger than 3000 was found to have inducing activity for HGF production.

**Table 1**

| Concentration of Sample (1) (µg/ml) | Amount of HGF Produced (%) |
|---|---|
| 0 | 100 |
| 0.001 | 123 |
| 0.01 | 118 |
| 0.1 | 182 |
| 1 | 570 |
| (Here, the amount of HGF produced in the control was 8.79 ng/ml.) | |

**Table 2**

| Concentration of Sample (µg/ml) | Amount of HGF Produced of Sample (2) (%) | Amount of HGF Produced of Sample (3) (%) |
|---|---|---|
| 0 | 100 | 100 |
| 1 | 106 | 417 |
| 10 | 109 | 584 |
| 100 | 216 | 476 |
| (Here, the amount was 9.99 ng/ml.) of HGF produced in the control | | |

(2) About 5 g of a powdered bamboo blade was stirred for 15 minutes or more together with 70 ml of 50% acetone, and the resulting extract was subjected to suction filtration, and the residue was extracted again with the same volume of the solvent. The enhancing activity for HGF production of this crude extract was evaluated in the same manner as in item (1) of Example 4. As a result, the activity was confirmed as shown in Table 3. The crude extract was concentrated under reduced pressure, and the concentrate was diluted 20-folds with water. A part of the dilution was applied to Amberlite XAD-2 (manufactured by Organo) of which amount corresponds to 10 ml, and sequentially eluted with 30 ml each of a 30%-, 40%-, 50%- and 60%-aqueous methanol solution, and methanol. The inducing activity for HGF production of each fraction was assayed. As a result, the activity was confirmed in the eluted fractions of 40%-methanol and 30%-methanol. Each of the eluted fractions of 30%-methanol and 40%-methanol was concentrated and dried, and the residue was subjected to silica column chromatography using
chloroform : methanol: acetic acid = 3:1:2.5% as a developing solvent, and collected in 10-ml aliquots.
As a result of analysis by ¹H-NMR and FAB-MS, fractions containing isoorientin in a high concentration were obtained in 19th to 35th fractions, and these fractions confirmed to have the enhancing activity for HGF production as shown in Table 4.
¹H-NMR
isoorientin: a 7.57 (1H, dd, J 8, 2 Hz), 7.55 (1H, d, J 2 Hz), 7.02 (1H, d, J 8 Hz), 6.81 (1H, s), 6.63 (1H, d, J 8 Hz), 4.74 (1H, d, J 10 Hz), 4.19 (1H, t, J 4.5 Hz)
FAB-MASS
449 (M+H)

**Table 3**

| Crude Extract of Bamboo Blade (%) | Amount of HGF Produced (%) |
|---|---|
| 0 | 100 |
| 0.1 | 195 |
| 1 | 463 |
| (Here, the amount of HGF produced in the control was 9.09 ng/ml.) | |

**Table 4**

| Isoorientin-Containing Fraction (mg/ml) | Amount of HGF Produced (%) |
|---|---|
| 0 | 100 |
| 0.1 | 131 |
| 1 | 318 |
| (Here, the amount of HGF produced in the control was 9.62 ng/ml.) | |

(3) About 300 g of a commercially available dry plum was grounded in a mixer together with 500 ml of methanol, and the mixture was subjected to suction filtration to give a filtrate. One milliliter of this filtrate was concentrated to dryness. The enhancing activity for HGF production of the plum crude extract dissolved in 1 ml of DMSO was evaluated. As a result, the activity as shown in Table 5 was confirmed.
The resulting crude extract was concentrated to remove the extraction solvent, and diluted with water to make up a volume of 1 liter. The dilution was applied to 200 ml of Amberlite XAD-2 (manufactured by Organo), and washed with 500 ml of H₂O. Thereafter, the adsorbed fraction was eluted with methanol. The resulting eluted fraction was concentrated, and thereafter dissolved in a small amount of water. Subsequently, the solution was applied to Cosmosil 140 C₁₈-OPN (manufactured by nakalaitesque), and eluted with a gradient of water and a 25% aqueous acetonitrile solution in a total amount of 800 ml, and collected in about 10-ml aliquots. The resulting fraction was analyzed by ¹H-NMR. As a result, a fraction containing 5-caffeoyl-quinic acid in a high concentration and a fraction containing 4-caffeoyl-quinic acid in a high concentration were obtained in 26th to 32nd fractions and 41st to 48th fractions, respectively. The enhancing activity for HGF production of each fraction was confirmed as shown in Table 6.
¹H-NMR
5-caffeoyl-quinic acid: σ 7.63 (1H, d, J 15.5 Hz), 7.19 (1H, s), 7.12 (1H, d, J 8 Hz), 6.93 (1H, d, J 8 Hz), 5.40 (1H, m), 4.18 (1H, m), 3.77 (1H, dd, J 9.5, 4.5 Hz), 2.2-1.6 (5H, m)
4-caffeoyl-quinic acid: σ 7.49 (1H, d, J 15.5 Hz), 7.04 (1H, d, J 4 Hz), 6.99 (1H, dd, J 8, 4 Hz), 6.73 (1H, d, J 8 Hz), 6.27 (1H, d, J 16 Hz), 4.65 (1H, dd, J 8, 3 Hz), 4.08 (1H, m), 2.2-1.6 (5H, m)

**Table 5**

| Plum Crude Extract (%) | Amount of HGF Produced (%) |
|---|---|
| 0 | 100 |
| 0.01 | 111 |
| 0.1 | 214 |
| 1 | 306 |
| (Here, the amount of HGF produced in the control was 8.02 ng/ml.) | |

**Table 6**

| Sample | Concentration (mg/ml) | Amount of HGF Produced (%) |
|---|---|---|
| High-5-caffeoyl-quinic acid content fraction | 0 | 100 |
| | 0.01 | 111 |
| | 0.1 | 321 |
| High-4-caffeoyl-quinic acid content fraction | 0 | 100 |
| | 0.01 | 103 |
| | 0.1 | 142 |
| (Here, the amount of HGF produced in the control was 9.98 ng/ml.) | | |

(4) About 300 g of a commercially available broccoli was homogenized together with 350 ml of a 50% aqueous ethanol solution, and the homogenate was filtered to give a filtrate. The residue was homogenized again with 350 ml of a 50% aqueous ethanol solution, and the homogenate was filtered. The resulting filtrates were combined and concentrated under reduced pressure to a volume of 150 ml. The enhancing activity for HGF production of this concentrate was evaluated in the same manner as in item (1) of Example 4. As a result, the enhancing activity for HGF production was confirmed as shown in Table 7.

**Table 7**

| Broccoli Crude Extract (%) | Amount of HGF Produced (%) |
|---|---|
| 0 | 100 |
| 0.01 | 105 |
| 0.1 | 173 |
| 1 | 258 |
| (Here, the amount of HGF produced in the control was 6.80 ng/ml.) | |

### Example 5

The enhancing activities for HGF production of the onionskin extract (sample (1)) prepared in Example 2 and the biloba tea leaf extract (sample (2)) prepared in Example 3 were studied in the same manner as in item (1) of Example 4. Each of the samples was diluted 1-fold, 10-folds, or 100-folds with distilled water, and 5 µl of each dilution was added to a medium. As the negative control, distilled water was added in the same volume as that of the sample. The amount of HGF produced was expressed, assuming that the value of the negative control is 100%. The results are shown in Table 8. All of the experiments were carried out twice, and its average value was taken. As shown in Table 8, the samples (1) and (2) enhanced HGF production. From the above, it was shown that each of the onionskin extract and the biloba tea leaf extract has an enhancing activity for HGF production.

**Table 8**

| Concentration of Sample | Amount of HGF Produced of Sample (1) (%) | Amount of HGF Produced of Sample (2) (%) |
|---|---|---|
| 0 | 100 | 100 |
| 100-folds dilution | 122 | 214 |
| 10-folds dilution | 266 | 358 |
| 1-fold dilution | 248 | 199 |
| (Here, the amount of HGF produced in the control was 10.01 ng/ml.) | | |

### Example 6

(1) As *Artemisia L.,* a plant belonging to LINNE, KWANGHWA MUGWORT was used which was harvested, and thereafter aged for 3 years. The aged product was washed with water, and thereafter the washed product was subjected to hot-water extraction at 100° to 105°C for 7 hours with a steam heating-type extraction device, to give an extract KWANGHWA MUGWORT. This extract was further boiled down for additional 24 hours into a syrupy state, and thereafter formed into a tablet with a tableting machine.
(2) The procedures of suspending 15.563 g of a powder prepared by milling a tablet (manufactured by CHUN-HO FOOD COMPANY) prepared in item (1) of Example 6 in 100 ml of chloroform, and filtering the suspension to collect an insoluble fraction were repeated twice. Thereafter, the procedures of suspending the insoluble fractions in 100 ml of ethanol, and filtering the suspension to collect an insoluble fraction were repeated twice. Ethanol was completely removed from the insoluble fraction obtained by the above procedures, and the residue was suspended in 100 ml of distilled water. This suspension was incubated at 60°C for 1 hour, and the suspension was filtered. A 2.5-fold amount of ethanol was added to the filtrate, and the solution was cooled at -20°C. Thereafter, the cooled mixture was centrifuged at a low temperature to give precipitates. The precipitates were dissolved in distilled water, and lyophilized to give a fraction containing a powdery saccharide.
(3) The enhancing activity for HGF production of the extract of KWANGHWA MUGWORT prepared in item (2) of Example 6 was studied in the same manner as in item (1) of Example 4. The extract of KWANGHWA MUGWORT was added so as to have a final concentration of 1, 10 or 100 µg/ml. As the negative control, distilled water was added in the same volume as that of the sample. The amount of HGF produced was expressed, assuming that the value of the negative control is 100%. The results are shown in Table 9. Each experiment was carried out twice, and its average value was taken. As shown in Table 9, the extract of KWANGHWA MUGWORT enhanced HGF production.

**Table 9**

| Concentration of Extract of KWANGHWA MUGWORT (µg/ml) | Amount of HGF Produced (%) |
|---|---|
| 0 | 100 |
| 1 | 114 |
| 10 | 196 |
| 100 | 771 |
| (Here, the amount of HGF produced in the negative control was 5.27 ng/ml.) | |

### Example 7

(1) Four-hundred-and-eighty grams of a dried product of *Angelica keiskei* koidz. (manufactured by Sakamoto Kanpodo) was extracted twice with one-liter of ethyl acetate and twice with one-liter of ethanol. The resulting residue was extracted with 2 liters of water at 60°C for 1 hour. The extraction filtrate was concentrated to a volume of 250 ml. Thereafter, 50 ml of ethanol was added to the concentrate and insoluble substances were removed therefrom. The resulting clear supernatant was then concentrated under reduced pressure. About one-half the volume of the concentrate was applied to 300 ml of XAD-2 (manufactured by Organo), and washed with 600 ml of water, and thereafter the adsorbed fraction was stepwise eluted in the order of 600 ml of 30% methanol (fraction 1), 500 ml of 60% methanol (fraction 2), and 600 ml of 100% methanol (fraction 3). These eluates were dried, and thereafter each of the residues was dissolved in 50 ml of water, and the enhancing activity for HGF production of each fraction was assayed in the same manner as in item (1) of Example 4. Each of the fractions was added so as to have a final concentration of 0.01, 0.1 or 1%. The results are shown in Table 10. All of the experiments were carried out twice, and its average value was taken. As shown in Table 10, all of these fractions exhibited enhancing activity for HGF production.

**Table 10**

| Sample | Final Concentration (%) | Amount of HGF Produced (%) |
|---|---|---|
| Fraction 1 | 0.01 | 111 |
| | 0.1 | 150 |
| | 1 | 364 |
| Fraction 2 | 0.01 | 162 |
| | 0.1 | 223 |
| | 1 | 388 |
| Fraction 3 | 0.01 | 107 |
| | 0.1 | 145 |
| | 1 | 274 |
| (Here, the amount of HGF produced in the control was 10.88 ng/ml.) | | |

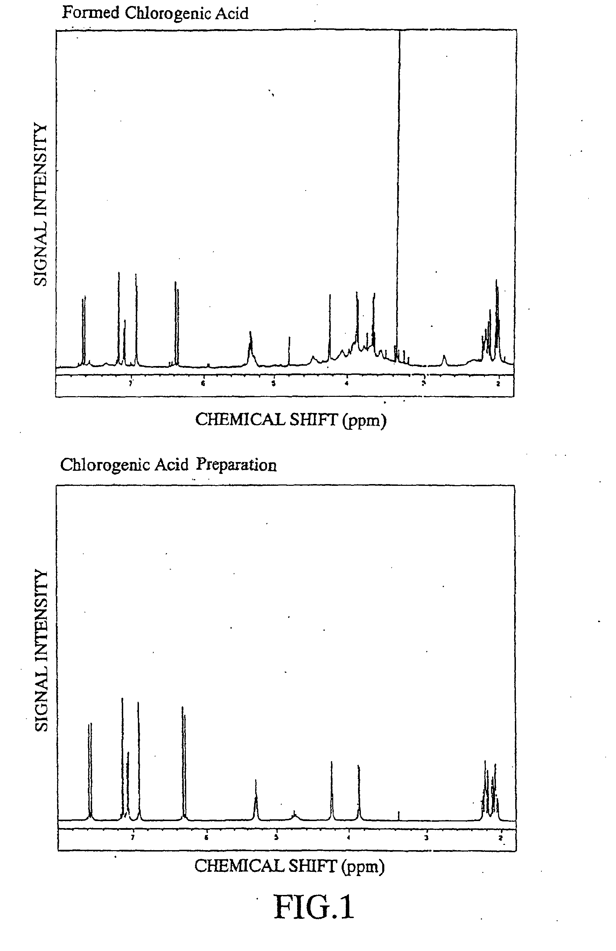

(2) Further, the fraction 3 was developed on a silica gel plate (manufactured by Merck) using butanol : ethanol: acetic acid : water = 10:10:1:1 as a developing solvent, and fractionation was carried out. The enhancing activity for HGF production of each spot was assayed in the same manner as in item (1) of Example 4. As a result, the enhancing activity for HGF production existed in a compound showing absorption at UV 254 nm, which was located at around 0.3 of Rf value (hereinafter referred to as "compound (1)"). This compound (1) was analyzed by ¹H-NMR. As a result, as shown in Figure 1, the NMR spectrum of the compound was identical to that of a chlorogenic acid preparation (manufactured by Sigma) so that the compound (1) was confirmed to be chlorogenic acid. In addition, the enhancing activity for HGF production of the chlorogenic acid preparation was studied in the same manner as in item (1) of Example 4. As a result, as shown in Table 11, the chlorogenic acid preparation was certainly confirmed to have an enhancing activity for HGF production.

**Table 11**

| Concentration of Chlorogenic Acid (µM) | Amount of HGF Produced (%) |
|---|---|
| 0 | 100 |
| 1 | 105 |
| 10 | 122 |
| 100 | 302 |
| 500 | 479 |
| 1000 | 624 |
| (Here, the amount of HGF produced in the control was 8.10 ng/ml.) | |

(3) Further, using the chlorogenic acid preparation as a standard, the amount of chlorogenic acid contained in each of the fractions 1 to 3 was analyzed by HPLC (TSK gel ODS-80Ts (manufactured by Tosoh Corporation), A solution: water (containing 0.1% TFA), B solution: 50% acetonitrile (containing 0.1% TFA), 0 minute-B 25%, 20 minutes-B 75%). The results are shown in Table 12.

**Table 12**

| | Concentration of Chlorogenic Acid (mM) |
|---|---|
| Fraction 1 | 7.5 |
| Fraction 2 | 35.7 |
| Fraction 3 | 5.3 |

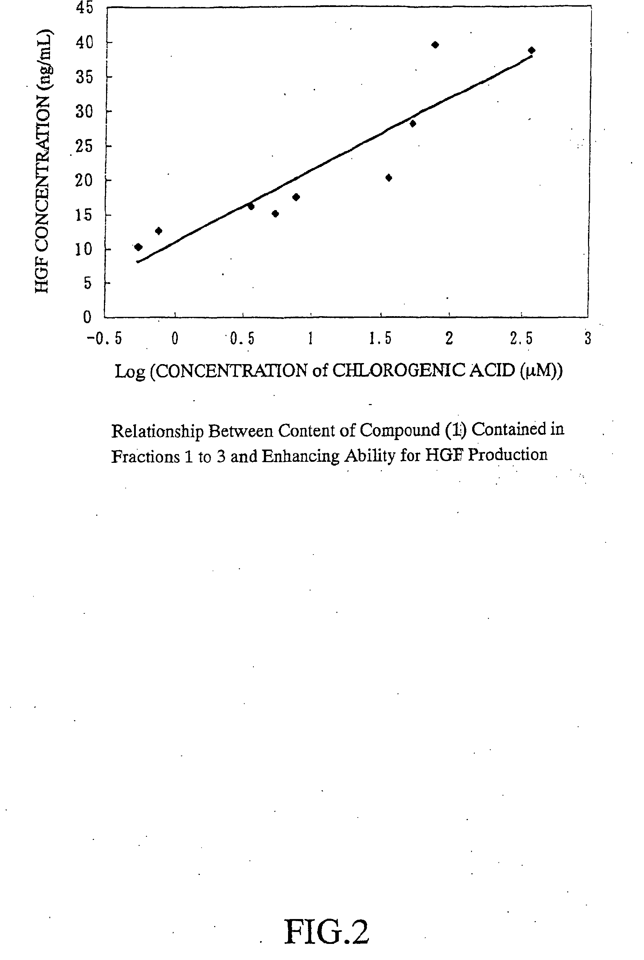

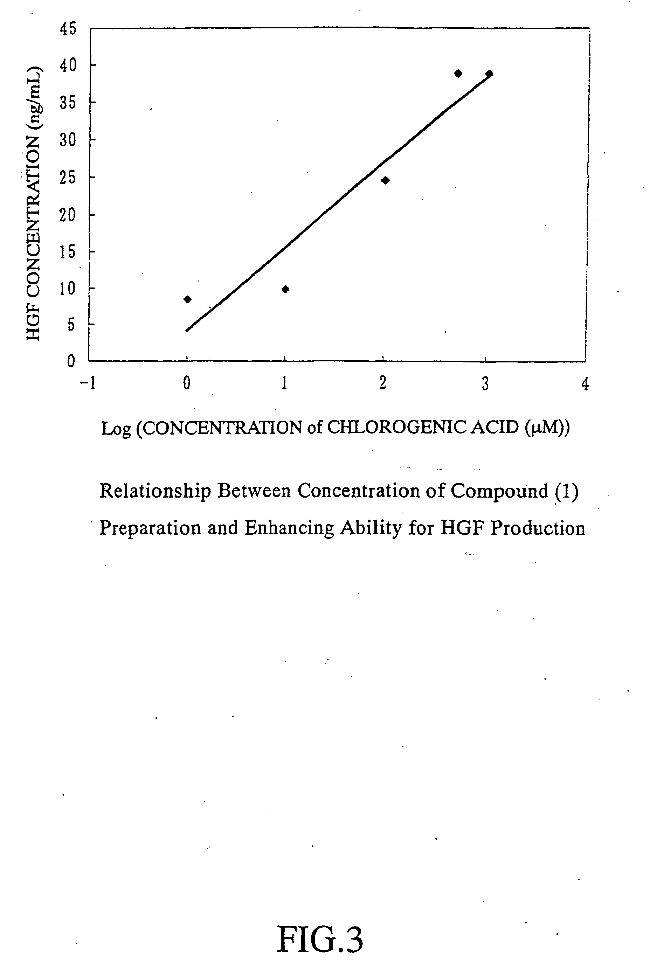

In addition, the correlation between the chlorogenic acid concentration contained in the fractions 1 to 3 and the enhancing activity for HGF production of each fraction on the basis of the results of Tables 10 and 12 is shown in Figure 2, and the correlation between the concentration of the chlorogenic acid preparation and the enhancing activity for HGF production on the basis of the results of Table 11 is shown in Figure 3. These correlations were almost congruent. It was confirmed from the above that the enhancing activity for HGF production of each fraction was mainly caused by chlorogenic acid. Here, in each of Figures 2 and 3, the axis of abscissas is logarithm of the concentration of chlorogenic acid, and the axis of ordinates is HGF concentration.

### Example 8

Forty grams of a dried product of *Artemisia princeps pampan* (manufactured by Sakamoto Kanpodo) were extracted thrice with 500 ml of 50% acetone, and thereafter the extract was concentrated under reduced pressure to a volume of about 15 ml. Thirty-five milliliters of a 10% aqueous citric acid was added to the concentrate, and the mixture was extracted thrice with 100 mL of ethyl acetate. Thereafter, the organic layer was dried over magnesium sulfate, and concentrated under reduced pressure. The concentrate was subjected to silica chromatography using chloroform : methanol : acetic acid = 2:1:1 as a developing solvent, and collected in 8-mL aliquots. The resulting fractions 7 to 13 were collected, and concentrated under reduced pressure. Thereafter, the concentrate was developed on the silica gel plate using
chloroform : methanol: acetic acid = 1:1:0.05 as a developing solvent, to collect 225 mg of a substance showing absorption at UV 254 nm, which was located at around 0.5 of Rf value (hereinafter referred to as "compound (2)"). The structure of this substance was analyzed by ¹H-nuclear magnetic resonance (NMR) spectrum (JNM-A500, manufactured by JEOL, Ltd.).
¹H-NMR: σ 7.46 (1H, d, J 15.5 Hz), 7.45 (1H, d, J 15.5 Hz), 7.02 (1H, m), 7.00 (1H, m), 6.93 (2H, m), 6.72 (1H, d, J 8.5 Hz), 6.70 (1H, d, J 8.5 Hz), 6.20 (1H, d, J 15.5 Hz), 6.17 (1H, d, J 15.5 Hz), 5.33 (1H, td, J 10.5, 4.5 Hz), 5.13 (1H, m), 3.68 (1H, dd, J 10, 3 Hz), 2.2-1.6 (5H, m)

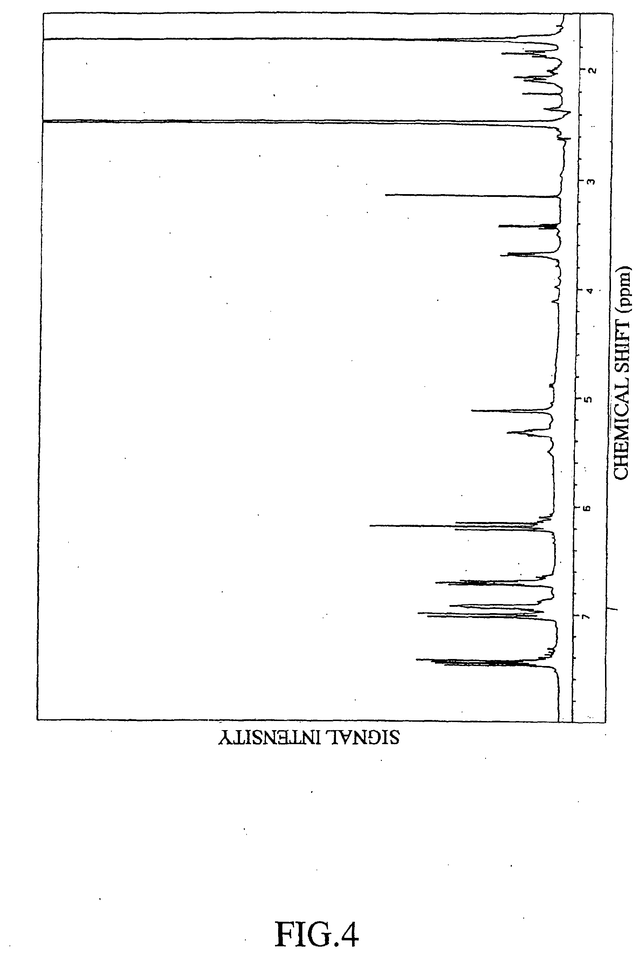

Figure 4 shows ¹H-NMR spectrum. In Figure 4, the axis of abscissas is the chemical shift (ppm), and the axis of ordinates is intensity of signal. It was confirmed from these results that the compound (2) is 3,5-dicaffeoyl-quinic acid. The enhancing activity for HGF production of 3,5-dicaffeoyl-quinic acid was studied in the same manner as in item (1) of Example 4. As a result, it was seen as shown in Table 13 that 3,5-dicaffeoyl-quinic acid has the enhancing activity for HGF production.

**Table 13**

| Concentration of 3,5-Dicaffeoyl-quinic Acid (µM) | Amount of HGF Produced (%) |
|---|---|
| 1 | 142 |
| 10 | 206 |
| 100 | 290 |
| (Here, the amount of HGF produced in the control was 6.27 ng/ml.) | |

### Example 9

(1) Ten grams of a dried product of *Artemisia princeps pampan* (manufactured by Sakamoto Kanpodo) were cut into thin pieces, and extracted five times with 100 ml of chloroform and five times with 100 ml of ethanol. The resulting residue was extracted with 150 ml of water at 60°C for 1 hour, to give an extraction filtrate. The amount 326 ml of ethanol was added to 131 ml of this filtrate, and the mixture was allowed to stand at -20°C for 30 minutes, and centrifuged. The resulting clear supernatant was lyophilized to obtain a hot water-extracted, low-molecular fraction of *Artemisia princeps pampan* (fraction 4). This fraction was dissolved in 4.5 ml of water, and the solution was added to the medium so as to have a final concentration of 0.01 or 0.1%. The enhancing activity for HGF production was studied in the same manner as in item (1) of Example 4. As a result, it was seen as shown in Table 14 that the fraction 4 has the enhancing activity for HGF production.

**Table 14**

| Final Concentration (%) of Fraction 4 | Amount of HGF Produced (%) |
|---|---|
| 0.01 | 258 |
| 0.1 | 294 |
| (Here, the amount of HGF produced in the control was 7.76 ng/ml.) | |

(2) Each of the contents of chlorogenic acid and 3,5-dicaffeoyl-quinic acid contained in the fraction 4 was quantified in the same manner as in item (3) of Example 7. As a result, each was contained in the sample raw material solution in a concentration of 7.7 mM and 13 mM. It was clarified from the above results that each of chlorogenic acid and 3,5-dicaffeoyl-quinic acid is one of active substances for enhancing activity for HGF production of extracts of*Artemisia princeps pampan.*

### Example 10

(1) Fifteen grams of *Chrysanthemum morifolium* (dried flowers of Chrysanthemum, manufactured by JA Aomori Keizairen, JA Nanbucho) was lyophilized and thereafter cut into thin pieces, and the thinly cut pieces were extracted with 1000 ml of chloroform at room temperature. A liquid portion resulting from removal of the residue was concentrated to dryness with a rotary evaporator, to give a chloroform-extracted fraction. Next, the residue was extracted with 500 ml of ethanol at room temperature. A liquid portion resulting from removal of the residue was concentrated to dryness with a rotary evaporator, to give an ethanol-extracted fraction. Next, the residue was extracted with 150 ml of distilled water at 60°C for 2 hours. A 2.5-fold amount of ethanol was added to a liquid portion resulting from removal of the residue, and the mixture was allowed to stand at -30°C for 2 hours. Thereafter, a liquid portion resulting from removal of precipitates by centrifugation at 10000 G was concentrated to dryness with a rotary evaporator, to give a water-extracted fraction of *Chrysanthemum morifolium*.
(2) The enhancing activity for HGF production of the water-extracted fraction of *Chrysanthemum morifolium* obtained in item (1) of Example 10 was studied in the same manner as in item (1) of Example 4. The water-extracted fraction of *Chrysanthemum morifolium* was added so as to have a final concentration of 1%. As a result, it was seen as shown in Table 15 that the water-extracted fraction of *Chrysanthemum morifolium* has enhancing activity for HGF production.

**Table 15**

| Final Concentration (mg/ml) of Water-Extracted Fraction of *Chrysanthemum morifolium* | Amount of HGF Produced (%) |
|---|---|
| 4.04 | 383 |
| (Here, the amount of HGF produced in the control was 12.51 ng/ml.) | |

(3) Twenty grams of *Chrysanthemum morifolium* made in China (available in a local market in Dalian, China) was lyophilized and thereafter cut into thin pieces, and the thinly cut pieces were extracted with 500 ml of chloroform at room temperature. A liquid portion resulting from removal of the residue was concentrated to dryness with a rotary evaporator, to give a chloroform-extracted fraction. Next, the residue was extracted with 500 ml of ethanol at room temperature. A liquid portion resulting from removal of the residue was concentrated to dryness with a rotary evaporator, to give an ethanol-extracted fraction. Next, the residue was extracted with 300 ml of distilled water at 60°C for 2 hours. A 2.5-fold amount of ethanol was added to a liquid portion resulting from removal of the residue, and the mixture was allowed to stand at -30°C for 2 hours. Thereafter, the mixture was centrifuged at 10000 G to fractionate the mixture into precipitates and a liquid portion. The liquid portion was concentrated to dryness with a rotary evaporator, to give a water-extracted fraction.
(4) The enhancing activity for HGF production of the water-extracted fraction of *Chrysanthemum morifolium* made in China obtained in item (3) of Example 10 was studied in the same manner as in item (1) of Example 4. The water-extracted fraction of *Chrysanthemum morifolium* made in China was added so as to have a final concentration of 1, 5 or 10%. As a result, it was seen as shown in Table 16 that the water-extracted fraction of *Chrysanthemum morifolium* made in China has the enhancing activity for HGF production.

**Table 16**

| Final Concentration (mg/ml) of Water-Extracted Fraction of *Chrysanthemum morifolium* Made in China | Amount of HGF Produced (%) |
|---|---|
| 0.71 | 267 |
| 3.55 | 373 |
| 7.10 | 367 |
| (Here, the amount of HGF produced in the control was 12.51 ng/ml.) | |

### Example 11

The enhancing activity for HGF production of each of the ethanol-extracted fraction-2, the water-extracted fraction-1 and the water-extracted fraction-2 of *Curcuma zedoaeia Roscoe* prepared in item (1) of Example 38 described below was studied in the same manner as in item (1) of Example 4. The ethanol-extracted fraction-2 was added so as to have a final concentration of 10 µg/ml, the water-extracted fraction-1 was added so as to have a final concentration of 132 or 1320 µg/ml and the water-extracted fraction-2 was added so as to have a final concentration of 2.2 or 22 µg/ml. The amount of HGF produced was expressed, assuming that the value of the negative control is 100%. The results are shown in Table 17. Each experiment was carried out twice, and its average value was taken. As shown in Table 17, each of the ethanol-extracted fraction-2, the water-extracted fraction-1 and the water-extracted fraction-2 of *Curcuma zedoaeia Roscoe* enhanced HGF production.

**Table 17**

| Sample | Concentration (µg/ml) | Amount of HGF Produced (%) |
|---|---|---|
| Ethanol-extracted fraction-2 of *Curcuma zedoaeia Roscoe* | 0 | 100 |
| | 10 | 157 |
| Water-extracted fraction-1 of *Curcuma zedoaeia Roscoe* | 0 | 100 |
| | 132 | 185 |
| | 1320 | 324 |
| Water-extracted fraction-2 of *Curcuma zedoaeia Roscoe* | 0 | 100 |
| | 2.2 | 182 |
| | 22 | 207 |
| (Here, the amount of HGF produced in the control was 13.99 ng/ml.) | | |

### Example 12

(1) One-hundred milliliters of chloroform was added with stirring to 10 g of a product obtained by lyophilizing *Chrysanthemum coronarium*, and powdering the lyophilized product, and the mixture was filtered to collect the residue. The procedures of addition of chloroform and collection of the residue were carried out a total of 5 times. One-hundred milliliters of ethanol was added to the residue with stirring, and the mixture was filtered to collect the residue. The procedures of addition of ethanol and collection of the residue were carried out a total of 5 times. One-hundred milliliters of distilled water was added to the residue, and the mixture was incubated at 60°C for 1 hour, and then filtered. Thereafter, the filtrate was lyophilized to give a sample. This extract was dissolved in distilled water, and the enhancing activity for HGF production of the extract was evaluated in the same manner as in item (1) of Example 4. As a result, the activity as shown in Table 18 was confirmed.

**Table 18**

| Final Concentration (µg/ml) of Fraction-4 | Amount of HGF Produced (%) |
|---|---|
| 0 | 100 |
| 1 | 125 |
| 10 | 148 |
| 100 | 314 |
| (Here, the amount of HGF produced in the control was 8.21 ng/ml.) | |

(2) Nine-hundred grams of commercially available *Chrysanthemum coronarium* was lyophilized, and milled together with 2 liters of a 80% ethanol in a mixer, and the milled product was filtered with a gauze, to give an extract. This extract was concentrated to a volume of 200 ml by concentration under reduced pressure, to prepare a crude extract of *Chrysanthemum coronarium.*

Next, a 100-ml portion of the resulting crude extract was applied to 200 mL of Amberlite XAD-2 resin (manufactured by Organo), and the product was sequentially eluted with 500 ml each of water, each aqueous solution of 30%-, 40%- and 60%-methanol, and methanol. Each of the eluted fractions was concentrated to a volume of 50 ml, and the enhancing activity for HGF production of each fraction was confirmed in the same manner as in item (1) of Example 4. As a result, as shown in Table 19, the concentrate of the 60%-methanol eluted fraction was found to have an enhancing activity for HGF production.

Here, this concentrate was added so as to have a concentration of 0.1%, 10% (volume ratio).

This 60%-methanol eluted fraction was analyzed by ¹H-NMR. As a result, it was confirmed that 3,5-dicaffeoyl-quinic acid was contained in a high concentration.

**Table 19**

| | | | |
|---|---|---|---|
| Sample Concentration (%) | 0 | 0.1 | 1 |
| Amount of HGF Produced (%) | 100 | 220 | 349 |
| (Here, the amount of HGF produced in the control was 8.22 ng/ml.) | | | |

### Example 13

L-M cells (ATCC CCL-1.2) from murine fibroblasts were suspended in an M199 medium (manufactured by ICN) containing 0.5% bactopeptone (manufactured by Gibco) so as to have a concentration of 1.5 × 10⁵ cells/ml. The suspension was put in a 96 well plate in an amount of 0.1 ml each well, and the cells were aseptically cultured. After culturing the cells for 3 days, the medium was removed therefrom, and exchanged with an M199 medium containing 0.5% bovine serum albumin (manufactured by Sigma). Thereto was added an yellow pigment (powder Sun Yellow No. 2, manufactured by San Ei Gen F.F.I.) obtained by extraction from flower of *Carthamus tinctorius* LINNE so as to have a final concentration of 1.25, 2.5 or 5 mg/ml, and the cells were cultured for 20 hours. After the termination of the culture, the concentration of the nerve growth factor in the culture medium was assayed by an enzyme immunoassay method (NGF Emax Immuno Assay System, manufactured by Promega). The enhancement for NGF production was expressed, assuming that the NGF concentration in the cell culture medium with no addition of the sample is 100%. The experiment was carried out twice, and an average value was taken. As a result, the yellow pigment from *Carthamus tinctorius* enhanced NGF production of the L-M cells in a concentration dependent manner. The results are shown in Table 20.

**Table 20**

| Concentration (mg/ml) of Yellow Pigment from *Carthamus tinctorius* | Amount of NGF Produced (%) |
|---|---|
| 0 | 100 |
| 1.25 | 277.6 |
| 2.5 | 441.3 |
| 5 | 808.4 |
| (Here, the amount of NGF control was 0.507 ng/ml.) produced in the | |

### Example 14

The active component in the yellow pigment of *Carthamus tinctorius* described in Example 13 was purified and isolated by reverse phase chromatography. The conditions are shown below. The column used was TSK gel ODS 80Ts (diameter: 21.5 mm, length: 30 cm, manufactured by Tosoh Corporation). The elution ratio of Solvent A (0.1% aqueous trifluoroacetic acid) and Solvent B (mixture of distilled water and acetonitrile in a volume ratio of 1:1, containing 0.1% trifluoroacetic acid) was such that the ratio of Solvent B was increased linearly from 0 to 100% from 0 to 50 minutes, the ratio of Solvent B was retained at 100% for the subsequent 15 minutes, and the ratio of Solvent B was finally decreased to 0% and retained thereat for 15 minutes. The elution rate was 5 ml/minute, and the detection was carried out at 215 nm. A fraction was collected every 3 minutes, and the enhancing activity for NGF production for each fraction was assayed in the same manner as in Example 13. As a result, it was clarified that each of the fractions including peaks detected at retention time of 32.5 minutes and 41.8 minutes has the enhancing activity for NGF production. The fraction including a peak at a retention time of 32.5 minutes was analyzed by mass spectrum. As a result, a signal corresponding to a molecular weight of 613 was detected. Further, as a result of analyses by the ¹H-NMR spectrum and the ¹³C-NMR spectrum, the active component was identified as safflomin A (molecular weight: 612.53). The enhancing activity for NGF production of the thus obtained purified safflomin A was assayed in the same manner as in Example 13. The purified safflomin A was added so as to have a concentration of 2.5 mg/ml. As a result, the purified safflomin A enhanced NGF production of L-M cells. The results for safflomin A are shown in Table 21, and the results for the fraction including a peak at a retention time of 41.8 minutes are shown in Table 22.

**Table 21**

| Concentration (mg/ml) of Safflomin A | Amount of NGF Produced (%) |
|---|---|
| 0 | 100 |
| 2.5 | 130.7 |
| (Here, the amount of NGF produced in the control was 0.739 ng/ml.) | |

**Table 22**

| Fraction Concentration (mg/ml) of Fractionated Yellow Pigment from *Carthamus tinctorius* | Amount of NGF Produced (%) |
|---|---|
| 0 | 100 |
| 1.25 | 350.7 |
| 5 | 643.4 |
| (Here, the amount of NGF produced in the negative control was 0.736 ng/ml.) | |

### Example 15

Ten grams of *Carthamus tinctorius* LINNE (manufactured by Sakamoto Kanpodo) was cut into thin pieces, and the thinly cut pieces were extracted with 800 ml of chloroform at room temperature. A liquid portion resulting from removal of the residue was concentrated to dryness with a rotary evaporator, to give a chloroform-extracted fraction. Next, the residue was extracted with 1400 ml of ethanol at room temperature. A liquid portion resulting from removal of the residue was concentrated to dryness with a rotary evaporator, to give an ethanol-extracted fraction. Next, the residue was extracted with 150 ml of distilled water at 60°C for 2 hours. A 2.5-fold amount of ethanol was added to a liquid portion resulting from removal of the residue, and the mixture was allowed to stand at -30°C for 2 hours. Thereafter, a liquid portion resulting from removal of precipitates by centrifugation at 10000 G was concentrated to dryness with a rotary evaporator, to give a water-extracted fraction. The enhancing activity for NGF production of each fraction prepared as described above was assayed in the same manner as in Example 13. The chloroform-extracted fraction of *Carthamus tinctorius* was added to the medium so as to have a concentration of 0.393 or 0.785 mg/ml. The ethanol-extracted fraction was added to the medium so as to have a concentration of 0.313 or 0.625 mg/ml. The water-extracted fraction was added to the medium so as to have a concentration of 2.76 or 5.51 mg/ml. The results are shown in Table 23. Each of the chloroform-extracted fraction, the ethanol-extracted fraction and the water-extracted fraction of *Carthamus tinctorius* enhanced NGF production of L-M cells in a concentration-dependent manner.

**Table 23**

| Sample | Concentration (mg/ml) | Amount of NGF Produced (%) |
|---|---|---|
| Chloroform-extracted fraction of *Carthamustinctorius* | 0 | 100 |
| | 0.393 | 535.6 |
| | 0.785 | 986.0 |
| Ethanol-extracted fraction of *Carthamus tinctorius* | 0 | 100 |
| | 0.313 | 345.3 |
| | 0.625 | 1449.5 |
| Water-extracted fraction of *Carthamus tinctorius* | 0 | 100 |
| | 2.76 | 477.8 |
| | 5.51 | 806.3 |
| (Here, the amount of NGF produced in the negative control was 0.190 ng/ml.) | | |

### Example 16

Fifteen grams of *Chrysanthemum morifolium* (dried flowers of Chrysanthemum, manufactured by JA Aomori Keizairen, JA Nanbucho) was lyophilized and thereafter cut into thin pieces, and the thinly cut pieces were extracted with 1000 ml of chloroform at room temperature. A liquid portion resulting from removal of the residue was concentrated to dryness with a rotary evaporator, to give a chloroform-extracted fraction. Next, the residue was extracted with 500 ml of ethanol at room temperature. A liquid portion resulting from removal of the residue was concentrated to dryness with a rotary evaporator, to give an ethanol-extracted fraction. Next, the residue was extracted with 150 ml of distilled water at 60°C for 2 hours. A 2.5-fold amount of ethanol was added to a liquid portion resulting from removal of the residue, and the mixture was allowed to stand at -30°C for 2 hours. Thereafter, a liquid portion resulting from removal of precipitates by centrifugation at 10000 G was concentrated to dryness with a rotary evaporator, to give a water-extracted fraction. The enhancing activity for NGF production of each fraction prepared as described above was assayed in the same manner as in Example 13. The chloroform-extracted fraction of *Chrysanthemum morifolium* was added to the medium so as to have a concentration of 1.8, 3.6 or 7.2 mg/ml. The ethanol-extracted fraction of *Chrysanthemum morifolium* was added to the medium so as to have a concentration of 0.517, 1.03 or 2.07 mg/ml. The water-extracted fraction of *Chrysanthemum morifolium* was added to the medium so as to have a concentration of 16.8, 33.7 or 67.3 mg/ml. Each of the chloroform-extracted fraction, the ethanol-extracted fraction and the water-extracted fraction of *Chrysanthemum morifolium* enhanced NGF production of L-M cells in a concentration-dependent manner. The results are shown in Tables 24 to 26.

**Table 24**

| Concentration (mg/ml) of Chloroform-Extracted Fraction of *Chrysanthemum morifolium* | Amount of NGF Produced (%) |
|---|---|
| 0 | 100 |
| 1.80 | 209.6 |
| 3.60 | 447.3 |
| 7.20 | 1019.9 |
| (Here, the amount of NGF produced in the control was 0.474 ng/ml.) | |

**Table 25**

| Concentration (mg/ml) of Ethanol-Extracted Fraction of *Chrysanthemum morifolium* | Amount of NGF Produced (%) |
|---|---|
| 0 | 100 |
| 0.517 | 164.6 |
| 1.03 | 195.3 |
| 2.07 | 265.1 |
| (Here, the amount of NGF produced in the control was 0.474 ng/ml.) | |

**Table 26**

| Concentration (mg/ml) of Water-Extracted Fraction of *Chrysanthemum morifolium* | Amount of NGF Produced *(%)* |
|---|---|
| 0 | 100 |
| 16.8 | 200.2 |
| 33.7 | 257.6 |
| 67.3 | 336.9 |
| (Here, the amount of NGF produced in the control was 0.474 ng/ml.) | |

### Example 17

(1) The amount 245 g of *Chrysanthemum morifolium* (dried flowers of Chrysanthemum, manufactured by JA Aomori Keizairen, JA Nanbucho) was lyophilized and thereafter cut into thin pieces, and the thinly cut pieces were extracted with 9000 ml of chloroform at room temperature to fractionate into a chloroform extract and a chloroform extract residue. The chloroform extract was concentrated with a rotary evaporator, to give a chloroform extract of Chrysanthemum.
(2) The chloroform extract of Chrysanthemum was subjected to silica chromatography, and eluted with chloroform : methanol = 100:1 (1250 ml) as an eluting solvent, and collected in 8 ml aliquots per fraction. The resulting fractions 44 to 99 were concentrated under reduced pressure, to give a chloroform-extracted fraction A of *Chrysanthemum morifolium*. Next, elution was carried out using methanol (400 ml) as an eluting solvent, and the eluate was concentrated under reduced pressure, to give a chloroform-extracted fraction B of *Chrysanthemum morifolium.*
(3) The enhancing activity for NGF production of the fraction A and the fraction B prepared in item (2) of Example 17 was assayed in the same manner as in Example 13. The fraction A was added to the medium so as to have a concentration of 0.5, 1.0 or 2.0 mg/ml. The fraction B was added to the medium so as to have a concentration of 0.5, 1.0 or 2.0 mg/ml. The results are shown in Table 27. Each of the fractions A and B enhanced NGF production of L-M cells in a concentration-dependent manner.

**Table 27**

| Sample | Concentration (mg/ml) | Amount of NGF Produced (%) |
|---|---|---|
| Chloroform-Extracted Fraction A of *Chrysanthemum morifolium* | 0 | 100 |
| | 0.5 | 114.0 |
| | 1.0 | 304.6 |
| | 2.0 | 677.0 |
| Chloroform-Extracted Fraction B of *Chrysanthemum morifolium* | 0 | 100 |
| | 0.5 | 121.3 |
| | 1.0 | 134.9 |
| | 2.0 | 154.4 |
| (Here, the amount of NGF produced in the negative control was 0.190 ng/ml.) | | |

### Example 18

(1) The chloroform-extracted fraction A of *Chrysanthemum morifolium* prepared in item (2) of Example 17 was further subjected to silica chromatography, and stepwise elution was carried out in the order of ethyl acetate : hexane = 1:10 (500 ml), 1:8 (500 ml), 1:6 (500 ml), and 1:4 (500 ml), and collected in 6-ml aliquots per fraction. The resulting fractions 264 to 290 were concentrated under reduced pressure, to give a chloroform-extracted fraction A-a of *Chrysanthemum morifolium.* The resulting fractions 206 to 240 were concentrated under reduced pressure, to give a chloroform-extracted fraction A-b of *Chrysanthemum morifolium.* The resulting fractions 241 to 263 were concentrated under reduced pressure, to give a chloroform-extracted fraction A-c of *Chrysanthemum morifolium.* A combined mixture of the resulting fractions 291 to 320 and an eluate obtained by using methanol (400 ml) as a developing solvent was concentrated under reduced pressure, to give a chloroform-extracted fraction A-d of *Chrysanthemum morifolium.*
(2) The enhancing activity for NGF production of the fraction A-a, the fraction A-b, the fraction A-c and the fraction A-d prepared in item (1) of Example 18 was assayed in the same manner as in Example 13. The fraction A-a was added to the medium so as to have a concentration of 0.125 or 0.25 mg/ml. The fraction A-b was added to the medium so as to have a concentration of 0.25 or 0.5 mg/ml. The fraction A-c was added to the medium so as to have a concentration of 0.25 or 0.5 mg/ml. The fraction A-d was added to the medium so as to have a concentration of 0.25 or 0.5 mg/ml. The results are shown in Tables 28 and 29. Each of the fraction A-a, the fraction A-b, the fraction A-c and the fraction A-d enhanced NGF production of L-M cells in a concentration-dependent manner.

**Table 28**

| Sample | Concentration (mg/ml) | Amount of NGF Produced (%) |
|---|---|---|
| Chloroform-Extracted Fraction A-a of *Chrysanthemum morifolium* | 0 | 100 |
| | 0.125 | 120.0 |
| | 0.25 | 280.6 |
| Chloroform-Extracted Fraction A-b of *Chrysanthemum morifolium* | 0 | 100 |
| | 0.25 | 216.1 |
| | 0.5 | 959.1 |
| Chloroform-Extracted Fraction A-c of *Chrysanthemum morifolium* | 0 | 100 |
| | 0.25 | 124.4 |
| | 0.5 | 224.8 |
| (Here, the amount of NGF produced in the negative control was 0.489 ng/ml.) | | |

**Table 29**

| Concentration of Chloroform-Extracted Fraction A-d of *Chrysanthemum morifolium* (mg/ml) | Amount of NGF Produced (%) |
|---|---|
| 0 | 100 |
| 0.25 | 157.1 |
| 0.5 | 409.6 |
| (Here, the amount of NGF produced in the negative control was 0.575 ng/ml.) | |

### Example 19

(1) The chloroform-extracted fraction A-a of *Chrysanthemum morifolium* prepared in item (1) of Example 18 was developed on thin-layer chromatography using chloroform : methanol = 50:1 as a developing solvent. A portion corresponding to Rf value of 0.9 to 0.95 was scraped off, and eluted again with the developing solvent to give a chloroform-extracted fraction A-a-1 of *Chrysanthemum morifolium.* A portion corresponding to Rf value of 0.55 to 0.68 was scraped off, and eluted again with the developing solvent to give a chloroform-extracted fraction A-a-2 of *Chrysanthemum morifolium.* A portion corresponding to Rf value of 0.30 to 0.38 was scraped off, and eluted again with the developing solvent to give a chloroform-extracted fraction A-a-3 of *Chrysanthemum morifolium.*
(2) The enhancing activity for NGF production of the fraction A-a-1, the fraction A-a-2 and the fraction A-a-3 prepared in item (1) of Example 19 was assayed in the same manner as in Example 13. The fraction A-a-1 was added to the medium so as to have a concentration of 0.25 or 0.5 mg/ml. The fraction A-a-2 was added to the medium so as to have a concentration of 0.025 or 0.05 mg/ml. The fraction A-a-3 was added to the medium so as to have a concentration of 1.0 mg/ml. The results are shown in Table 30. Each of the fraction A-a-1, the fraction A-a-2 and the fraction A-a-3 enhanced NGF production of L-M cells in a concentration-dependent manner.

**Table 30**

| Sample | Concentration (mg/ml) | Amount of NGF Produced (%) |
|---|---|---|
| Chloroform-Extracted Fraction A-a-1 of *Chrysanthemum morifolium* | 0 | 100 |
| | 0.25 | 193.3 |
| | 0.5 | 639.1 |
| Chloroform-Extracted Fraction A-a-2 of *Chrysanthemum morifolium* | 0 | 100 |
| | 0.025 | 126.4 |
| | 0.05 | 871.9 |
| Chloroform-Extracted Fraction A-a-3 of *Chrysanthemum morifolium* | 0 | 100 |
| | 1.0 | 220.6 |
| (Here, the amount of NGF produced in the negative control was 0.183 ng/ml.) | | |

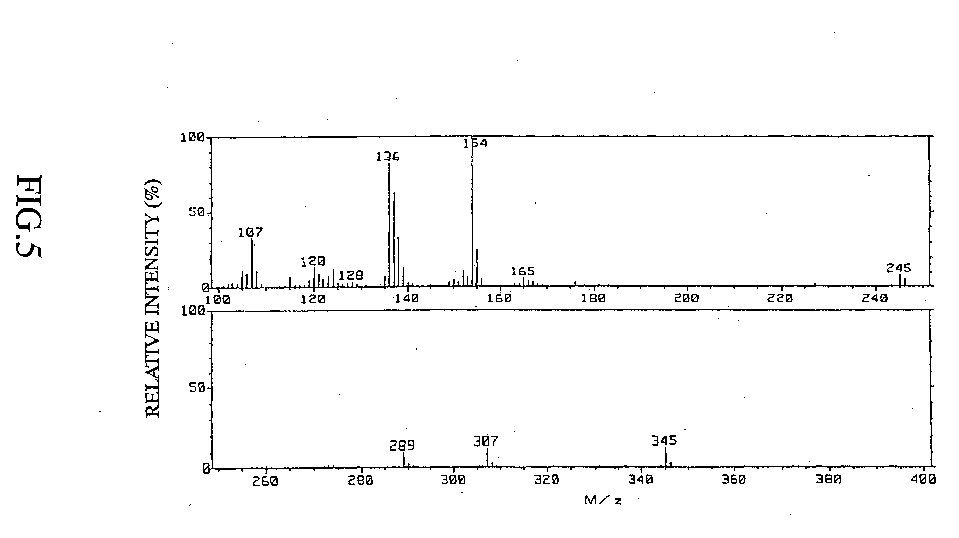

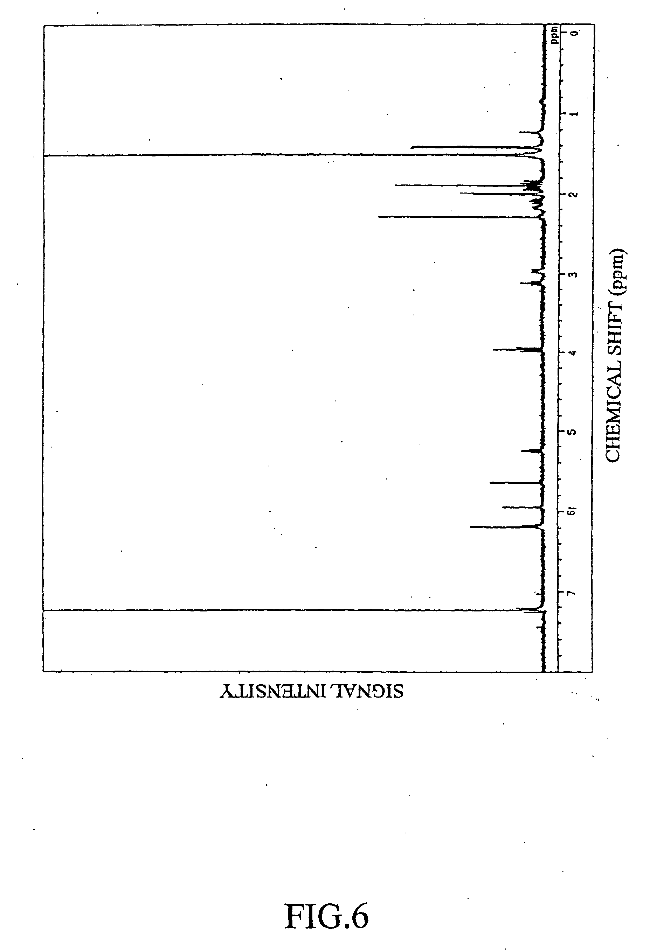

(3) The mass spectrum (MS) of the chloroform-extracted fraction A-a-2 of *Chrysanthemum morifolium* prepared in item (1) of Example 19, of which activity was confirmed in item (2) of Example 19, was determined by mass spectrometer (DX302, manufactured by JEOL LTD.) by FAB-MS technique. As the matrix, nitromethylbenzyl alcohol was used. As a result, a peak of m/z 345 (M+H)⁺ was detected. Figure 5 shows MS spectrum of the chloroform-extracted fraction A-a-2 of *Chrysanthemum morifolium*. In Figure 5, the axis of abscissas is m/z value, and the axis of ordinates is relative intensity.
(4) The structure of the chloroform-extracted fraction A-a-2 of *Chrysanthemum morifolium* prepared in item (1) of Example 19, of which activity was confirmed in item (2) of Example 19, was analyzed by determining various spectra using nuclear magnetic resonance (NMR) spectrometer (JNM-A500, manufactured by JEOL LTD.). The signals of NMR are listed below.
¹H-NMR: δ 1.42 (3H, d, J=7.0 Hz, 14-H), 1.85 (1H, dt, J=14.0, 2.5 Hz, 9-H), 1.88 (3H, t, J=1.5 Hz, 5'-H), 1.94 (1H, t, =5.5 Hz, 1-H), 2.00 (3H, dd, J=1.5, 7.0 Hz, 4'-H), 2.10 (1H, d, J=14.0 Hz, 9-H), 2.17 (1H, m, 10-H), 2.29 (3H, s, 15-H), 2.96 (1H, dd, J=5.5, 10.0 Hz, 5-H), 3.11 (1H, tt, J=3.0, 10.0 Hz, 7-H), 3.96 (1H, t, J=10.0 Hz, 6-H), 5.25 (1H, dt, J=2.5, 10.0 Hz, 8-H), 5.63 (1H, d, J=3.0 Hz, 13-H), 5.94 (1H, brs, 3-H), 6.18 (1H, d, J=3.0 Hz, 13-H), 6.22 (1H, dq, J=1.5, 7.0 Hz, 3'-H)
Here, the sample was dissolved in deuterated chloroform, and the chemical shift of the residual chloroform was expressed as 7.24 ppm. Figure 6 shows ¹H-NMR spectrum of the chloroform-extracted fraction A-a-2 of *Chrysanthemum morifolium.* In Figure 6, the axis of abscissas is the chemical shift (ppm), and the axis of ordinates is the intensity of signal.
(5) The chloroform-extracted fraction A-a-2 of *Chrysanthemum morifolium* prepared in item (1) of Example 19, of which activity was confirmed in item (2) of Example 19, was analyzed using mass spectrum and NMR spectrum. As a result, the active component was identified to be a guaianolide (2-oxo-8-angeloyloxy-guaia-3(4),11(13)-dien-12,6-olide) (molecular weight: 344).

### Example 20

(1) The chloroform-extracted fraction A-b of *Chrysanthemum morifolium* prepared in item (1) of Example 18 was developed on thin-layer chromatography using chloroform : methanol = 100:1 as a developing solvent. A portion corresponding to Rf value of 0.43 to 0.57 was scraped off, and eluted again with the developing solvent to give a chloroform-extracted fraction A-b-1 of *Chrysanthemum morifolium.*
(2) The enhancing activity for NGF production of the fraction A-b-1 prepared in item (1) of Example 20 was assayed in the same manner as in Example 13. The fraction A-b-1 was added to the medium so as to have a concentration of 0.025 or 0.05 mg/ml. The results are shown in Table 31. The fraction A-b-1 enhanced NGF production of L-M cells in a concentration-dependent manner.

**Table 31**

| Concentration (mg/ml) of Chloroform-Extracted Fraction A-b-1 of *Chrysanthemum morifolium* | Amount of NGF Produced (%) |
|---|---|
| 0 | 100 |
| 0.025 | 183.4 |
| 0.05 | 1053.3 |
| (Here, the amount of NGF produced in the negative control was 0.183 ng/ml.) | |

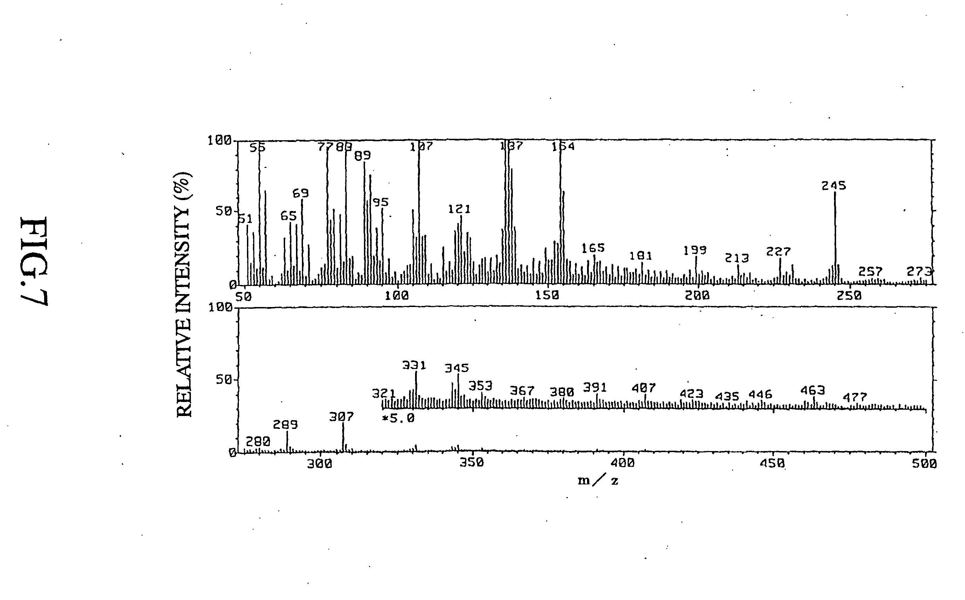

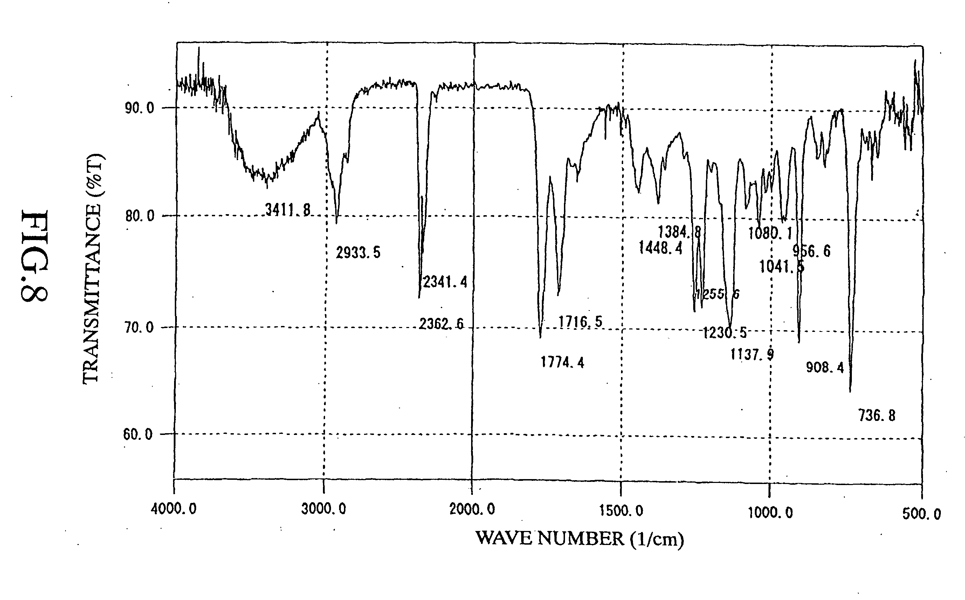

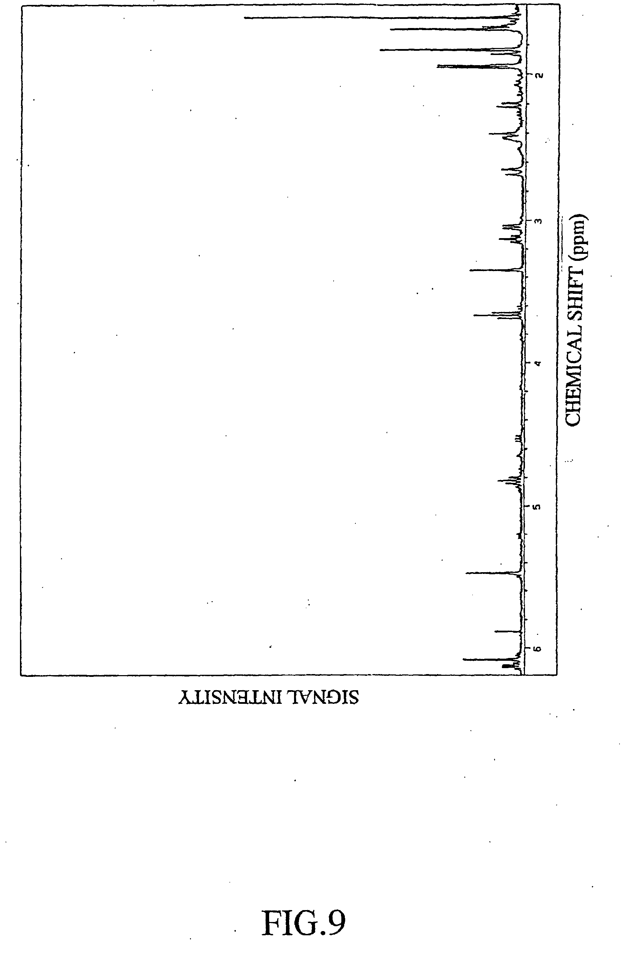

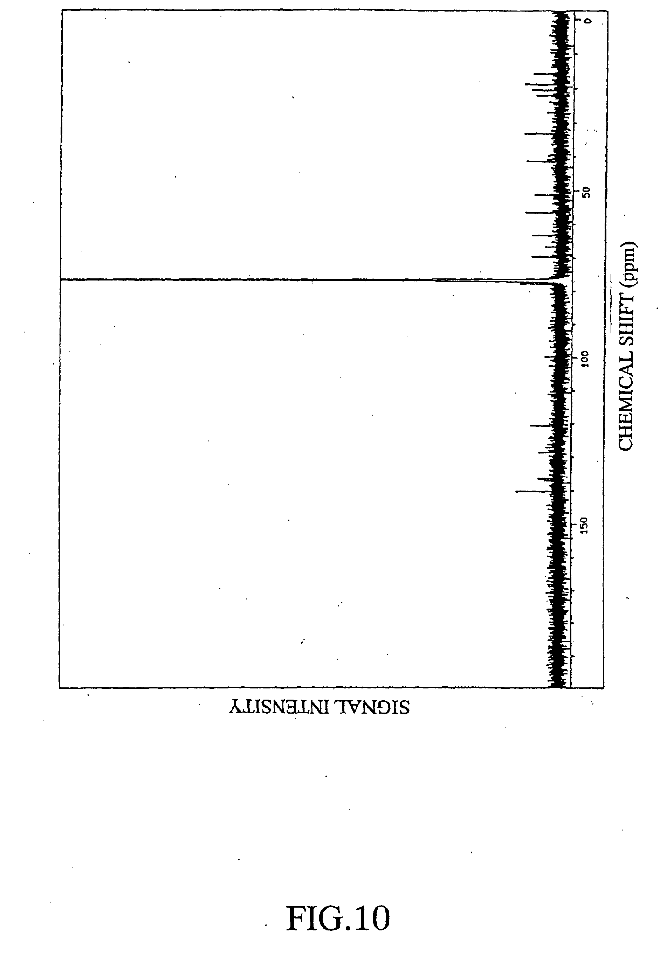

(3) The mass spectrum (MS) of the chloroform-extracted fraction A-b-1 of *Chrysanthemum morifolium* prepared in item (1) of Example 20, of which activity was confirmed in item (2) of Example 20, was analyzed by mass spectrometer (DX302, manufactured by JEOL LTD.). As the matrix, nitromethylbenzyl alcohol was used. As a result, a peak of m/z 345 (M+H)⁺ was detected. Figure 7 shows MS spectrum of the chloroform-extracted fraction A-b-1 of *Chrysanthemum morifolium.* In Figure 7, the axis of abscissas is m/z value, and the axis of ordinates is relative intensity.
(4) The structure of the chloroform-extracted fraction A-b-1 of *Chrysanthemum morifolium* prepared in item (1) of Example 20, of which activity was confirmed in item (2) of Example 20, was analyzed by infrared absorption (IR) spectrum (FTIR-8200PC, manufactured by Shimadzu Corporation). As a result, absorptions of 1716.5 cm⁻¹ and 1774.4 cm⁻¹ were detected. Figure 8 shows IR spectrum of the chloroform-extracted fraction A-b-1 of *Chrysanthemum morifolium.* In Figure 8, the axis of abscissas is an inverse of the wavelength of infrared ray, and the axis of ordinates is transmittance.
(5) The structure of the chloroform-extracted fraction A-b-1 of *Chrysanthemum morifolium* prepared in item (1) of Example 20, of which activity was confirmed in item (2) of Example 20, was analyzed by ¹H-nuclear magnetic resonance (NMR) spectrum (JNM-A500, manufactured by JEOL LTD.).
¹H-NMR: δ 1.66 (3H, s, H-15), 1.74 (3H, d, 1.0 Hz, H-14), 1.88 (3H, t, J_{3',5'} 1.5 Hz, J_{4',5'} 1.5 Hz, H-5'), 2.0 (3H, dd, J_{3',4'} 7.5 Hz, H-4'), 2.26 (1H, dd, J_{a,b} 13.5 Hz, J_{8,9} 2.0 Hz, H-9), 2.45-2.5 (2H, m, H-2, H-9), 2.72 (1H, d, J_{a,b} 17.0 Hz, H-2), 3.09 (1H, d, J_{5,6} 10.0 Hz, H-5), 3.18 (1H, ddd, J_{6,7} 10.0 Hz, J_{7,8} 10.5 Hz, J_{7,13} 3.0 Hz, H-7), 3.4 (1H, s, H-3), 3.71 (1H, t, H-6), 4.92 (1H, dd, H-8), *5.52* (1H, d, H-13), 6.13 (1H, d, H-13), 6.18 (1H, qd, H-3')
Here, the sample was dissolved in deuterated chloroform, and the chemical shift of the residual chloroform was expressed as 7.24 ppm. Figure 9 shows ¹H-NMR spectrum of the chloroform-extracted fraction A-b-1 of *Chrysanthemum morifolium.* In Figure 9, the axis of abscissas is the chemical shift (ppm), and the axis of ordinates is the intensity of signal.
(6) The structure of the chloroform-extracted fraction A-b-1 of *Chrysanthemum morifolium* prepared in item (1) of Example 20, of which activity was confirmed in item (2) of Example 20 was analyzed by ¹³C-nuclear magnetic resonance (NMR) spectrum (JNM-A500, manufactured by JEOL LTD.).
¹³C-NMR: δ 15.87 (C-4'), 18.88 (C-15), 20.45 (C-5'), 22.06 (C-14), 33.18 (C-2), 41.45 (C-9), 51.39 (C-5), 56.59 (C-7), 64.33 (C-3), 66.78 (C-4), 69.64 (C-8), 77.93 (C-6), 120.62 (C-13), 128.0 (C-2'), 128.61 (C-1), 136.41 (C-10), 136.95 (C-11), 140.13 (C-3'), 167.0 (C-1'), 169.0 (C-12)
Here, the sample was dissolved in deuterated chloroform, and the chemical shift of the residual chloroform was expressed as 77.93 ppm. Figure 10 shows ¹³C-NMR spectrum of the chloroform-extracted fraction A-b-1 of *Chrysanthemum morifolium.* In Figure 10, the axis of abscissas is the chemical shift (ppm), and the axis of ordinates is the intensity of signal.
(7) The chloroform-extracted fraction A-b-1 of *Chrysanthemum morifolium* prepared in item (1) of Example 20, of which activity was confirmed in item (2) of Example 20, was analyzed by mass spectrum, IR spectrum, ¹H-NMR spectrum and ¹³C-NMR spectrum. As a result, the active component was identified to be a guaianolide (3,4-epoxy-8-angeloyloxy-guaia-1(10),11(13)-dien-12,6-olide) (molecular weight: 344).

### Example 21

(1) The chloroform-extracted fraction A-c of *Chrysanthemum morifolium* prepared in item (1) of Example 18 was developed on thin-layer chromatography using chloroform : ethyl acetate = 10:1 as a developing solvent. A portion corresponding to Rf value of 0.33 to 0.42 was scraped off, and eluted again with the developing solvent to give a chloroform-extracted fraction A-c-1 of *Chrysanthemum morifolium.*
(2) The enhancing activity for NGF production of the fraction A-c-1 prepared in item (1) of Example 21 was assayed in the same manner as in Example 13. The fraction A-c-1 was added to the medium so as to have a concentration of 0.25 or 0.5 mg/ml. The results are shown in Table 32. The fraction A-c-1 enhanced NGF production of L-M cells in a concentration-dependent manner.

**Table 32**

| Concentration (mg/ml) of Chloroform-Extracted Fraction A-c-1 of *Chrysanthemum morifolium* | Amount of NGF Produced (%) |
|---|---|
| 0 | 100 |
| 0.25 | 150.8 |
| 0.5 | 434.4 |
| (Here, the amount of NGF produced in the control was 0.575 ng/ml.) | |

### Example 22

(1) The chloroform-extracted fraction A-d of *Chrysanthemum morifolium* prepared in item (1) of Example 18 was further subjected to silica chromatography, and stepwise elution was carried out in the order of ethyl acetate : hexane = 1:3 (600 ml) and 1:2 (600 ml), and collected in 6-ml aliquots per fraction. The resulting fractions 1 to 43 were concentrated under reduced pressure, to give a chloroform-extracted fraction A-d-1 of *Chrysanthemum morifolium*. The resulting fractions 44 to 73 were concentrated under reduced pressure, to give a chloroform-extracted fraction A-d-2 of *Chrysanthemum morifolium.* The resulting fractions 74 to 80 were concentrated under reduced pressure, to give a chloroform-extracted fraction A-d-3 of *Chrysanthemum morifolium.* A combined mixture of the resulting fractions 124 to 140 and an eluate obtained by using methanol (400 ml) as a developing solvent was concentrated under reduced pressure, to give a chloroform-extracted fraction A-d-4 of *Chrysanthemum morifolium.*
(2) The enhancing activity for NGF production of each of the fraction A-d-1, the fraction A-d-2, the fraction A-d-3 and the fraction A-d-4 prepared in item (1) of Example 22 was assayed in the same manner as in Example 13. The fraction A-d-1 was added to the medium so as to have a concentration of 2.0 mg/ml. The fraction A-d-2 was added to the medium so as to have a concentration of 0.5 or 1.0 mg/ml. The fraction A-d-3 was added to the medium so as to have a concentration of 0.25 or 0.5 mg/ml. The fraction A-d-4 was added to the medium so as to have a concentration of 0.5 or 1.0 mg/ml. The results are shown in Tables 33 and 34. Each of the fraction A-d-1, the fraction A-d-2, the fraction A-d-3 and the fraction A-d-4 enhanced NGF production of L-M cells in a concentration-dependent manner.

**Table 33**

| Sample | Concentration (mg/ml) | Amount of NGF Produced (%) |
|---|---|---|
| Chloroform-Extracted Fraction A-d-1 of *Chrysanthemum morifolium* | 0 | 100 |
| | 2.0 | 371.9 |
| Chloroform-Extracted Fraction A-d-2 of *Chrysanthemum morifolium* | 0 | 100 |
| | 0.5 | 119.3 |
| | 1.0 | 440.3 |
| Chloroform-Extracted Fraction A-d-3 of *Chrysanthemum morifolium* | 0 | 100 |
| | 0.25 | 156.6 |
| | 0.5 | 687.2 |
| (Here, the amount of NGF produced in the control was 0.183 ng/ml.) | | |

**Table 34**

| Concentration (mg/ml) of Chloroform-Extracted Fraction A-d-4 of *Chrysanthemum morifolium* | Amount of NGF Produced (%) |
|---|---|
| 0 | 100 |
| 0.5 | 182.1 |
| 1.0 | 640.3 |
| (Here, the amount of NGF produced in the control was 0.107 ng/ml.) | |

### Example 23

(1) The chloroform-extracted fraction B of *Chrysanthemum morifolium* prepared in item (2) of Example 17 was subjected to silica chromatography, eluted using chloroform: methanol = 20:1 (600 ml) as a developing solvent, and collected in 6-ml aliquots per fraction. The resulting fractions 15 to 29 were concentrated under reduced pressure, to give a chloroform-extracted fraction B-a of *Chrysanthemum morifolium*
The enhancing activity for NGF production of the fraction B-a prepared was assayed in the same manner as in Example 13. The fraction B-a was added to the medium so as to have a concentration of 1.0 mg/ml. The results are shown in Table 35. The fraction B-a enhanced NGF production of L-M cells.

**Table 35**

| Concentration (mg/ml) of Chloroform-Extracted Fraction B-a of *Chrysanthemum morifolium* | Amount of NGF Produced (%) |
|---|---|
| 0 | 100 |
| 1.0 | 358.0 |
| (Here, the amount of NGF produced in the negative control was 0.280 ng/ml.) | |

### Example 24

(1) The residue resulting from the chloroform-extraction in Example 16 was extracted with 8000 ml of ethanol at room temperature to fractionate into an ethanol-extracted solution of Chrysanthemum and an ethanol-extracted residue. The ethanol-extracted solution of Chrysanthemum was concentrated with a rotary evaporator, to give an ethanol extract of Chrysanthemum.
(2) The ethanol-extracted fraction of Chrysanthemum prepared in item (1) of Example 24 was subjected to silica chromatography, eluted with chloroform : methanol = 9:1 (600 ml) as a developing solvent, and collected in 6-ml aliquots per fraction. The resulting fractions 15 to 22 were concentrated under reduced pressure, to give a chloroform-extracted fraction C of *Chrysanthemum morifolium.* Next, the elution was carried out using methanol (400 ml) as a developing solvent, and the resulting eluate was concentrated under reduced pressure, to give a chloroform-extracted fraction D of *Chrysanthemum morifolium.*
(3) The enhancing activity for NGF production of each of the fraction C and the fraction D prepared in item (2) of Example 24 was assayed in the same manner as in Example 13. The fraction C was added to the medium so as to have a concentration of 1.0 mg/ml. The fraction D was added to the medium so as to have a concentration of 2.0 mg/ml. The results are shown in Table 36. Each of the fraction C and the fraction D enhanced NGF production of L-M cells in a concentration-dependent manner.

**Table 36**

| Sample | Concentration (mg/ml) | Amount of NGF Produced (%) |
|---|---|---|
| Chloroform-Extracted Fraction C of *Chrysanthemum morifolium* | 0 | 100 |
| | 1.0 | 1120.1 |
| Chloroform-Extracted Fraction D of *Chrysanthemum morifolium* | 0 | 100 |
| | 2.0 | 455.9 |
| (Here, the amount of NGF produced in the negative control was 0.107 ng/ml.) | | |

### Example 25

Twenty grams of *Chrysanthemum morifolium* made in China (available in a local market in Dalian, China) was lyophilized and thereafter cut into thin pieces, and the thinly cut pieces were extracted with 500 ml of chloroform at room temperature. A liquid portion resulting from removal of the residue was concentrated to dryness with a rotary evaporator, to give a chloroform-extracted fraction. Next, the residue was extracted with 500 ml of ethanol at room temperature. A liquid portion resulting from removal of the residue was concentrated to dryness with a rotary evaporator, to give an ethanol-extracted fraction. Next, the residue was extracted with 300 ml of distilled water at 60°C for 2 hours. A 2.5-fold amount of ethanol was added to a liquid portion resulting from removal of the residue, and the mixture was allowed to stand at -30°C for 2 hours. Thereafter, the mixture was centrifuged at 10000 G to fractionate the mixture into precipitates and a liquid portion. The liquid portion was concentrated to dryness with a rotary evaporator, to give a water-extracted fraction-1. On the other hand, the precipitates were re-dissolved in distilled water, to give a water-extracted fraction-2. The enhancing activity for NGF production of each fraction prepared as described above was assayed in the same manner as in Example 13. The chloroform-extracted fraction of Chrysanthemum was added to the medium so as to have a concentration of 0.114, 0.227 or 0.454 mg/ml. The ethanol-extracted fraction of Chrysanthemum was added to the medium so as to have a concentration of 0.241, 0.481 or 0.962 mg/ml. The water-extracted fraction-1 of Chrysanthemum was added to the medium so as to have a concentration of 8.88, 17.8 or 35.5 mg/ml. The water-extracted fraction-2 of Chrysanthemum was added to the medium so as to have a concentration of 0.02 or 0.04 mg/ml. As a result, each of the chloroform-extracted fraction, the ethanol-extracted fraction, and the water-extracted fraction-1 and fraction-2 of Chrysanthemum enhanced NGF production of L-M cells in a concentration-dependent manner. These results are shown in Table 37.

**Table 37**

| Sample | Concentration (mg/ml) | Amount of NGF Produced (%) |
|---|---|---|
| Chloroform-Extracted Fraction of *Chrysanthemum morifolium*Made in China | 0 | 100 |
| | 0.114 | 168.2 |
| | 0.227 | 297.1 |
| | 0.454 | 912.5 |
| Ethanol-Extracted Fraction of *Chrysanthemum morifolium* Made in China | 0 | 100 |
| | 0.241 | 326.5 |
| | 0.481 | 450.1 |
| | 0.962 | 582.5 |
| Water-Extracted Fraction-1 of *Chrysanthemum morifolium* Made in China | 0 | 100 |
| | 8.88 | 264.1 |
| | 17.8 | 261.8 |
| | 35.5 | 229.1 |
| Water-Extracted Fraction-2 of *Chrysanthemum morifolium* Made in China | 0 | 100 |
| | 0.02 | 135.0 |
| | 0.04 | 187.3 |
| (Here, the amount of NGF produced in the control was 0.516 ng/ml.) | | |

### Example 26

Twenty grams of leaf-and-stem portions of dry *Angelica keiskei* koidz. (manufactured by Sakamoto Kanpodo) were cut into thin pieces, and the thinly cut pieces were extracted with 600 ml of chloroform at room temperature. The residue resulting from removal of a liquid portion was extracted with 600 ml of ethanol at room temperature. The residue resulting from removal of the liquid portion was extracted with 360 ml of distilled water at 60°C for 2 hours. A 2.5-fold amount of ethanol was added to the medium to a liquid portion resulting from removal of the solid residue, and the mixture was allowed to stand at -30°C for 2 hours. Thereafter, the mixture was fractionated into precipitates and a liquid portion by centrifugation at 10000 G. The liquid portion was concentrated to dryness with a rotary evaporator, to give a water-extracted fraction-1 of leaf-and-stem portions of *Angelica keiskei* koidz. On the other hand, the precipitates were re-dissolved in distilled water, to give a water-extracted fraction-2 of leaf-and-stem portions of *Angelica keiskei* koidz. The enhancing activity for NGF production of each of the water-extracted fraction-1 and fraction-2 of leaf-and-stem portions of *Angelica keiskei* koidz. prepared as described above was assayed in the same manner as in Example 13. The water-extracted fraction-1 of leaf-and-stem portions of *Angelica keiskei* koidz. was added to the medium so as to have a concentration of 2.59 or 5.19 mg/ml. The water-extracted fraction-2 of leaf-and-stem portions of *Angelica keiskei* koidz. was added to the medium so as to have a concentration of 0.35 or 0.7 mg/ml. As a result, each of the water-extracted fraction-1 and fraction-2 of leaf-and-stem portions of *Angelica keiskei* koidz. enhanced NGF production of L-M cells in a concentration-dependent manner. These results are shown in Table 38.

**Table 38**

| Sample | Concentration (mg/ml) | Amount of NGF Produced (%) |
|---|---|---|
| Water-Extracted Fraction-1 of leaf-and-stem portions of *Angelica keiskei* koidz. | 0 | 100 |
| | 2.59 | 475.2 |
| | 5.19 | 644.9 |
| Water-Extracted Fraction-2 of leaf-and-stem portions of *Angelica keiskei* koidz. | 0 | 100 |
| | 0.35 | 149.3 |
| | 0.70 | 186.7 |
| (Here, the amount of NGF produced in the control was 0.553 ng/ml.) | | |

### Example 27

Twenty grams of root portions of dry *Angelica keiskei* koidz. (collected in Korea) were cut into thin pieces, and the thinly cut pieces were extracted with 300 ml of chloroform at room temperature. A liquid portion resulting from removal of the residue was concentrated to dryness with a rotary evaporator, to give a chloroform-extracted fraction. Next, the residue was extracted with 300 ml of ethanol at room temperature. A liquid portion resulting from removal of the residue was concentrated to dryness with a rotary evaporator, to give a ethanol-extracted fraction. Subsequently, the residue was extracted with 150 ml of distilled water at 60°C for 2 hours. A 2.5-fold amount of ethanol was added to a liquid portion resulting from removal of the solid residue, and the mixture was allowed to stand at -30°C for 2 hours. Thereafter, the mixture was centrifuged at 10000 G to fractionate the mixture into precipitates and a liquid portion. The liquid portion was concentrated to dryness with a rotary evaporator, to give a water-extracted fraction-1 of root portions of *Angelica keiskei* koidz. On the other hand, the precipitates were re-dissolved in distilled water, to give a water-extracted fraction-2 of leaf-and-stem portions of *Angelica keiskei* koidz. The enhancing activity for NGF production of each fraction prepared as described above was assayed in the same manner as in Example 13. The chloroform-extracted fraction of root portions of *Angelica keiskei* koidz. was added to the medium so as to have a concentration of 0.03 mg/ml. The ethanol-extracted fraction of root portions of *Angelica keiskei* koidz. was added to the medium so as to have a concentration of 0.275, 0.55 or 1.1 mg/ml. The water-extracted fraction-1 of root portions of *Angelica keiskei* koidz. was added to the medium so as to have a concentration of 4.15, 8.3 or 16.6 mg/ml. The water-extracted fraction-2 of root portions of *Angelica keiskei* koidz. was added to the medium so as to have a concentration of 0.55 or 1.1 mg/ml. As a result, each of the chloroform-extracted fraction of root portions of *Angelica keiskei* koidz., and the ethanol-extracted fraction of root portions of *Angelica keiskei* koidz. the water-extracted fraction-1 and fraction-2 of root portions of *Angelica keiskei* koidz. enhanced NGF production of L-M cells. These results are shown in Table 39.

**Table 39**

| Sample | Concentration (mg/ml) | Amount of NGF Produced (%) |
|---|---|---|
| Chloroform-Extracted Fraction of Root Portions of *Angelica keiskei* koidz. | 0 | 100 |
| | 0.03 | 198.4 |
| Ethanol-Extracted Fraction of Root Portions of *Angelica keiskei* koidz. | 0 | 100 |
| | 0.275 | 185.8 |
| | 0.550 | 254.9 |
| | 1.10 | 305.7 |
| Water-Extracted Fraction-1 of Root Portions of *Angelica keiskei* koidz. | 0 | 100 |
| | 4.15 | 350.8 |
| | 8.30 | 496.7 |
| | 16.6 | 830.3 |
| Water-Extracted Fraction-2 of Root Portions of *Angelicakeiskei* koidz. | 0 | 100 |
| | 0.55 | 174.6 |
| | 1.10 | 242.3 |
| (Here, the amount of NGF produced in the control was 0.473 ng/ml.) | | |

### Example 28

(1) Six-hundred grams of a lyophilized product of root portions of *Angelica keiskei* koidz. (collected in Korea) was cut into thin pieces, and the thinly cut pieces were extracted with 18000 ml of chloroform at room temperature. The residue resulting from removal of the chloroform extract by filtration was extracted with 18000 ml of ethanol at room temperature. The residue resulting from removal of the ethanol extract by filtration was extracted with 4000 ml of distilled water at 60°C for 2 hours. A 2.5-fold amount of ethanol was added to a liquid portion resulting from removal of the solid residue, and the mixture was allowed to stand at -30°C for 2 hours. Thereafter, precipitates were removed by centrifugation at 10000 G, and the liquid portion was concentrated to a volume of 200 ml with a rotary evaporator. The concentrate was applied to Amberlite XAD-2 (manufactured by Organo; amount of resin: 400 ml), and non-adsorbed substances were sufficiently washed out with 2000 ml of distilled water. Next, the adsorbed substances were eluted with 2000 ml of methanol. The methanol eluate was concentrated with a rotary evaporator, to give a water-extracted, low-molecular XAD-2-treated fraction of root portions of *Angelica keiskei* koidz.
(2) The active component of the water-extracted, low-molecular XAD-2-treated fraction of root portions of *Angelica keiskei* koidz. prepared in item (1) of Example 28 was fractionated using reverse phase chromatography. The conditions therefor are given below. The column used was TSK gel ODS 80Ts (diameter: 21.5 mm, length: 30 cm, manufactured by Tosoh Corporation). The elution ratio of Solvent A (distilled water) and Solvent B (mixture of distilled water and acetonitrile in a volume ratio of 1:1) was such that the ratio of Solvent B was increased linearly from 0 to 100% from 0 to 120 minutes, the ratio of Solvent B was retained at 100% for the subsequent 20 minutes, and the ratio of Solvent B was finally decreased to 0% and retained thereat for 20 minutes. The elution rate was 5 ml/minute, and the detection was carried out at 215 nm. A fraction was collected every 8 minutes, and the activity was assayed for each fraction in the same manner as in Example 13. As a result, it was clarified that each of the fractions including peaks detected at retention time of 21.0, 22.8, 23.6, 26.7, 34.8, 42.4, 47.8, 51.5, 51.7, 51.9, 52.1, 52.2, 53.8, 55.6, 56.2, 56.6, 56.7, 58.1, 61.5, 68.6, 71.4, 79.7, 84.9, 85.8, 89.7, 101.5, 104.5, 120.9 and 124.7 minutes has the enhancing activity for NGF production. The results are shown in Table 40.

**Table 40**

| Fractionated Fraction (Detected Peaks: minutes) | Concentration (mg/ml) | Amount of NGF Produced (%) |
|---|---|---|
| 1 (21.0, 22.8, 23.6) | 2.50 | 441.3 |
| 2 (26.7) | 2.50 | 379.8 |
| 3 (34.8) | 2.50 | 200.3 |
| 4 (42.4) | 2.84 | 453.3 |
| 5 (47.8) | 3.47 | 385.8 |
| 6 (51.5, 51.7, 51.9, 52.1, 52.2, 53.8) | 1.48 | 409.9 |
| 7 (55.6, 56.2, 56.6, 56.7, 58.1, 61.5) | 1.24 | 268.4 |
| 8 (68.6) | 0.965 | 299.7 |
| 9 (79.7) | 1.16 | 1104.5 |
| 10 (84.9, 85.8, 89.7) | 1.25 | 1196.4 |
| 11 (101.5, 104.5) | 3.17 | 267.9 |
| 12 (120.9, 124.7) | 5.28 | 277.5 |
| (Here, the amount of NGF produced in the control was 0.210 ng/ml.) | | |

(3) The active component of the water-extracted, low-molecular XAD-2-treated fraction of root portions of *Angelica keiskei* koidz. was fractionated by using gel filtration chromatography. The conditions therefor are given below. As the column resin, TOYOPEARL HW-40C (amount of resin: 2000 ml, manufactured by Tosoh Corporation) was used. As the solvent, distilled water was used. The concentrate of the water-extracted, low-molecular XAD-2-treated fraction of root portions of *Angelica keiskei* koidz. in an amount corresponding to 1.0 g on a dry basis was applied to the column, and fractions were collected in 10-ml aliquots. The enhancing activity for NGF production of each fraction was assayed in the same manner as in Example 13. Each fraction sample was concentrated 10-folds, and thereafter the concentrated sample was added so as to have a volume of one-tenth of the medium. As a result, the enhancing activity for NGF production was confirmed in many of the fractions.
(4) The fractions 69 to 79, the part of the fractions of which activity was confirmed in item (3) of Example 28, was collected and concentrated, and the concentrate was fractionated by using reverse phase chromatography. The conditions therefor are given below. The column used was TSK gel ODS 80Ts (diameter: 21.5 mm, length: 30 cm, manufactured by Tosoh Corporation). The elution ratio of Solvent A (0.1% aqueous trifluoroacetic acid) and Solvent B (mixture of distilled water and acetonitrile in a volume ratio of 1:1, containing 0.1% trifluoroacetic acid) was such that the ratio of Solvent B was increased linearly from 25 to 100% from 0 to 60 minutes, the ratio of Solvent B was retained at 100% for the subsequent 10 minutes, and the ratio of Solvent B was finally decreased to 25% and retained thereat for 10 minutes. The elution rate was 5 ml/minute, and the detection was carried out at 215 nm. The enhancing activity for NGF production was assayed for each collected fraction in the same manner as in Example 13. As a result, it was clarified that each of the fractions having retention time of 0-22.5 minutes, 22.5-25 minutes, 25-31.5 minutes, 31.5-32.5 minutes, 32.5-37 minutes, 37-43.5 minutes, 43.5-47 minutes, *47-50.5* minutes, 50.5-77 minutes, 77-78.5 minutes, and 78.5-81.5 minutes has the enhancing activity for NGF production. The results are shown in Table 41.

**Table 41**

| Fraction (Retention Time: minutes) | Concentration (mg/ml) | Amount of NGF Produced (%) |
|---|---|---|
| 1 (0-22.5) | 9.60 | 415.02 |
| 2 (22.5-25) | 0.175 | 172.10 |
| 3 (25-31.5) | 7.00 | 854.94 |
| 4 (22.5-25) | 0.30 | 149.79 |
| 5 (31.5-32.5) | 0.50 | 153.65 |
| 6 (32.5-37) | 1.70 | 272.96 |
| 7 (37-43.5) | 1.50 | 369.96 |
| 8 (43.5-47) | 0.475 | 190.99 |
| 9 (47-50.5) | 2.90 | 507.30 |
| 10 (50.5-77) | 0.70 | 179.83 |
| 11 (78.5-81.5) | 0.15 | 166.52 |
| (Here, the amount of NGF produced in the control was 0.143 ng/ml.) | | |

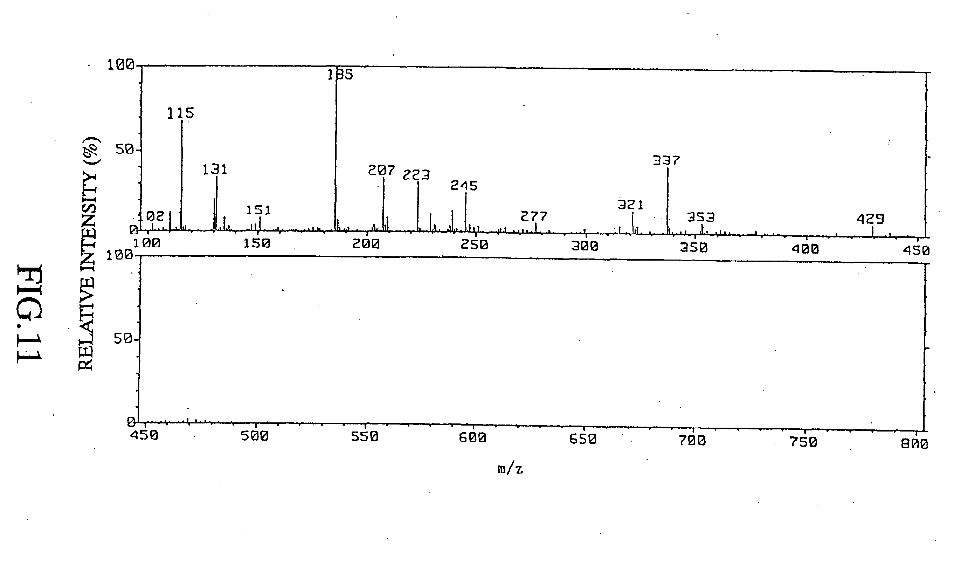

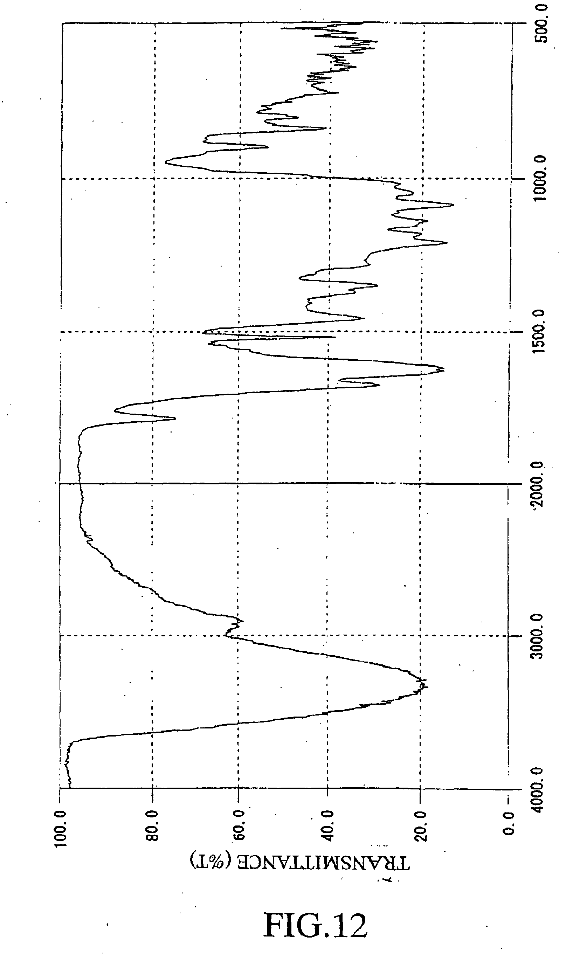

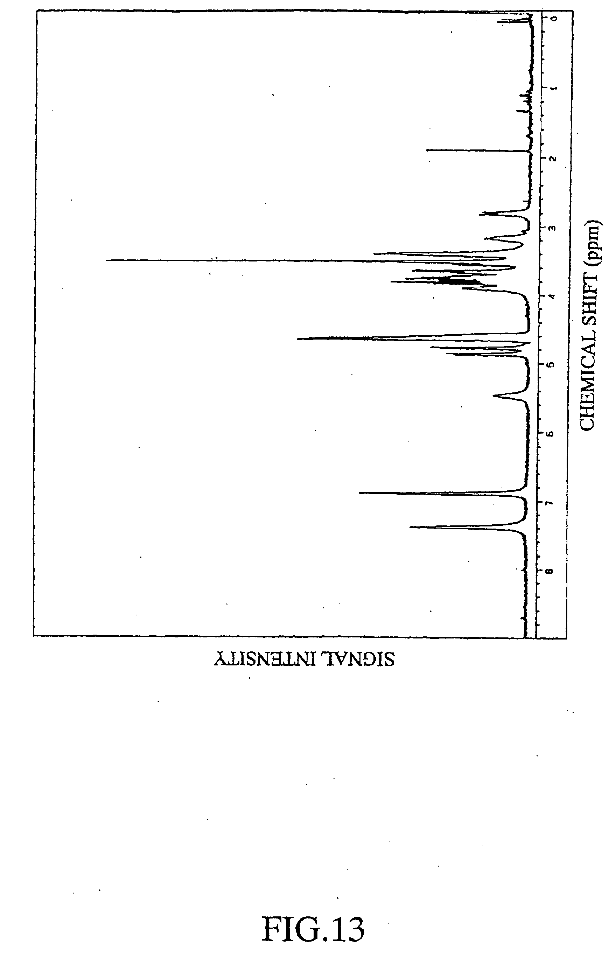

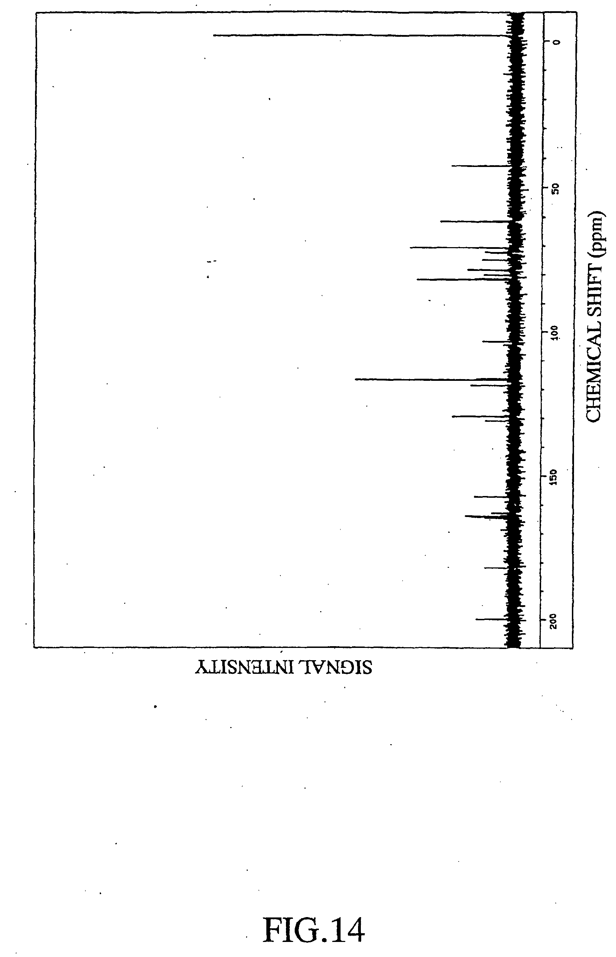

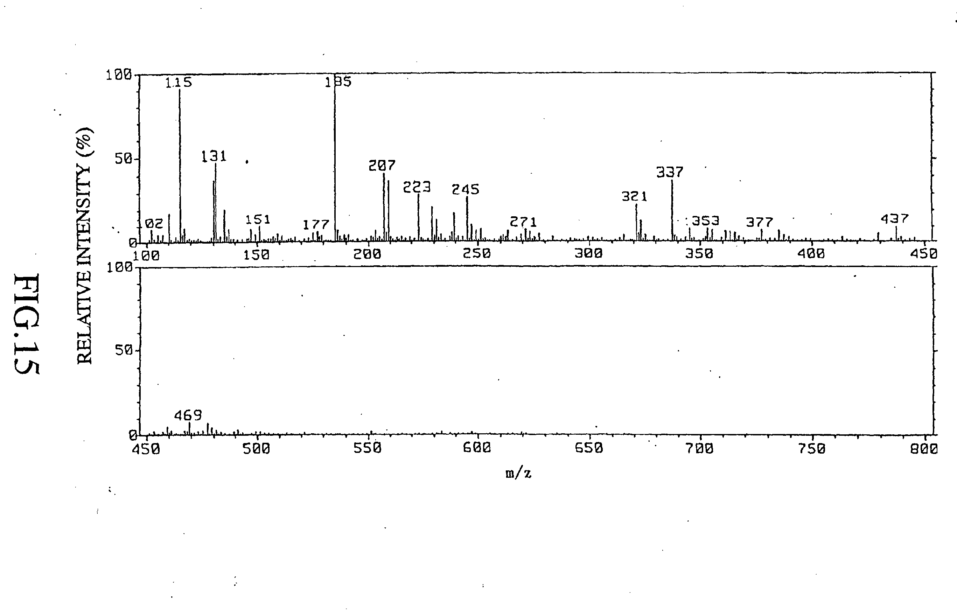

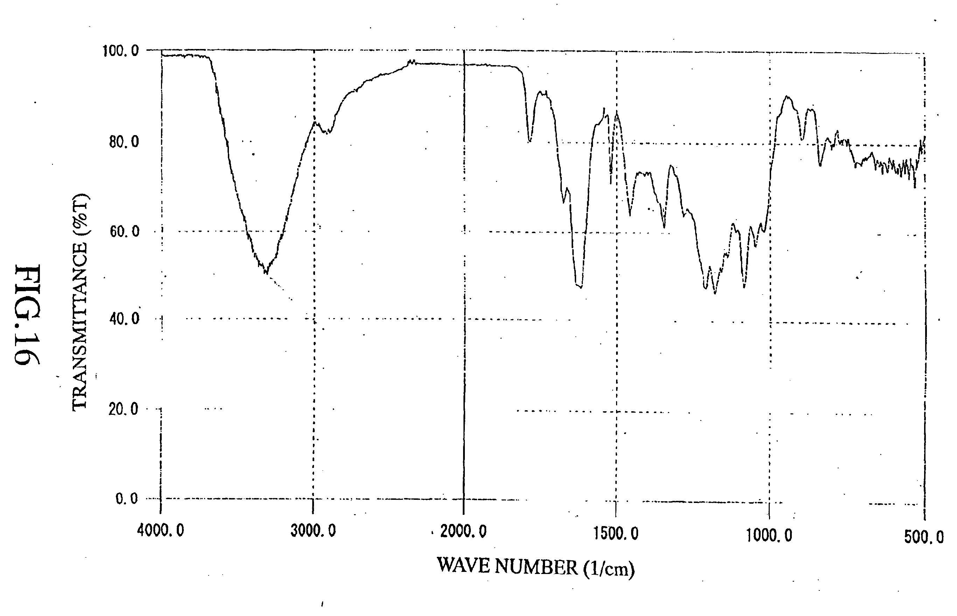

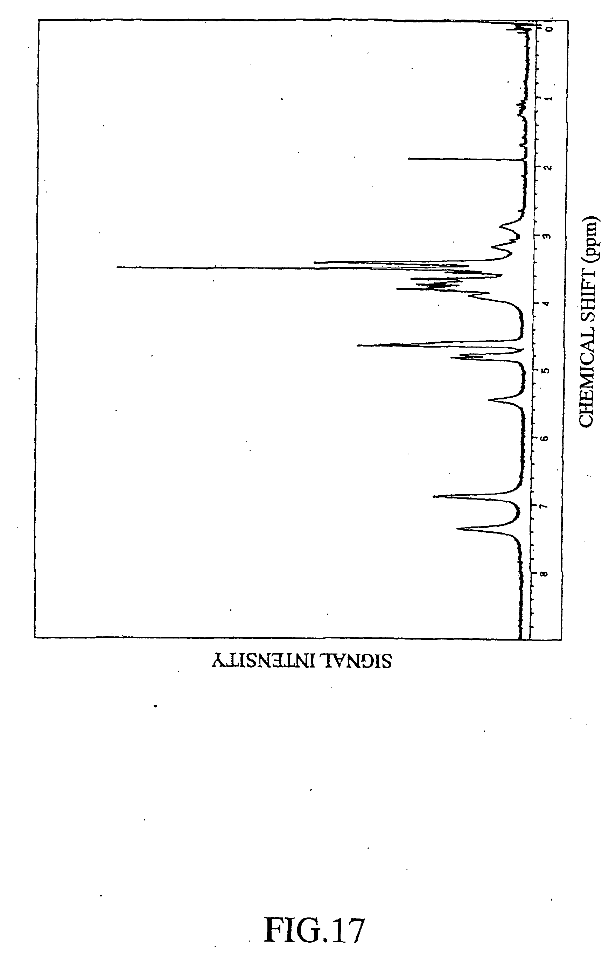

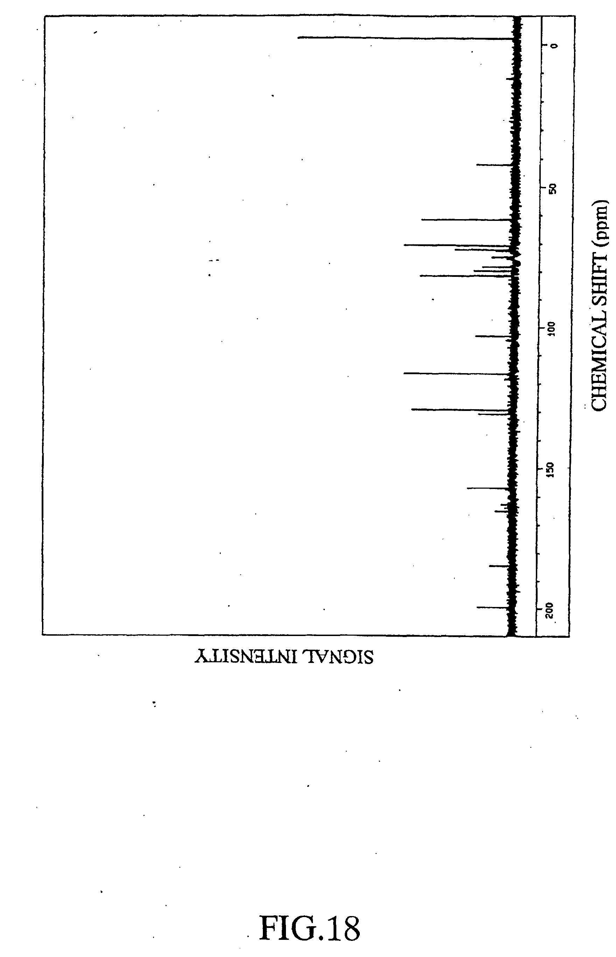

(5) The fraction having a retention time of 25-31.5 minutes, of which potent activity was confirmed in item (4) of Example 28, was further fractionated by using reverse phase chromatography. The conditions therefor are given below. The column used was TSK-GEL Carbon-500 (diameter: 4.6 mm, length: 10 cm, manufactured by Tosoh Corporation). The elution ratio of Solvent A (distilled water) and Solvent B (mixture of distilled water and acetonitrile in a volume ratio of 1:1) was such that the ratio of Solvent B was increased linearly from 25 to 45% from 0 to 15 minutes, the ratio of Solvent B was retained at 45% for the subsequent 10 minutes, and the ratio of Solvent B was finally decreased to 25% and retained thereat for 5 minutes. The elution rate was 1 ml/minute, and the detection was carried out at 215 nm. As a result, there could be fractionated into two fractions, each including peaks of 7.82 minutes and 11.09 minutes.
(6) The mass spectrum (MS) of the fraction including the peak at 7.82 minutes fractionated by TSK-GEL Carbon-500 chromatography in item (5) of Example 28 was analyzed by mass spectrometer (DX302, manufactured by JEOL LTD.). As the matrix, glycerol was used. As a result, peaks of m/z 131, 185, 223 and 321 were detected. Figure 11 shows the mass spectrum. In Figure 11, the axis of abscissas is m/z value, and the axis of ordinates is relative intensity.
(7) The structure of the fraction including the peak at 7.82 minutes fractionated by TSK-GEL Carbon-500 chromatography in item (5) of Example 28 was analyzed by infrared absorption (IR) spectrum (FTIR-8200PC, manufactured by Shimadzu Corporation). Figure 12 shows IR spectrum. In Figure 12, the axis of abscissas is an inverse of the wavelength of infrared ray, and the axis of ordinates is transmittance.
(8) The structure of the fraction including the peak at 7.82 minutes fractionated by TSK-GEL Carbon-500 chromatography in item (5) of Example 28 was analyzed by ¹H-nuclear magnetic resonance (NMR) spectrum (JNM-A500, manufactured by JEOL, Ltd.). The sample was dissolved in heavy water. Figure 13 shows ¹H-NMR spectrum. In Figure 13, the axis of abscissas is chemical shift (ppm), and the axis of ordinates is intensity of signal.
(9) The structure of the fraction including the peak at 7.82 minutes fractionated by TSK-GEL Carbon-500 chromatography in item (5) of Example 28 was analyzed by ¹³C-nuclear magnetic resonance (NMR) spectrum (JNM-A500, manufactured by JEOL, Ltd.). The sample was dissolved in heavy water. Figure 14 shows ¹³C-NMR spectrum. In Figure 14, the axis of abscissas is chemical shift (ppm), and the axis of ordinates is intensity of signal.
(10) The mass spectrum (MS) of the fraction including the peak at 11.09 minutes fractionated by TSK-GEL Carbon-500 chromatography in item (5) of Example 28 was analyzed by mass spectrometer (DX302, manufactured by JEOL LTD.). As the matrix, glycerol was used. As a result, peaks of m/z 131, 185, 223 and 321 were detected. Figure 15 shows the mass spectrum. In Figure 15, the axis of abscissas is m/z value, and the axis of ordinates is relative intensity.
(11) The structure of the fraction including the peak at 11.09 minutes fractionated by TSK-GEL Carbon-500 chromatography in item (5) of Example 28 was analyzed by infrared absorption (IR) spectrum (FTIR-8200PC, manufactured by Shimadzu Corporation). Figure 16 shows IR spectrum. In Figure 16, the axis of abscissas is an inverse of the wavelength of infrared ray, and the axis of ordinates is transmittance.
(12) The structure of the fraction including the peak at 11.09 minutes fractionated by TSK-GEL Carbon-500 chromatography in item (5) of Example 28 was analyzed by ¹H-nuclear magnetic resonance (NMR) spectrum (JNM-A500, manufactured by JEOL, Ltd.). The sample was dissolved in heavy water. Figure 17 shows ¹H-NMR spectrum. In Figure 17, the axis of abscissas is chemical shift (ppm), and the axis of ordinates is intensity of signal.
(13) The structure of the fraction including the peak at 11.09 minutes fractionated by TSK-GEL Carbon-500 chromatography in item (5) of Example 28 was analyzed by ¹³C-nuclear magnetic resonance (NMR) spectrum (JNM-A500, manufactured by JEOL, Ltd.). The sample was dissolved in heavy water. Figure 18 shows ¹³C-NMR spectrum of the fraction including the peak. In Figure 18, the axis of abscissas is chemical shift (ppm), and the axis of ordinates is intensity of signal.

### Example 29

(1) Four-hundred and eighty grams of a dry product of *Angelica keiskei* koidz. (manufactured by Sakamoto Kanpodo) was powdered with a food processor, and the powder was extracted twice with about one-liter of ethyl acetate. The resulting organic layer fraction was concentrated under reduced pressure, and thereafter the concentrate was subjected to silica chromatography. The adsorbed substances were stepwise eluted with
chloroform : methanol= 100:1 (600 ml) and 12:1 (600 ml), in this order, and collected in 8-mL aliquots per fraction. The resulting fractions 76 and on were collected and concentrated under reduced pressure, and the concentrate was subjected to silica chromatography using hexane : ethyl acetate = 1.8:1 as a developing solvent, to give fractions containing xanthoangelol in a high concentration, which consist of fractions 25 to 50. By recrystallization from these fractions with ethyl acetate and hexane, about 200 mg of high-purity xanthoangelol was obtained. The determination results for NMR spectrum are given below:
¹H-NMR: δ 1.58 (3H, s, -Me), 1.66 (3H, s, -Me), 1.81 (3H, s, -Me), 2.08 (4H, m), 3.48 (2H, d, J 7 Hz), 5.04 (1H, m), 5.29 (1H, m), 6.40 (1H, d J_{5',6}, 9 Hz, H-5'), 6.86 (2H, d, J_{5,6} 9, J_{2,3} 9 Hz, H-2, 6), 7.45 (1H, d, J_{α,β} 15 Hz, H-α), 7.54 (2H, d, H-3, 5), 7.71 (1H, d, H-6'), 7.82 (1H, d, H-β)
Here, the sample was dissolved in deuterated chloroform, and the chemical shift of the residual chloroform was expressed as 2.49 ppm.
(2) The enhancing activity for NGF production of xanthoangelol prepared in item (1) of Example 29 was assayed in the same manner as in Example 13. Xanthoangelol was added to the medium so as to have a concentration of 25, 50 or 100 µM. The results are shown in Table 42. Xanthoangelol enhanced NGF production of L-M cells.

**Table 42**

| Concentration of Xanthoangelol (µM) | Amount of NGF Produced (%) |
|---|---|
| 25 | 126.1 |
| 50 | 202.6 |
| 100 | 265.7 |
| (Here, the amount of NGF produced in the control was 0.199 ng/ml.) | |

### Example 30

(1) The amount 5.8 kg of a dry powder of root portions of *Angelica keiskei* koidz. was extracted with 24 liters of ethyl acetate at room temperature for 3 hours. The residue after the suction filtration was extracted overnight at room temperature with 18 liters of ethanol. Next, the residue after the suction filtration was extracted with 52 liters of distilled water at 60°C for 3 hours. A liquid portion resulting from removal of the solid residue was concentrated with a rotary evaporator, and a 2.5-fold amount of ethanol was added to the concentrate, and the mixture was allowed to stand overnight at 4°C. Thereafter, the mixture was fractionated by suction filtration into precipitates and a liquid portion. The liquid portion was concentrated to dryness with a rotary evaporator, to give a water-extracted, low-molecular fraction of root portions of *Angelica keiskei* koidz. Next, the water-extracted, low-molecular fraction of root portions of *Angelica keiskei* koidz. was applied to Amberlite XAD-2 (manufactured by Organo; the amount of resin: 2 liters), and the non-adsorbed substances were sufficiently washed out with 30 liters of distilled water. Next, the adsorbed substances were eluted with 16 liters of methanol. The methanol eluate was concentrated to dryness with a rotary evaporator, to give a water-extracted, low-molecular XAD-2-treated fraction of root portions of *Angelica keiskei* koidz.
(2) The enhancing activity for NGF production of the above-mentioned water-extracted, low-molecular XAD-2-treated fraction of root portions of *Angelica keiskei* koidz. described in item (1) of Example 30 was assayed in the same manner as in Example 13. The water-extracted, low-molecular XAD-2-treated fraction of root portions of *Angelica keiskei* koidz. was added to the medium so as to have a concentration of 0.85, 1.7 or 3.4 mg/ml. As shown in Table 43, the water-extracted, low-molecular XAD-2-treated fraction of root portions of *Angelica keiskei* koidz. enhanced NGF production of L-M cells in a concentration-dependent manner.

**Table 43**

| | | | | |
|---|---|---|---|---|
| Sample Concentration (mg/ml) | 0 | 0.85 | 1.7 | 3.4 |
| NGF (%) | 100 | 655.3 | 858.8 | 1127.6 |
| (Here, the amount of NGF produced in the control was 0.074 ng/ml.) | | | | |

(3) The active component of the water-extracted, low-molecular XAD-2-treated fraction of root portions of *Angelica keiskei* koidz. described in item (1) of Example 30 was fractionated using reverse phase chromatography. The conditions therefor are given below. The resin used was Cosmosil 140 C₁₈-OPN (manufactured by nakalaitesque, amount of resin: 400 ml). The water-extracted, low-molecular XAD-2-treated fraction of root portions of *Angelica keiskei* koidz. was applied to the column and eluted using as the developing solvents 1 liter each of distilled water, a 20% aqueous acetonitrile solution, a 25% aqueous acetonitrile solution, a 40% aqueous acetonitrile solution, and methanol, in this order. Each eluted fraction was concentrated under reduced pressure, to prepare each product fractionated by Cosmosil.
(4) The enhancing activity for NGF production of the product fractionated by Cosmosil 140 chromatography described in item (3) of Example 30 was assayed in the same manner as in Example 13. As a result, each of the fraction eluted with a 20% aqueous acetonitrile solution, the fraction eluted with a 25% aqueous acetonitrile solution, and the fraction eluted with a 40% aqueous acetonitrile solution has enhancing activity for NGF production. The results are shown in Table 44.

**Table 44**

| Fraction | Sample Concentration (mg/ml) | Amount of NGF Produced (%) |
|---|---|---|
| Fraction Eluted with 20% Aqueous Acetonitrile Solution | 0 | 100 |
| | 4.05 | 301.0 |
| | 8.1 | 436.7 |
| | 16.2 | 1263.3 |
| Fraction Eluted with 25% Aqueous Acetonitrile Solution Fraction Eluted with 40% Aqueous Acetonitrile Solution | 0.7 | 161.7 |
| | 1.5 | 1028.8 |
| | 2.8 | 2110.4 |
| | 0.575 | 465.3 |
| | 1.15 | 653.1 |
| | 2.3 | 1226.2 |
| (Here, the amount of NGF produced in the control for the fraction eluted with a 20% aqueous acetonitrile solution was 0.074 ng/ml, and that for the fraction eluted with a 25% aqueous acetonitrile solution and the fraction eluted with a 40% aqueous acetonitrile solution was 0.087 ng/ml.) | | |

(5) The active component of the fraction eluted with a 25% aqueous acetonitrile solution of Cosmosil 140 described in item (3) of Example 30 was fractionated using reverse phase chromatography.
The conditions therefor are given below. The column used was TSK gel ODS 80Ts (diameter: 21.5 mm, length: 30 cm, manufactured by Tosoh Corporation). The elution ratio of Solvent A (distilled water) and Solvent B (mixture of distilled water and acetonitrile in a volume ratio of 1:1) was such that the ratio of Solvent B was increased linearly from 25 to 100% from 0 to 120 minutes, the ratio of Solvent B was retained at 100% for the subsequent 20 minutes, and the ratio of Solvent B was finally decreased to 25% and retained thereat for 20 minutes. The elution rate was 5 ml/minute, and the detection was carried out at 235 nm. The fractions were collected using ultraviolet absorption as an index.
(6) The activity of the fractionated product of ODS-80Ts chromatography of the fraction eluted with a 25% aqueous acetonitrile solution of Cosmosil described in item (5) of Example 30 was assayed in the same manner as in Example 13. As a result, it was clarified that many of the fractions have enhancing activity for NGF production. The results are shown in Table 45.

**Table 45**

| Fractionated Fraction (Detected Peaks: minutes) | Concentration (mg/ml) | Amount of NGF Produced (%) |
|---|---|---|
| 1 (46.0, 47.2, 49.0) | 2.00 | 250.8 |
| 2 (53.2) | 2.00 | 275.2 |
| 3 (54.0) | 2.00 | 318.7 |
| 4 (54.9, 55.5, 56.4) | 2.00 | 293.0 |
| 5 (57.6) | 2.00 | 320.5 |
| 6 (58.6) | 2.00 | 297.8 |
| 7 (59.3) | 2.00 | 324.8 |
| 8 (60.5) | 2.00 | 324.8 |
| 9 (61.3) | 2.00 | 402.8 |
| 10 (62.2) | 2.00 | *565.5* |
| 11 (63.0) | 2.00 | 616.9 |
| 12 (64.1) | 2.00 | 575.4 |
| 13 (66.9) | 2.00 | 805.3 |
| 14 (68.4) | 2.00 | 819.6 |
| 15 (69.2) | 2.00 | 510.2 |
| 16 (70.3) | 1.00 | 389.2 |
| 17 (71.3, 71.9) | 1.00 | 609.1 |
| 18 (73.0, 73.8, 74.9) | 0.50 | 1002.5 |
| 19 (75.4) | 0.50 | 851.3 |
| 20 (76.5) | 1.00 | 838.5 |
| 21 (77.7) | 1.00 | 249.4 |
| 22 (79.6, 80.5) | 0.50 | 234.3 |
| 23 (82.4) | 0.25 | 359.1 |
| 24 (84.1) | 0.25 | 285.8 |
| 25 (85.5) | 0.0625 | 359.1 |
| 26 (86.9, 87.5) | 0.10 | 411.6 |
| 27 (89.1) | 0.50 | 443.3 |
| 28 (91.4) | 0.05 | 1058.1 |
| 29 (92.8) | 0.20 | 672.0 |
| 30 (93.8, 95.8, 97.5, 100.0, 100.6) | 0.50 | 593.6 |
| (Here, the amount of NGF produced in the control for fractions 1 to 9 was 0.355 ng/ml, that for fractions 10 to 18 was 0.382 ng/ml, that for fractions 19 to 27 was 0.415 ng/ml, and that for fractions 28 to 30 was 0.450 ng/ml.) | | |

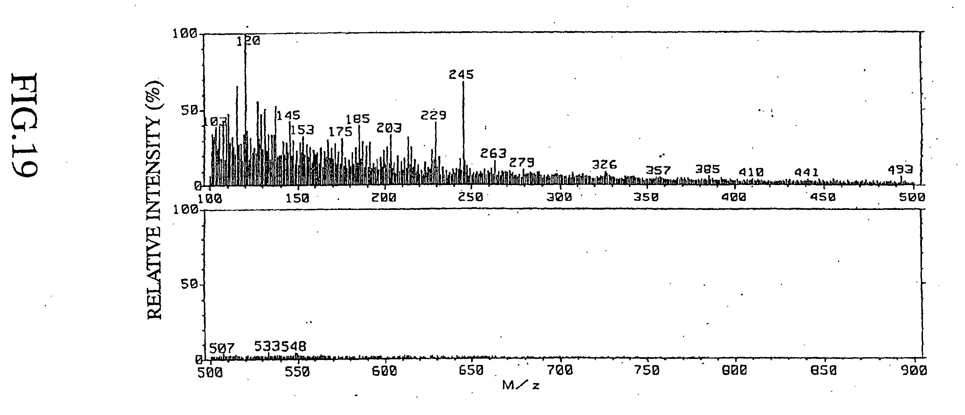

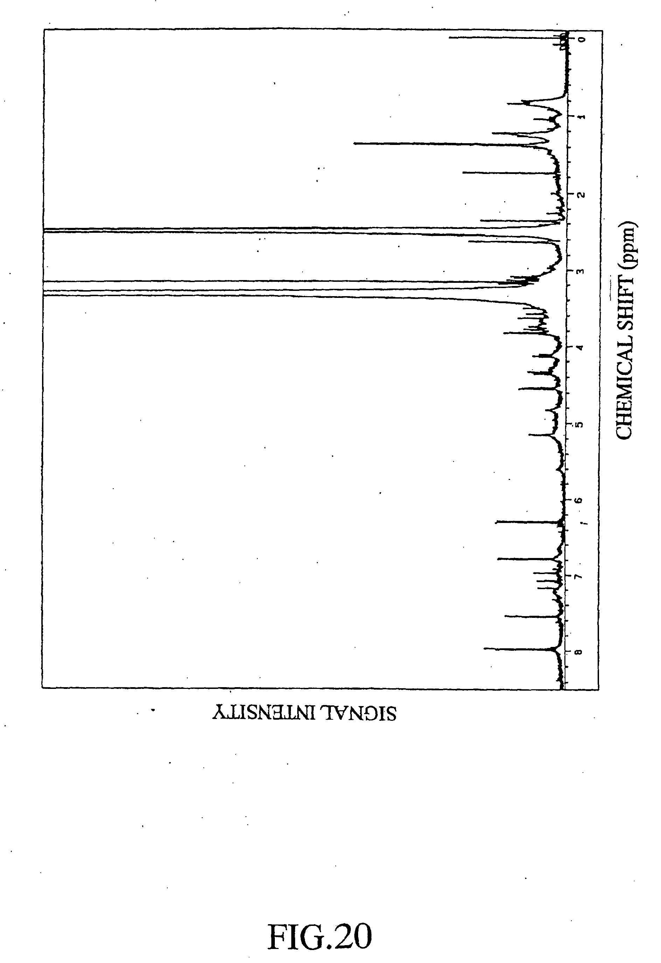

(7) The mass spectrum (MS) of the fraction 10 derived from root portions of *Angelica keiskei* koidz. (fraction including a peak detected at a retention time of 62.2 minutes) of which activity was confirmed in item (6) of Example 30 was measured by mass spectrometer (DX302, manufactured by JEOL LTD.) by FAB-MS technique. As the matrix, glycerol was used. As a result, a peak of m/z 245 (M-OGlc)⁺ was detected. Figure 19 shows the FAB-MS spectrum of the fraction 10 derived from root portions of *Angelica keiskei* koidz. In Figure 19, the axis of abscissas is m/z value, and the axis of ordinates is relative intensity.
The structure of the fraction 10 derived from root portions of *Angelica keiskei* koidz. was analyzed by measuring various NMR spectra using nuclear magnetic resonance (NMR) spectrometer (JNM-A500, manufactured by JEOL, Ltd.). The signals of NMR are shown below.
¹H-NMR: δ 1.36 (3H, s, 2'-CH₃), 1.37 (3H, s, 2'-CH₃), 3.08 (2H, m, 2"-H and 4"-H), 3.12 (1H, m, 3"-H), 3.41 (1H, m, 5"-H), 3.81 (1H, d, J=4.5 Hz, 3'-H), 4.11 (1H, dd, J=7.0, 11.5 Hz, 6"-H), 4.35 (1H, brd, J=11.5 Hz, 6"-H), 4.53 (1H, d, J=7.5 Hz, 1"-H), 5.14 (1H, d, J=4.5 Hz, 4'-H), 6.28 (1H, d, J=9.5 Hz, 3-H), 6.78 (1H, d, J=8.5 Hz, 6-H), 7.54 (1H, d, J=8.5 Hz, 5-H), 7.98 (1H, d, J=9.5 Hz, 4-H)
Here, in ¹H-NMR, the sample was dissolved in deuterated dimethyl sulfoxide, and the chemical shift of the residual dimethyl sulfoxide was expressed as 2.49 ppm. Figure 20 shows ¹H-NMR spectrum of the fraction 10 derived from root portions of *Angelica keiskei* koidz. In Figure 20, the axis of abscissas is chemical shift (ppm), and the axis of ordinates is intensity of signal.
As a result of the analyses of the mass spectrum and NMR spectra for the fraction 10 derived from root portions of *Angelica keiskei* koidz., it was identified that the active component is 3'-O-β-D-glucopyranoyl khellactone (molecular weight: 424).
(8) The fraction 13 (the fraction including the peak detected at a retention time of 66.9 minutes), of which potent activity was confirmed in item (6) of Example 30, was further fractionated using reverse phase chromatography.
The conditions therefor are given below. The column used was TSK gel ODS 80TsQA (diameter: 4.6 mm, length: 25 cm, manufactured by Tosoh Corporation). The elution ratio of Solvent A (distilled water containing 0.1% trifluoroacetic acid) and Solvent B (mixture of distilled water and acetonitrile in a volume ratio of 1:1, containing 0.1% trifluoroacetic acid) was such that the ratio of Solvent B was retained at 50% for 20 minutes. The elution rate was 1 ml/minute, and the detection was carried out at 235 nm. The fractions were collected using ultraviolet absorption as an index.
(9) The activity of the fractions fractionated by TSK gel ODS 80TsQA chromatography in item (8) of Example 30 was assayed in the same manner as in Example 13. As a result, it was clarified that each of the fractions including peaks detected at retention time of 7.9, 8.7, 9.2, 10.2, 11.5, 12.7 and 13.9 minutes has the enhancing activity for NGF production. The results are shown in Table 46.

**Table 46**

| Fractionated Fraction (Detected Peaks: minutes) | Concentration (mg/ml) | Amount of NGF Produced (%) |
|---|---|---|
| 13-1 (7.9) | 0.56 | 654.3 |
| 13-2 (8.7) | 1.00 | 792.1 |
| 13-3 (9.2) | 0.56 | 366.9 |
| 13-4 (9.7) | 0.96 | 363.9 |
| 13-5 (10.2) | 0.96 | 361.0 |
| 13-6 (11.5) | 1.00 | 232.0 |
| 13-7 (12.7) | 0.50 | 261.3 |
| 13-8 (13.9) | 1.00 | 630.8 |
| (Here, the amount of NGF produced in the control was 0.053 ng/ml.) | | |

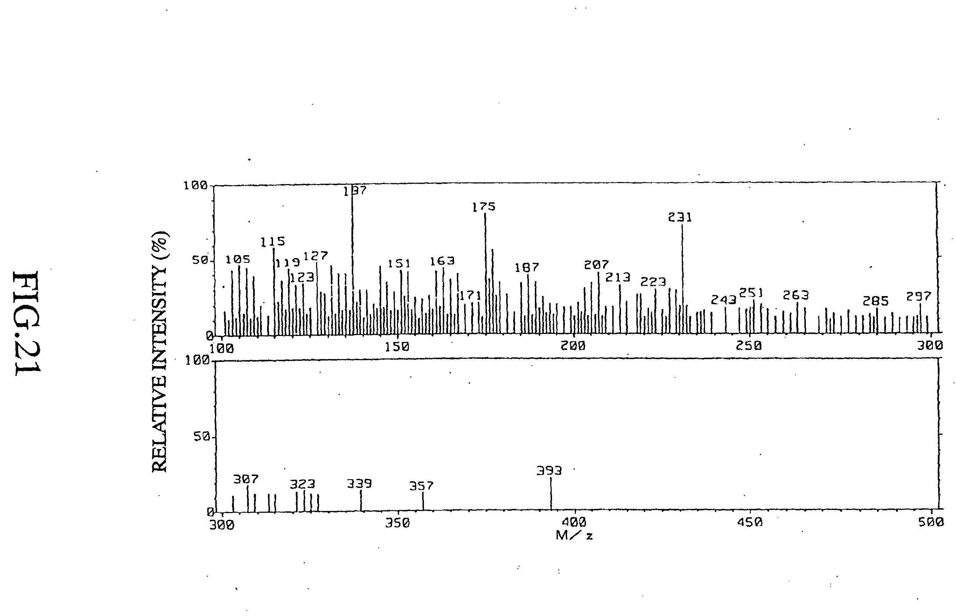

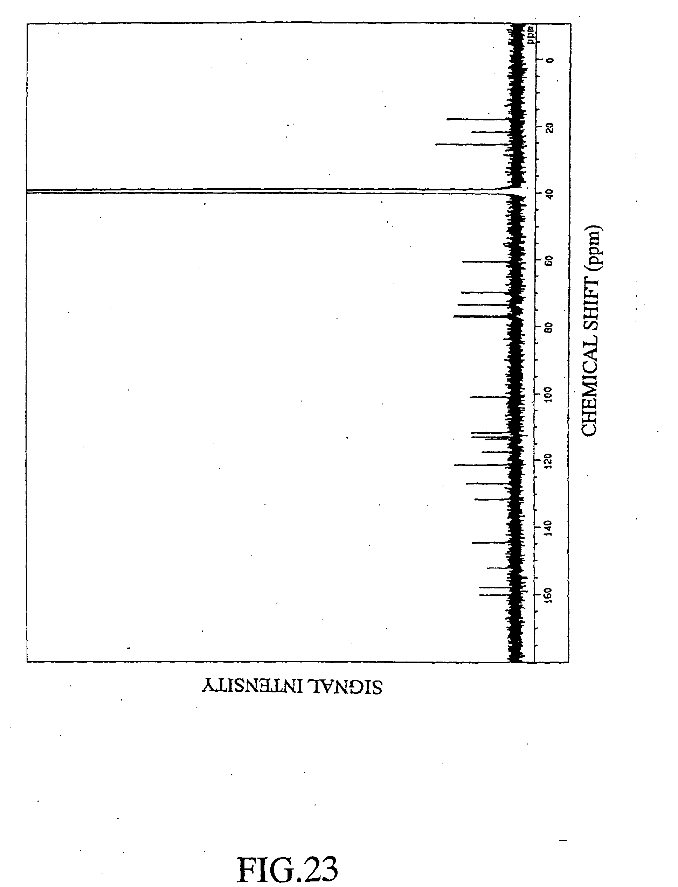

(10) The mass spectrum and the NMR spectrum of the fraction 13-2 derived from root portions of *Angelica keiskei* koidz., of which activity was confirmed in item (9) of Example 30, were determined in the same manner as in item (7) of Example 30.
According to the mass spectroscopy, a peak of m/z 393 (M+H)⁺ was detected. Figure 21 shows the MS spectrum of the fraction 13-2 derived from root portions of *Angelica keiskei* koidz. In Figure 21, the axis of abscissas is m/z value, and the axis of ordinates is relative intensity.
The signals of nuclear magnetic resonance (NMR) for the fraction 13-2 derived from root portions of *Angelica keiskei* koidz. are shown below.
¹H-NMR: δ 1.61 (3H, s, 3'-CH₃), 1.78 (3H, s, 3'-CH₃), 3.17 (1H, t, J=9.5 Hz, 4"-H), 3.28 (1H, m, 3"-H), 3.29 (1H, m, 2"-H), 3.38 (1H, m, 5"-H), 3.40 (1H, m, 1'-H), 3.46 (1H, m, 6"-H), 3.58 (1H, m, 1'-H), 3.69 (1H, m, 6"-H), 4.94 (1H, d, J=7.5 Hz, 1"-H), 5.20 (1H, m, 2'-H), 6.30 (1H, d, J=9.5 Hz, 3-H), 7.11 (1H, d, J=8.5 Hz, 6-H), 7.51 (1H, d, J=8.5 Hz, 5-H), 7.98 (1H, d, J=9.5 Hz, 4-H)

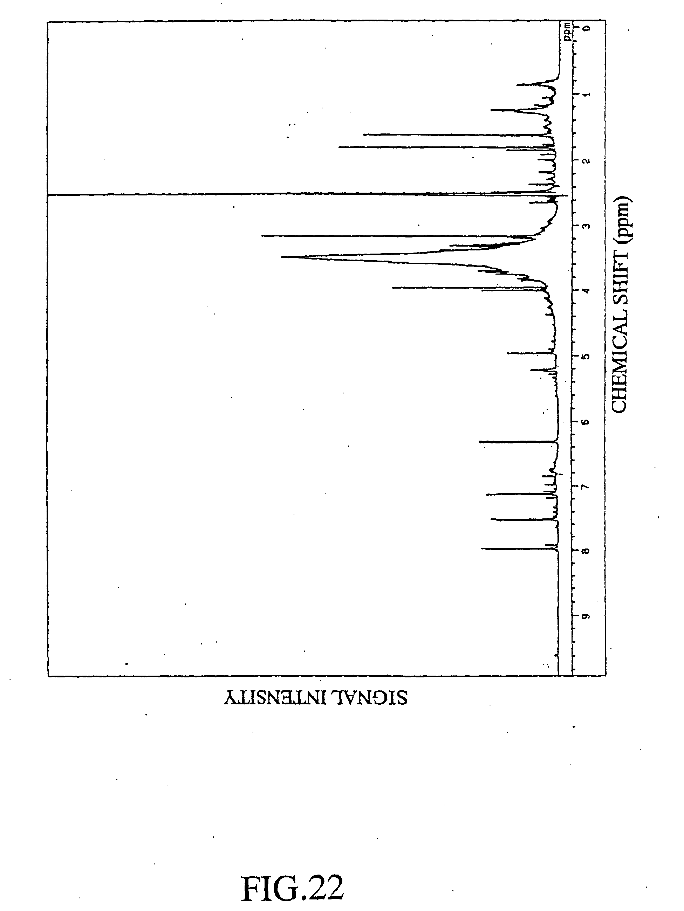

Figure 22 shows ¹H-NMR spectrum of the fraction 13-2 derived from root portions of *Angelica keiskei* koidz. In Figure 22, the axis of abscissas is chemical shift (ppm), and the axis of ordinates is intensity of signal.
¹³C-NMR: δ 17.8 (3'-CH₃), 21.6 (1'-c), 25.5 (3'-CH₃), 60.6 (6"-c), 69.7 (4"-c), 73.4 (2"-c), 76.7 (3"-c), 77.1 (5"-c), 100.8 (1"-c), 111.5 (6-c), 112.8 (3-c), 113.4 (10-c), 117.4 (8-c), 121.3 (2'-c), 126.8 (5-c), 131.5 (3'-c), 144.5 (4-c), 152.1 (9-c), 157.8 (7-c), 160.2 (2-c)
Here, in ¹³C-NMR, the sample was dissolved in deuterated dimethyl sulfoxide, and the chemical shift of the deuterated dimethyl sulfoxide was expressed as 39.5 ppm. Figure 23 shows ¹³C-NMR spectrum of the fraction 13-2 derived from root portions of *Angelica keiskei* koidz. In Figure 23, the axis of abscissas is chemical shift (ppm), and the axis of ordinates is intensity of signal.
As a result of the analyses of the mass spectrum and NMR spectra for the fraction 13-2 derived from root portions of *Angelica keiskei* koidz., it was identified that the active component is 7-O-β-D-glucopyranosyloxy-8-prenylcoumarin (molecular weight: 392).
(11) The fraction 18 (the fraction including the peaks detected at retention time of 73.0, 73.8 and 74.97 minutes), of which potent activity was confirmed in item (6) of Example 30, was further fractionated using reverse phase chromatography in the same manner as in item (8) of Example 30.
(12) The activity of the fractions fractionated by TSK gel ODS 80TsQA chromatography in item (11) of Example 30 was assayed in the same manner as in Example 13. As a result, it was clarified that each of the fractions including peaks detected at retention time of 9.3, 10.1, 10.6, 11.2, 12.0, 12.8, 13.3, 14.0, 15.2, 16.1, and 18.6 minutes has the enhancing activity for NGF production. The results are shown in Table 47.

**Table 47**

| Fractionated Fraction (Detected Peaks: minutes) | Concentration (mg/ml) | Amount of NGF Produced (%) |
|---|---|---|
| 18-1 (9.3) | 0.20 | 1489.6 |
| 18-2 (10.1) | 0.50 | 2222.9 |
| 18-3 (10.6) | 0.25 | 3039.6 |
| 18-4 (10.9) | 0.0375 | 2510.4 |
| 18-5 (11.2) | 0.125 | 610.4 |
| 18-6 (12.0) | 0.50 | 560.4 |
| 18-7 (12.8) | 0.50 | 681.3 |
| 18-8 (13.3) | 0.40 | 339.6 |
| 18-9 (14.0) | 1.00 | 410.4 |
| 18-10 (15.2) | 1.00 | 2510.7 |
| 18-11 (16.1) | 1.00 | 3360.7 |
| 18-12 (18.6) | 1.00 | 1189.3 |
| (Here, the amount of NGF produced in the control for fractions 18-1 to -9 was 0.027 ng/ml, and that for fractions 18-10 to -12 was 0.015 ng/ml.) | | |

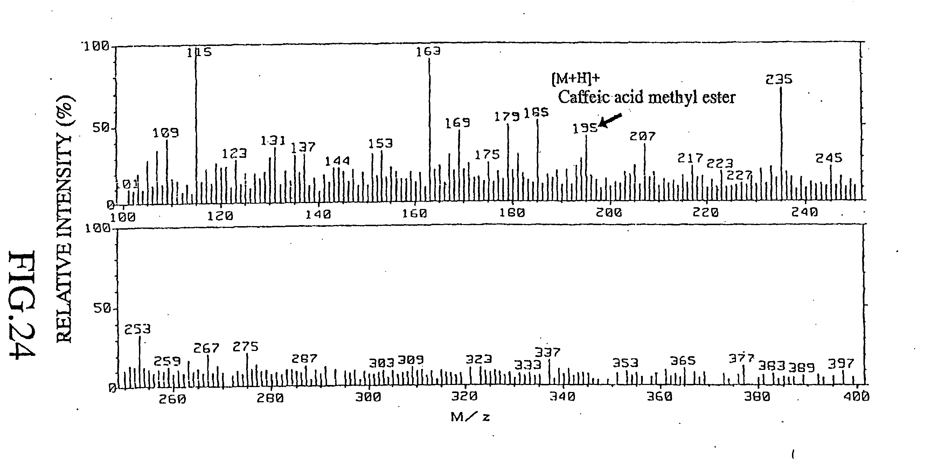

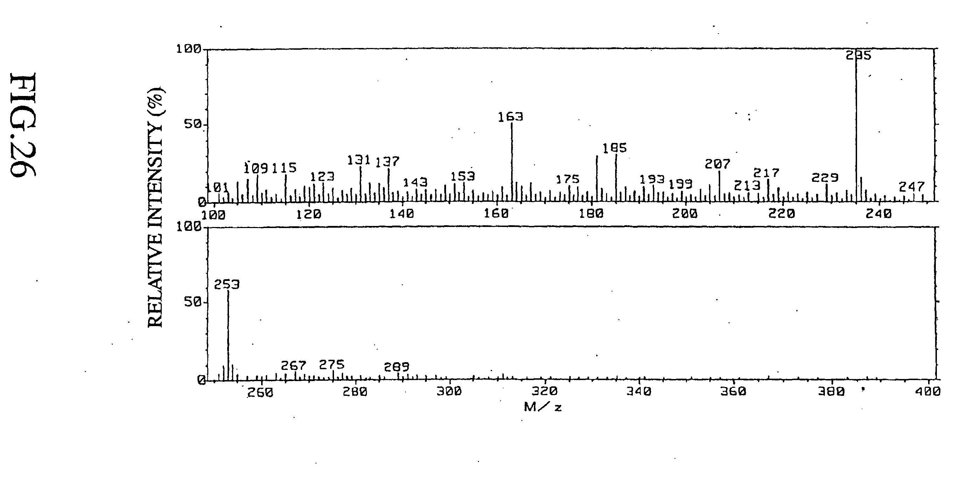

(13) The mass spectrum and the NMR spectrum of the fraction 18-3 derived from root portions of *Angelica keiskei* koidz., of which activity was confirmed in item (12) of Example 30, were determined in the same manner as in item (7) of Example 30.
According to the mass spectroscopy, a peak of m/z 195 (M+H)⁺ was detected. Figure 24 shows the MS spectrum of the fraction 18-3 derived from root portions of *Angelica keiskei* koidz. In Figure 24, the axis of abscissas is m/z value, and the axis of ordinates is relative intensity.
The signals of NMR are shown below.
¹H-NMR: δ 3.68 (3H, s, OCH₃), 6.25 (1H, d, J=16.0 Hz, 2'-H), 6.75 (1H, d, J=8.0 Hz, 5-H), 6.98 (1H, dd, J=2.0, 8.0 Hz, 6-H), 7.03 (1H, d, J=2.0, 2-H), 7.46 (1H, d, J=16.0 Hz, 3'-H), 9.10 (1H, s, 3-OH), 9.56 (1H, s, 4-OH)

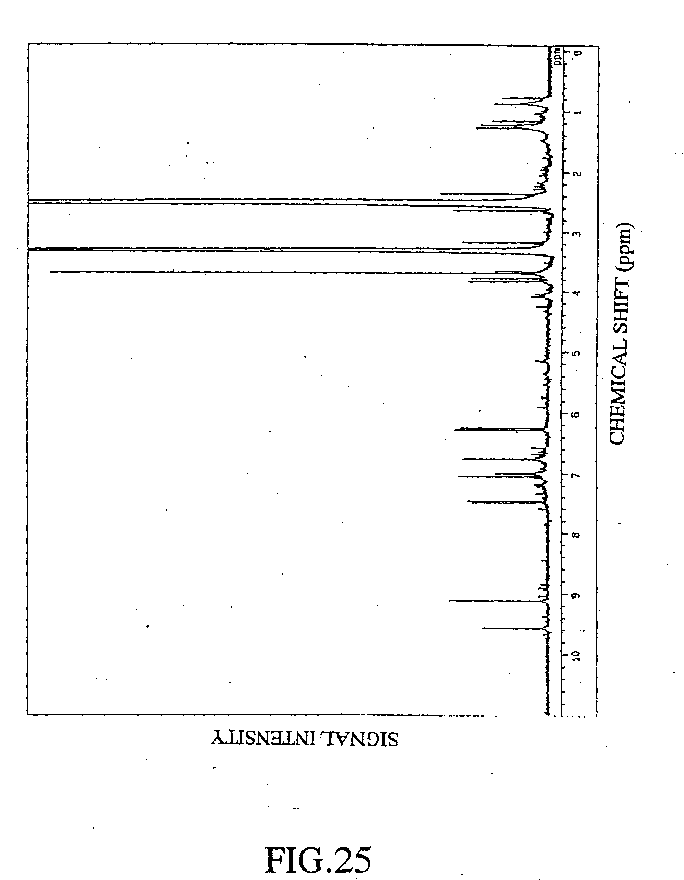

Figure 25 shows ¹H-NMR spectrum of the fraction 18-3 derived from root portions of *Angelica keiskei* koidz. In Figure 25, the axis of abscissas is chemical shift (ppm), and the axis of ordinates is intensity of signal.
As a result of the analyses of the mass spectrum and NMR spectrum for the fraction 18-3 derived from root portions of *Angelica keiskei* koidz., it was identified that the active component is caffeic acid methyl ester (molecular weight: 194).
(14) The mass spectrum and the NMR spectrum of the fraction 18-4 derived from root portions of *Angelica keiskei* koidz., of which activity was confirmed in item (12) of Example 30, were determined in the same manner as in item (5) of Example 30.
According to the mass spectroscopy, a peak of m/z 253 (M+H)⁺ was detected. Figure 26 shows the MS spectrum of the fraction 18-4 derived from root portions of *Angelica keiskei* koidz. In Figure 26, the axis of abscissas is m/z value, and the axis of ordinates is relative intensity.
The signals of NMR are shown below.
¹H-NMR: δ 1.15 (3H, s, 2-CH₃), 1.27 (3H, s, 2-CH₃), 2.39 (1H, dd, J=8.0, 17.0 Hz, 4-H), 2.76 (,1H, dd, J=5.0, 17.0 Hz, 4-H), 3.62 (1H, m, 3-H), 3.81 (3H, s, 5-OCH₃), 5.16 (1H, d, J=4.5 Hz, 3-OH), 6.56 (1H, d, J=9.0 Hz, 6-H), 7.59 (1H, d, J=9.0 Hz, 10-H), 11.86 (1H, brs, 8-COOH)

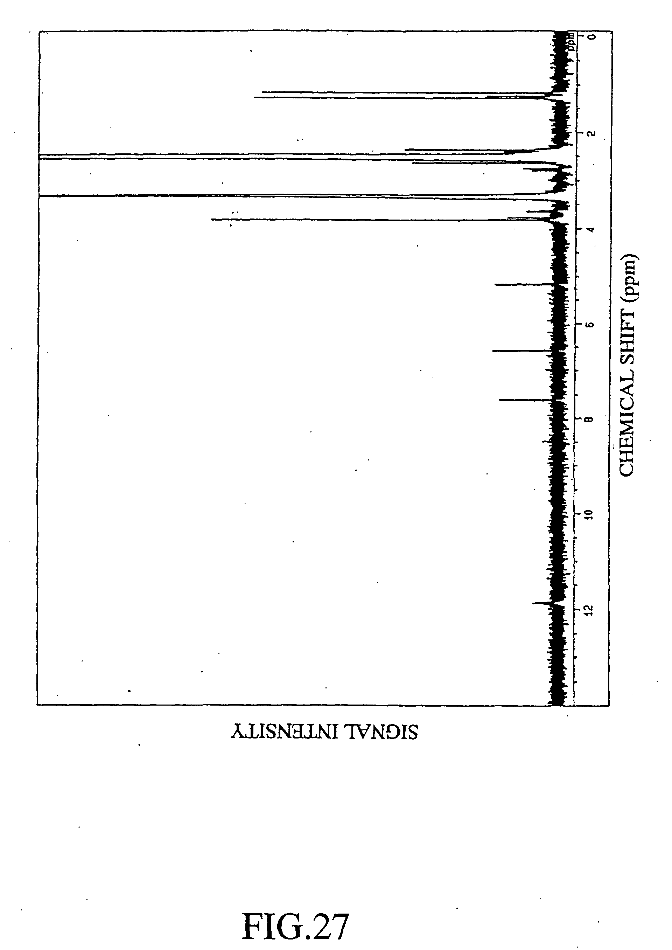

Figure 27 shows ¹H-NMR spectrum of the fraction 18-4 derived from root portions of *Angelica keiskei* koidz. In Figure 27, the axis of abscissas is chemical shift (ppm), and the axis of ordinates is intensity of signal.
¹³C-NMR: δ 20.4 (2-CH₃), 25.4 (2-CH₃), 26.2 (4-C), 55.7 (5-OCH₃), 67.0 (3-C), 77.6 (2-C), 101.8 (6-C), 109.4 (10-C), 112.5 (8-C), 130.7 (7-C), 153.3 (9-C), 160.4 (5-C), 166.6 (8-COOH)

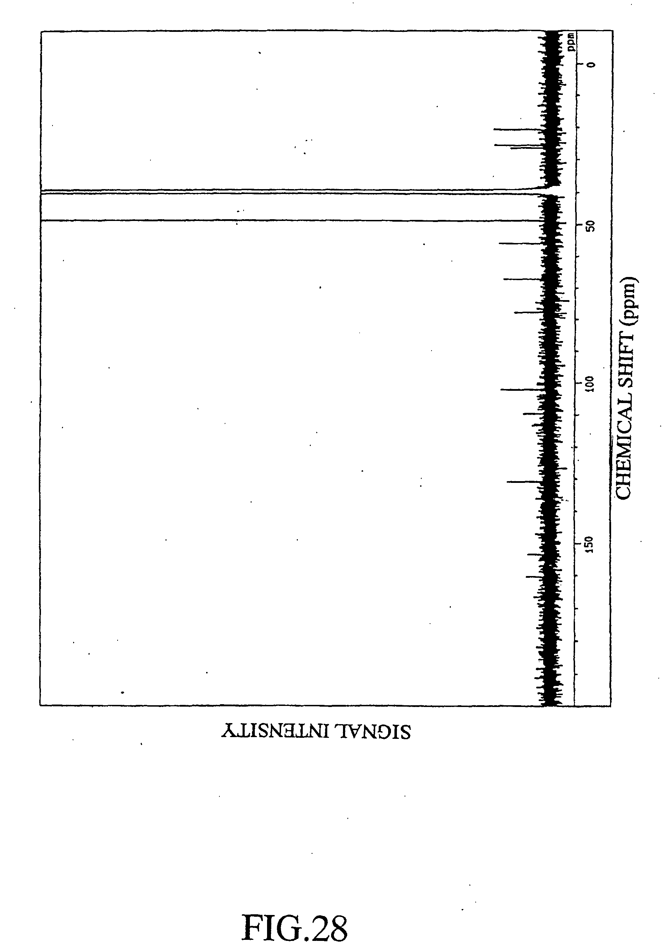

Figure 28 shows ¹³C-NMR spectrum of the fraction 18-4 derived from root portions of *Angelica keiskei* koidz. In Figure 28, the axis of abscissas is chemical shift (ppm), and the axis of ordinates is intensity of signal.
As a result of the analyses of the mass spectrum and NMR spectra for the fraction 18-4 derived from root portions of *Angelica keiskei* koidz., it was identified that the active component is 8-carboxyl-3-hydroxy-5-methoxyl-2-dimethylchroman (molecular weight: 252).
(15) The fraction 19 (the fraction including a peak detected at retention time of 75.4 minutes) and the fraction 20 (the fraction including a peak detected at retention time of 76.5 minutes), each of which potent activity was confirmed in item (6) of Example 30, were mixed, and the mixture was further fractionated using reverse phase chromatography in the same manner as in item (8) of Example 30.
(16) The activity of each of the fractions fractionated by TSK gel ODS 80TsQA chromatography in item (15) of Example 30 was assayed in the same manner as in Example 13. As a result, it was clarified that each of the fractions including peaks detected at retention time of 13.7, 14.3, 15.1, 15.5, 16.4, 18.8 and 20.5 minutes has the enhancing activity for NGF production. The results are shown in Table 48.

**Table 48**

| Fractionated Fraction (Detected Peaks: minutes) | Concentration (mg/ml) | Amount of NGF Produced (%) |
|---|---|---|
| 19-, 20-1 (13.7) | 0.60 | 192.4 |
| 19-, 20-2 (14.3) | 1.00 | 246.6 |
| 19-, 20-3 (15.1) | 1.00 | 372.2 |
| 19-, 20-4 (15.5) | 0.60 | 529.8 |
| 19-, 20-5 (16.4) | 1.00 | 487.9 |
| 19-, 20-6 (18.8) | 1.00 | 337.7 |
| 19-, 20-7 (20.5) | 0.364 | 305.7 |
| (Here, the amount of NGF produced in the control was 0.063 ng/ml.) | | |

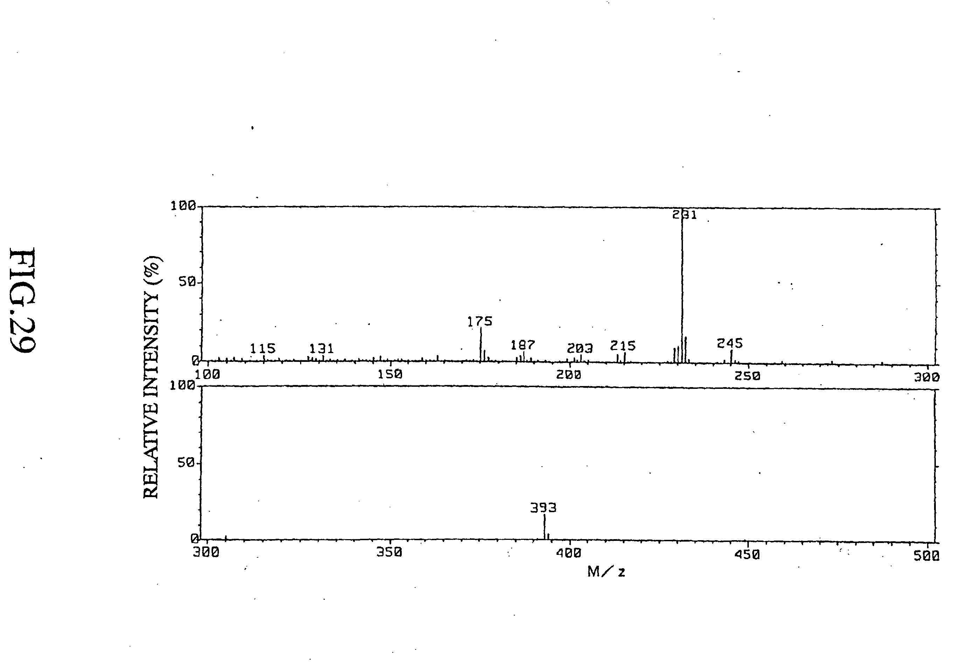

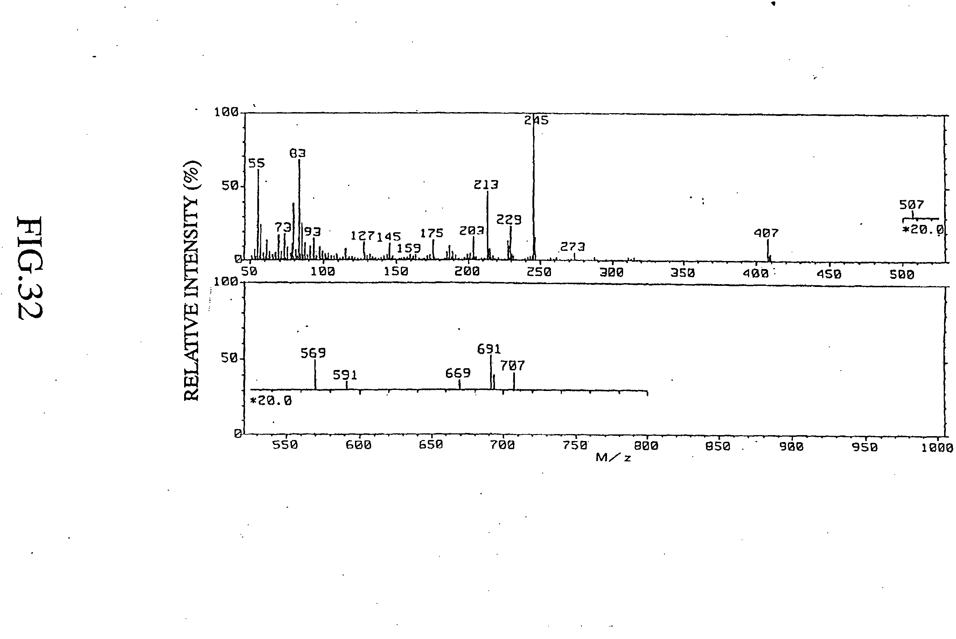

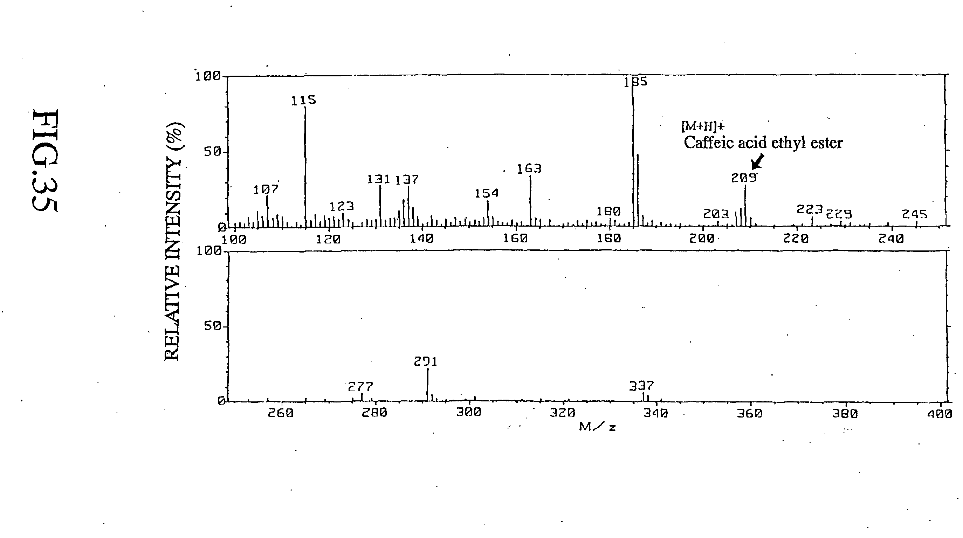

(17) The mass spectrum and the NMR spectrum of the fraction 19-, 20-5 derived from root portions of *Angelica keiskei* koidz., of which activity was confirmed in item (16) of Example 30, were determined in the same manner as in item (7) of Example 30.
According to the mass spectroscopy, a peak of m/z 393 (M+H)⁺ was detected. Figure 29 shows the MS spectrum of the fraction 19-, 20-5 derived from root portions of *Angelica keiskei* koidz. In Figure 29, the axis of abscissas is m/z value, and the axis of ordinates is relative intensity.
The signals of NMR are shown below.
¹H-NMR: δ 1.67 (3H, s, 3'-CH₃), 1.69 (3H, s, 3'-CH₃), 3.15 (1H, m, 4"-H), 3.29 (3H, m, 1'-H, 2"-H and 3"-H), 3.37 (1H, dd, J=7.5, 15.5 Hz, 1'-H), 3.45 (2H, m, 5"-H and 6"-H), 3.72 (1H, dd, J=10.5, 6"-H), 4.97 (1H, d, J=7.5 Hz, 1"-H), 5.31 (1H, t, J=7.5 Hz, 2'-H), 6.28 (1H, d, J=9.0 Hz, 3-H), 7.07 (1H, s, 5-H), 7.41 (1H, s, 8-H), 7.98(1H, d, J=9.0 Hz, 4-H)

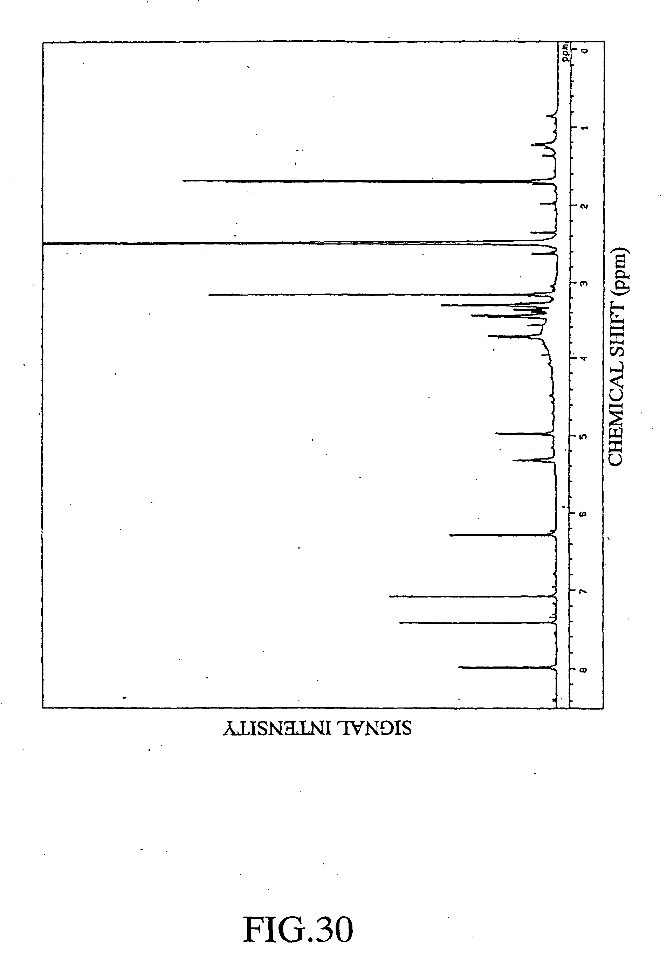

Figure 30 shows ¹H-NMR spectrum of the fraction 19-, 20-5 derived from root portions of *Angelica keiskei* koidz. In Figure 30, the axis of abscissas is chemical shift (ppm), and the axis of ordinates is intensity of signal.
¹³C-NMR: δ 17.6 (3'-CH₃), 25.5 (3'-CH₃), 27.4 (1'-C), 60.6 (6"-C), 69.6 (4"-C), 73.1 (2"-C), 76.3 (3"-C), 77.0 (5"-C), 100.4 (1"-C), 102.0 (8-C), 112.8 (10-C), 112.9 (3-C), 121.8 (2'-C), 127.4 (6-C), 128.0 (5-C), 132.4 (3'-C), 144.4 (4-C), 153.4 (9-C), 157.9 (7-C), 160.6 (2-C)

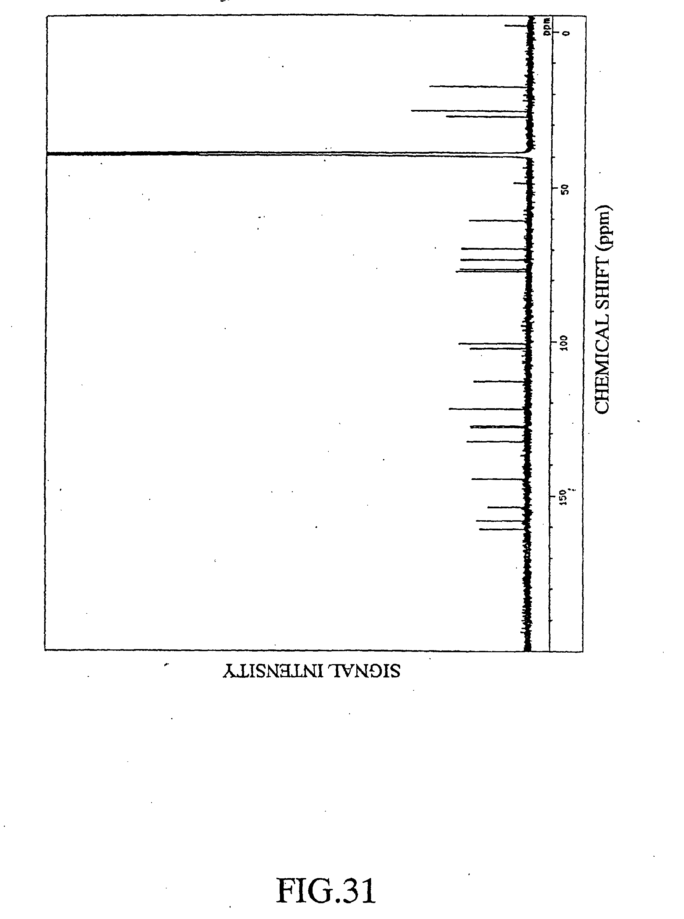

Figure 31 shows ¹³C-NMR spectrum of the fraction 19-, 20-5 derived from root portions of *Angelica keiskei* koidz. In Figure 31, the axis of abscissas is chemical shift (ppm), and the axis of ordinates is intensity of signal.
As a result of the analyses of the mass spectrum and NMR spectra for the fraction 19-, 20-5 derived from root portions of *Angelica keiskei* koidz., it was identified that the active component is 7-β-D-glucopyranosyloxy-6-prenylcoumarin (molecular weight: 392).
(18) The mass spectrum and the NMR spectrum of the fraction 19-, 20-6 derived from root portions of *Angelica keiskei* koidz. described in item (15) of Example 30 were determined.
Using a mass spectrometer, peaks of m/z 245 (M-2Glc-Angel)⁺, 669 (M+H)⁺, 691 (M+Na)⁺ and 707 (M+K)⁺ were detected. Figure 32 shows the MS spectrum of the fraction 19-, 20-6 derived from root portions of *Angelica keiskei* koidz. In Figure 32, the axis of abscissas is m/z value, and the axis of ordinates is relative intensity.
The signals of NMR are shown below.
¹H-NMR: δ 1.36 (3H, s, 2'-CH₃), 1.43 (3H, s, 2'-CH₃), 1.79 (3H, brs, 2"-CH₃), 1.86 (3H, brd, J=7.0 Hz, 3"-CH₃), 2.90 (1H, t, J=8.0 Hz, 2b-H), 2.99 (1H, m, 2a-H), 3.01 (1H, m, 4a-H), 3.02 (1H, m, 4b-H), 3.05 (1H, m, 3b-H), 3.13 (1H, t, J=9.5 Hz, 3a-H), 3.35 (1H, m, 5a-H), 3.39 (1H, m, 5b-H), 3.39 (1H, m, 6b-H), 3.49 (1H, dd, J=8.0, 11.0 Hz, 6a-H), 3.64 (1H, d, J=11.5 Hz, 6b-H), 4.02 (1H, d, J=11.0 Hz, 6a-H), 4.21 (1H, d, J=8.0 Hz, 1b-H), 4.37 (1H, d, J=4.5 Hz, 3'-H), 4.46 (1H, brs, 6b-OH), 4.48 (1H, d, J=7.5 Hz, 1a-H), 4.51 (1H, brs, 2a-OH), 4.72 (1H, brs, 3b-OH), 4.83 (1H, brs, 2b-OH), 4.86 (1H, brs, 4a-OH), 5.03 (2H, brs, 4b-OH, 3a-OH), 5.96 (1H, brq, J=7.0 Hz, 3"-H), 6.26 (1H, d, J=9.5 Hz, 3-H), 6.61 (1H, d, J=4.5 Hz, 4'-H), 6.83 (1H, d, J=8.5 Hz, 6-H), 7.59 (1H, d, J=8.5 Hz, 5-H), 7.96 (1H, d, J=9.5 Hz, 4-H)

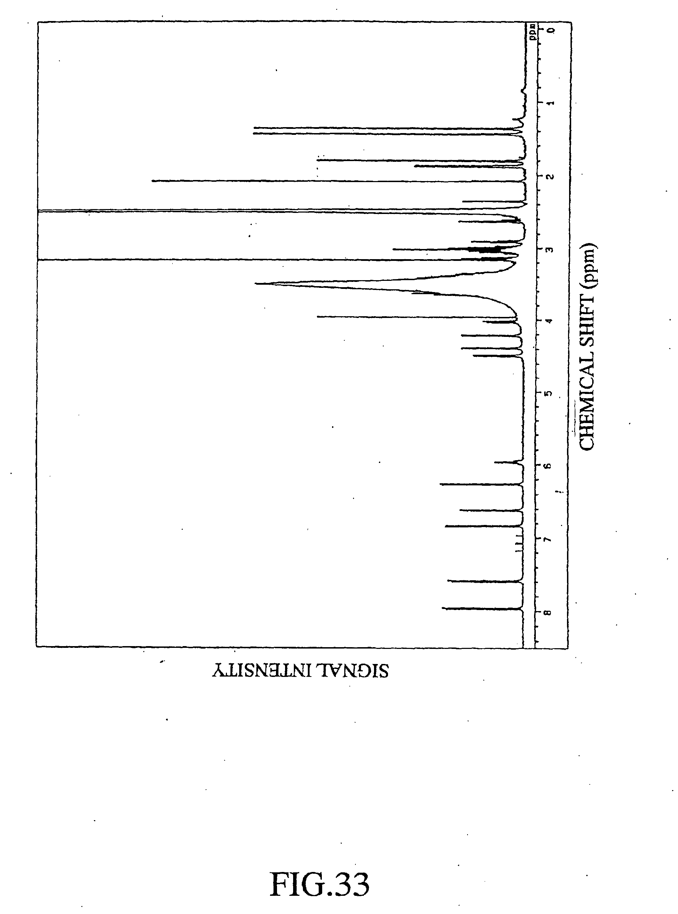

Figure 33 shows ¹H-NMR spectrum of the fraction 19-, 20-6 derived from root portions of *Angelica keiskei* koidz. In Figure 33, the axis of abscissas is chemical shift (ppm), and the axis of ordinates is intensity of signal.
¹³C-NMR: δ 15.2 (3"-CH₃), 20.0 (2"-CH₃), 21.3 (2'-CH₃), 26.4 (2'-CH₃), 59.1 (4'-C), 61.0 (6b-C), 69.2 (6a-C), 70.0 (glucose-C), 70.4 (glucose-C), 73.4 (glucose-C), 73.7 (3'-C), 73.9 (glucose-C), 76.1 (glucose-C), 76.55 (glucose-C), 76.59 (glucose-C), 76.8 (glucose-C), 77.8 (2'-C'), 100.5 (1a-C), 103.8 (1b-C), 107. (8-C), 112.1 (3-C), 112.1 (10-C), 114.2 (6-C), 128.0 (2"-C), 129.8 (5-C), 136.1 (3"-C), 144.5 (4-C), 153.6 (9-C), 156.2 (7-C), 159.4 (2-C), 166.3 (1"-C)

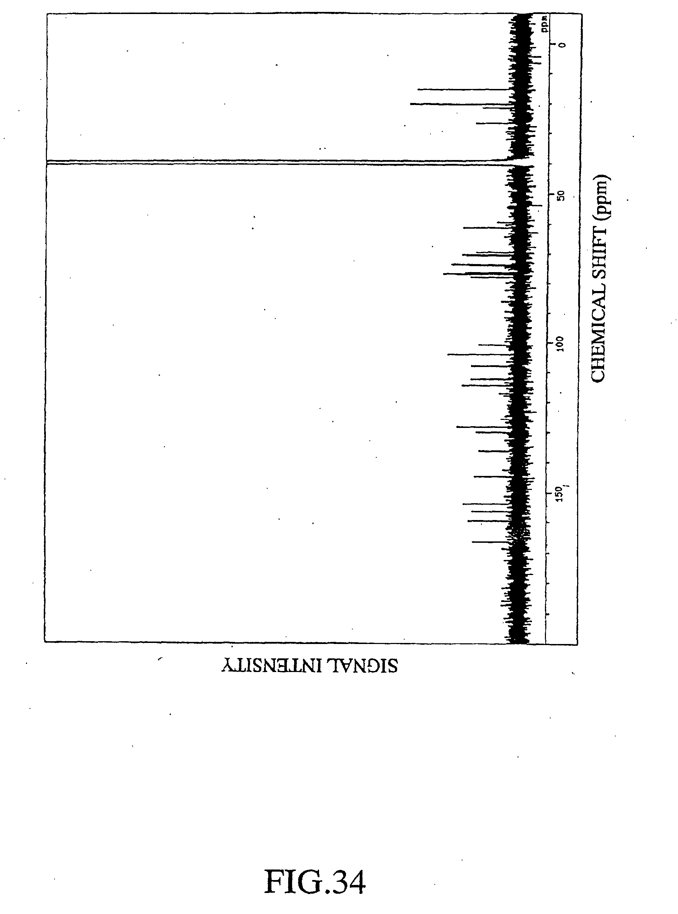

Figure 34 shows ¹³C-NMR spectrum of the fraction 19-, 20-6 derived from root portions of *Angelica keiskei* koidz. In Figure 34, the axis of abscissas is chemical shift (ppm), and the axis of ordinates is intensity of signal.
As a result of the analyses of the mass spectrum and NMR spectra for the fraction 19-, 20-6 derived from root portions of *Angelica keiskei* koidz., it was identified that the active component is 4'-O-angeloyl-3'-O-[6-O-(β-D-glucopyranosyl)-β-D-glucopyranosyl]-khellactone (molecular weight: 668).
(19) The mass spectrum and the NMR spectrum of the fraction 28 derived from root portions of *Angelica keiskei* koidz. (the fraction including the peak detected at a retention time of 91.4 minutes), of which activity was confirmed in item (6) of Example 30, were determined in the same manner as in item (7) of Example 30.
According to the mass spectroscopy, a peak of m/z 209 (M+H)⁺ was detected. Figure 35 shows the MS spectrum of the fraction 28 derived from root portions of *Angelica keiskei* koidz. In Figure 35, the axis of abscissas is m/z value, and the axis of ordinates is relative intensity.
The signals of NMR are shown below.
¹H-NMR: δ 1.23 (3H, t, J=7.0 Hz, 2"-H), 4.14 (2H, q, J=7.0 Hz, 1"-H), 6.24 (1H, d, J=16.0 Hz, 2'-H), 6.74 (1H, d, J=8.0 Hz, 5-H), 6.98 (1H, dd, J=2.0, 8.0 Hz, 6-H), 7.03 (1H, d, J=2.0 Hz, 2-H), *7.45* (1H, d, J=16.0 Hz, 3'-H), 9.11 (1H, s, 3-OH), 9.57 (1H, s, 4-OH)

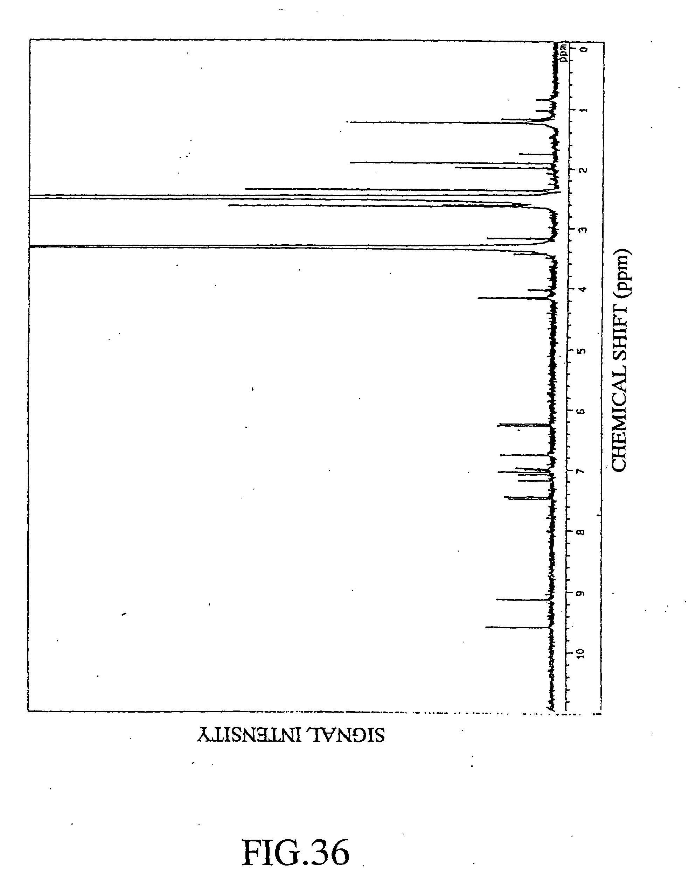

Figure 36 shows ¹H-NMR spectrum of the fraction 28 derived from root portions of *Angelica keiskei* koidz. In Figure 36, the axis of abscissas is chemical shift (ppm), and the axis of ordinates is intensity of signal.
As a result of the analyses of the mass spectrum and NMR spectrum for the fraction 28 derived from root portions of *Angelica keiskei* koidz., it was identified that the active component is caffeic acid ethyl ester (molecular weight: 208).
The enhancing activity for NGF production of each of caffeic acid ethyl ester and caffeic acid methyl ester was assayed in the same manner as in item (1) of Example 4. As shown in Table 49, each of caffeic acid ethyl ester and caffeic acid methyl ester enhanced NGF production of L-M cells.

**Table 49**

| | | |
|---|---|---|
| Caffeic acid ethyl ester (µg/ml) | 0 | 62.5 |
| Amount of NGF Produced (%) | 100 | 1279.1 |
| Caffeic acid methyl ester (µg/ml) | 0 | 62.5 |
| Amount of NGF Produced (%) | 100 | 824.4 |
| (Here, the amount of NGF produced in the control was 0.109 ng/ml.) | | |

### Example 31

(1) Twenty-five grams of onionskin was lyophilized, and cut into thin pieces, and the thin pieces were extracted with 500 ml of methanol at room temperature. A liquid portion resulting from removal of the residue was concentrated to dryness with a rotary evaporator, to give a methanol extract. The enhancing activity for NGF production of the methanol extract prepared as described above was assayed in the same manner as in Example 13. The methanol extract of onionskin was added to the medium so as to have a concentration of 0.096, 0.192, 0.384, 0.576, 0.768 or 0.96 mg/ml. As a result, the methanol extract of onionskin enhanced NGF production of L-M cells in a concentration-dependent manner. The results are shown in Table 50.

**Table 50**

| Concentration of Methanol Extract of Onionskin (mg/ml) | Amount of NGF Produced (%) |
|---|---|
| 0 | 100 |
| 0.096 | 140.4 |
| 0.192 | 136.4 |
| 0.384 | 200.5 |
| 0.576 | 277.3 |
| 0.768 | 307.6 |
| 0.960 | 349.0 |
| (Here, the amount of NGF produced in the control was 0.050 ng/ml.) | |

(2) Three grams of biloba tea leaves (100% Ginkgo, Biloba Tea, manufactured by GINKGOTON) were extracted with 200 ml of distilled water at 100°C. A liquid portion resulting from removal of the residue was taken, to give a water-extract of biloba tea leaves. The enhancing activity for NGF production of the water-extract prepared as described above was assayed in the same manner as in Example 13. The water-extract of biloba tea leaves was added to the medium so as to have a concentration of 0.625, 1.25, 2.5 or 5% (v/v). As a result, the water-extract of biloba tea leaves enhanced NGF production of L-M cells in a concentration-dependent manner. The results are shown in Table 51.

**Table 51**

| Concentration of Water-Extract of Biloba Tea Leaves (mg/ml) | Amount of NGF Produced (%) |
|---|---|
| 0 | 100 |
| 0.625 | 272.9 |
| 1.25 | 305.0 |
| 2.5 | 255.2 |
| 5.0 | 231.9 |
| (Here, the amount of NGF produced in the control was 0.055 ng/ml.) | |

### Example 32

Two grams of rose flowers (available in a local market in China) were cut into thin pieces, and the thin pieces were extracted with 40 ml of chloroform at room temperature. The residue resulting from removal of the chloroform extract by filtration was extracted with 40 ml of ethanol at room temperature. A liquid portion resulting from removal of the residue was concentrated to dryness with a rotary evaporator, to give an ethanol-extracted fraction. Next, the residue was extracted with 40 ml of distilled water at 60°C for 1 hour. A 2.5-fold amount of ethanol was added to a liquid portion resulting from removal of the residue, and the mixture was allowed to stand at -30°C for 2 hours. Thereafter, the mixture was centrifuged at 10000 G to fractionate the mixture into precipitates and a liquid portion. The liquid portion was concentrated to dryness with a rotary evaporator, to give a water-extracted fraction-1. On the other hand, the precipitates were re-dissolved in distilled water, to give a water-extracted fraction-2. The enhancing activity for NGF production of each fraction prepared as described above was assayed in the same manner as in Example 13. The ethanol-extracted fraction of rose flowers was added to the medium so as to have a concentration of 0.115 or 0.23 mg/ml. The water-extracted fraction-1 was added to the medium so as to have a concentration of 0.41 or 0.821 mg/ml. The water-extracted fraction-2 was added to the medium so as to have a concentration of 0.084 or 0.167 mg/ml. The results are shown in Table 52. Each of the ethanol-extracted fraction, the water-extracted fraction-1 and the water-extracted fraction-2 of rose flowers enhanced NGF production of L-M cells in a concentration-dependent manner.

**Table 52**

| Sample | Concentration (mg/ml) | Amount of NGF Produced (%) |
|---|---|---|
| Ethanol-Extracted Fraction of Rose Flowers | 0 | 100 |
| | 0.115 | 623.5 |
| | 0.23 | 615.6 |
| Water-Extracted Fraction-1 of Rose Flowers | 0 | 100 |
| | 0.41 | 307.7 |
| | 0.821 | 365.9 |
| Water-Extracted Fraction-2 of Rose Flowers | 0 | 100 |
| | 0.084 | 195.8 |
| | 0.167 | 219.7 |
| (Here, the amount of NGF produced in the control was 0.136 ng/ml.) | | |

### Example 33

The enhancing activity for NGF production of xanthohumol fraction derived from *Humulus lupulus* was assayed in the same manner as in Example 13. The xanthohumol fraction was added to the medium so as to have a final concentration of 0.0148, 0.0297, 0.0594 or 0.119 mg/ml. The results are shown in Table 53. The xanthohumol fraction enhanced NGF production of L-M cells in a concentration-dependent manner.

**Table 53**

| Concentration of Xanthohumol Fraction (mg/ml) | Amount of NGF Produced (%) |
|---|---|
| 0 | 100 |
| 0.0148 | 144.0 |
| 0.0297 | 242.7 |
| 0.0594 | 401.6 |
| 0.119 | 438.6 |
| (Here, the amount of NGF produced in the control was 0.206 ng/ml.) | |

### Example 34

(1) The amount 0.4 g of the xanthohumol fraction, of which enhancing activity for NGF production was confirmed in Example 33, was dissolved in 3 ml of chloroform, and the solution was subjected to silica chromatography. A stepwise elution was carried out with chloroform (1000 ml),
chloroform : methanol= 50:1 (1000 ml) and
chloroform : methanol= 20:1 (1000 ml), in this order, and 8-ml aliquots per fraction were collected. The resulting fractions 67 to 100 were concentrated under reduced pressure, to give a xanthohumol fraction-Fraction A derived from *Humulus lupulus.*
(2) The xanthohumol fraction-Fraction A mentioned above was further subjected to purification and isolation by using reverse phase chromatography. The conditions therefor are shown below. The column used was TSK gel ODS-80Ts (diameter: 21.5 mm, length: 30 cm, manufactured by Tosoh Corporation). The elution ratio of Solvent A (mixture of distilled water and acetonitrile in a volume ratio of 3:2, containing 0.1% trifluoroacetic acid) and Solvent B (mixture of distilled water and acetonitrile in a volume ratio of 1:4, containing 0.1% trifluoroacetic acid) was such that the ratio of Solvent B was increased linearly from 50 to 100% from 0 to 60 minutes, the ratio of Solvent B was retained at 100% for the subsequent 20 minutes, and the ratio of Solvent B was finally decreased to 50% and retained thereat for 20 minutes. The elution rate was 5 ml/minute, and the detection was carried out at 370 nm. A fraction was collected every minute.
(3) The enhancing activity for NGF production of the reverse phase chromatography fraction from the xanthohumol fraction-Fraction A mentioned above was assayed in the same manner as in Example 13. As a result, it was clarified that the fraction including peaks detected at retention time of 21.1, 22.2, 24.2, 25.7 or 28.6 minutes has the enhancing activity for NGF production. The results are shown in Table 54.

**Table 54**

| Fraction (Retention Time: minutes) | Concentration (mg/ml) | Amount of NGF Produced (%) |
|---|---|---|
| A-1 (21.1) | 0.025 | 92.4 |
| | 0.05 | 151.4 |
| A-2 (22.2) | 0.05 | 109.5 |
| | 0.1 | 136.2 |
| | 0.2 | 208.6 |
| A-3 (24.2) | 0.0125 | 134.3 |
| | 0.025 | 151.4 |
| | 0.05 | 202.9 |
| | 0.1 | 298.1 |
| A-4 (25.7) | 0.025 | 197.1 |
| | 0.05 | 345.7 |
| | 0.1 | 416.2 |
| A-5 (28.6) | 0.0125 | 145.7 |
| | 0.025 | 189.5 |
| | 0.05 | 229.5 |
| | 0.1 | 517.1 |
| (Here, the amount of NGF produced in the control was 0.093 ng/ml.) | | |

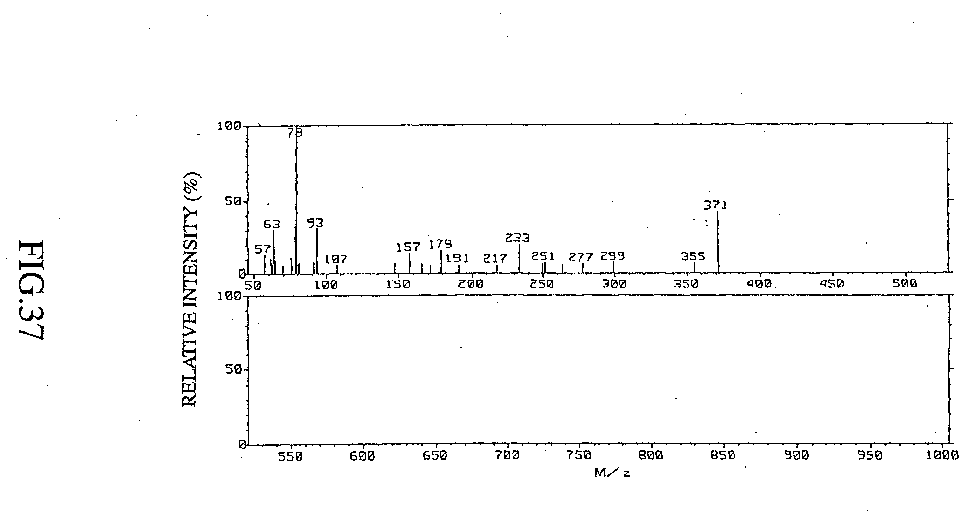

(4) The mass spectrum (MS) of the above-mentioned fraction A-5 (fraction including a peak detected at a retention time of 28.6 minutes) was determined by mass spectrometer (DX302, manufactured by JEOL LTD.) by FAB-MS technique. As the matrix, glycerol was used. As a result, a peak of m/z 371 (M+H)⁺ was detected. Figure 37 shows the MS spectrum of the fraction A-5 derived from the xanthohumol fraction. In Figure 37, the axis of abscissas is m/z value, and the axis of ordinates is relative intensity.
Further, the structure of the fraction was analyzed by measuring various NMR spectra using nuclear magnetic resonance (NMR) spectrophotometer (JNM-A500, manufactured by JEOL, Ltd.). As a result, the active component was identified to be a mixture of xanthohumol B (molecular weight: 370) and xanthohumol D (molecular weight: 370) (mixing ratio: 1:1.65). The signals of NMR are shown below.

### Xanthohumol B

¹H-NMR: δ 1.21 (3H, s, 6"-H), 1.27 (3H, s, 6"-H), 2.70 (2H, m, 4"-H), 3.65 (1H, m, 5"-H), 3.87 (3H, s, 6'-OCH₃), 6.00 (1H, s, 5'-H), 6.80 (2H, m, 3-H and 5-H), 7.55 (2H, m, 2-H and 6-H), 7.70 (1H, m, β-H), 7.75 (1H, m, α-H), 10.12 (1H, s, 4-OH), 14.18 (1H, s, 2'-OH)

### Xanthohumol D

¹H-NMR: δ 1.71 (3H, s, 5"-H), 2.60 (2H, m, 1"-H), 3.85 (3H, s, 6'-OCH₃), 4.20 (1H, m, 2"-H), 4.58 (1H, s, 4"-H), 4.61 (1H, s, 4"-H), 6.04 (1H, s, 5'-H), 6.80 (2H, m, 3-H and 5-H), 7.55 (2H, m, 2-H and 6-H), 7.70 (1H, m, β-H), 7.75 (1H, m, α-H), 10.09 (1H, s, 4-OH), 10.58 (1H, s, 4'-OH), 14.69 (1H, s, 2'-OH)

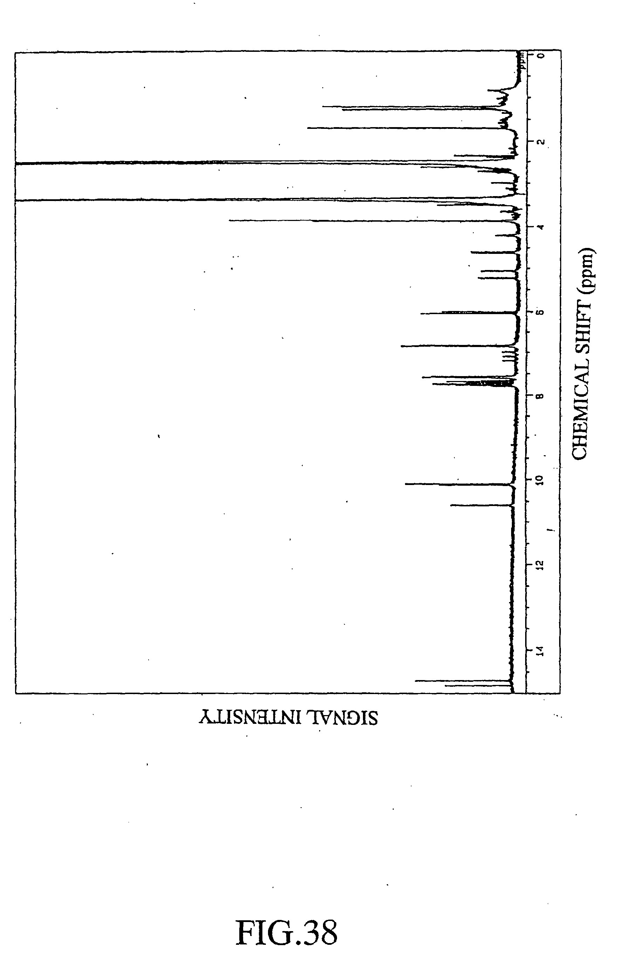

Here, the sample was dissolved in deuterated dimethyl sulfoxide, and the chemical shift of the residual dimethyl sulfoxide was expressed as 2.49 ppm. Figure 38 shows ¹H-NMR of the fraction A-5 derived from the xanthohumol fraction. In Figure 38, the axis of abscissas is chemical shift (ppm), and the axis of ordinates is intensity of signal.
(5) The active component of the xanthohumol fraction derived from *Humulus lupulus* was purified and isolated by reverse phase chromatography as follows.

The conditions therefor are given below.

The column used was TSK gel ODS 80TsQA (diameter: 4.6 mm, length: 25 cm, manufactured by Tosoh Corporation). The elution ratio of Solvent A (mixture of distilled water and acetonitrile in a volume ratio of 3:1, containing 0.1% trifluoroacetic acid) and Solvent B (mixture of distilled water and acetonitrile in a volume ratio of 1:3, containing 0.1% trifluoroacetic acid) was such that the ratio of Solvent B was increased linearly from 50 to 100% from 0 to 20 minutes, the ratio of Solvent B was retained at 100% for the subsequent 5 minutes, and the ratio of Solvent B was finally decreased to 50% and retained thereat for 5 minutes. The elution rate was 1 ml/minute, and the detection was carried out at 215 nm. A fraction was collected every minute, and the activity was assayed for each fraction in the same manner as in Example 13.

As a result, it was clarified that the fraction including a peak detected at retention time of 12.8 minutes has the activity. This fraction was analyzed by mass spectrum. As a result, a signal corresponding to a molecular weight of 355 was detected. Further, the fraction was analyzed by ¹H-NMR spectrum. As a result, it was clarified that the active component is xanthohumol (molecular weight 354.4). The determination results for the NMR spectrum and the mass spectrum are as follows.
¹H-NMR: δ 1.60 (3H, s, -Me), 1.69 (3H, s, -Me), 3.12 (2H, d, J 7 Hz, H-1"_{a, b}), 3.86 (3H, s, -OMe), 5.13 (1H, t, J 7 Hz, H-2"), 6.07 (1H, s, H-5'),
6.83 (2H, d, J_{2,3} 9 Hz, J_{5,6} 9 Hz, H-2, 6), 7.56 (2 H, d, H-3, 5),
7.66 (1H, d, J_{a,b} 16 Hz), 7.75 (1H, d), 10.05 (1H, s, OH-4), 10.55 (1H, s, OH-4'), 14.63 (1H, s, OH-2')

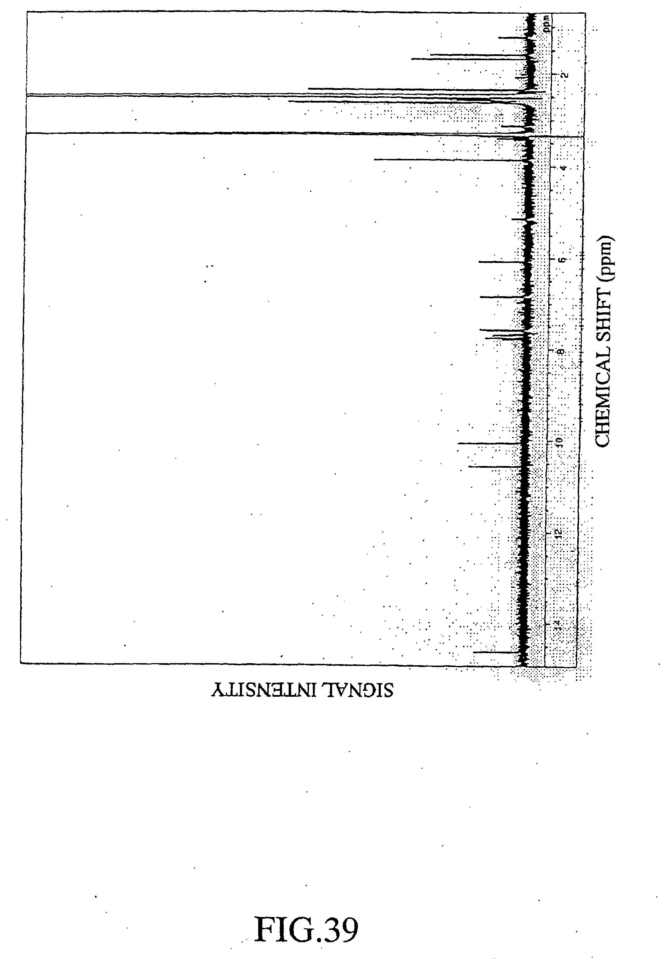

Here, the sample was dissolved in deuterated dimethyl sulfoxide, and the chemical shift of the residual dimethyl sulfoxide was expressed as 2.49 ppm. Figure 39 shows ¹H-NMR spectrum. In Figure 39, the axis of abscissas is chemical shift (ppm), and the axis of ordinates is intensity of signal.
FAB-MS: m/z 355 (M+H)⁺ (Here, glycerol was used as the matrix.)

### Example 35

(1) The enhancing activity for NGF production of the purified xanthohumol obtained in Example 34 was assayed in the same manner as in Example 13. The purified xanthohumol was added to the medium so as to have a concentration of 12.5, 25, or 50 µM. The results are shown in Table 55. The purified xanthohumol enhanced NGF production of L-M cells.

**Table 55**

| Concentration of Xanthohumol (µM) | Amount of NGF Produced (%) |
|---|---|
| 0 | 100 |
| 12.5 | 235.5 |
| 25 | 387.9 |
| 50 | 443.0 |
| (Here, the amount of NGF produced in the control was 0.095 ng/ml.) | |

(2) The amount 0.4 g of the xanthohumol fraction derived from *Humulus lupulus* was dissolved in 3 ml of chloroform, and the solution was subjected to silica chromatography. A stepwise elution was carried out with chloroform (1000 ml), chloroform : methanol = 50:1 (1000 ml) and chloroform : methanol = 20:1 (1000 ml), in this order, and 8-ml aliquots per fraction were collected. The resulting fractions 67 to 100 were concentrated under reduced pressure, to give a xanthohumol fraction derived from *Humulus lupulus*-Fraction A. The xanthohumol fraction derived from *Humulus lupulus*-Fraction A was further purified by using reverse phase chromatography. The conditions therefor are given below. The column used was TSK gel ODS 80Ts (diameter: 21.5 mm, length: 30 cm, manufactured by Tosoh Corporation). The elution ratio of Solvent A (mixture of distilled water and acetonitrile in a volume ratio of 3:2, containing 0.1% trifluoroacetic acid) and Solvent B (mixture of distilled water and acetonitrile in a volume ratio of 1:4, containing 0.1% trifluoroacetic acid) was such that the ratio of Solvent B was increased linearly from 50 to 100% from 0 to 60 minutes, the ratio of Solvent B was retained at 100% for the subsequent 20 minutes, and the ratio of Solvent B was finally decreased to 50% and retained thereat for 20 minutes. The elution rate was 5 ml/minute, and the detection was carried out at 370 nm. A fraction was collected every minute. A fraction including a peak detected at retention time of 42.5 minutes was concentrated to dryness, whereby high-purity xanthohumol (19.4 mg) could be prepared.
(3) The amount 13.5 mg of the prepared xanthohumol was dissolved in 0.75 ml of a dimethyl sulfoxide solution. The solution was added to 200 ml of 10 mM sodium acetate buffer (pH 5.5, containing 10% saccharose), and the mixture was heated at 100°C for 2 hours. After cooling the mixture, the reaction mixture was fractionated using reverse phase chromatography. The conditions therefor are given below. The resin used was Cosmosil 140 C₁₈-OPN (manufactured by nakalaitesque, amount of resin 20 ml). The reaction mixture was applied to the column, and the elution was carried out using as developing solvents 50 ml each of distilled water, 20%-, 30%-, 40%-, 50%-, 60%- and 70%-aqueous acetonitrile solution, and methanol, in this order. As a result, the fraction eluted with the 40%-aqueous acetonitrile solution was concentrated to dryness, whereby isoxanthohumol (2.1 mg) could be obtained. The structure thereof was confirmed by analyses of various NMR spectra determined.
(4) The enhancing activity for NGF production of the isoxanthohumol prepared was assayed in the same manner as in Example 13. Isoxanthohumol was added to the medium so as to have a concentration of 50, 100 or 200 µM. As shown in Table 56, isoxanthohumol enhanced NGF production of L-M cells in a concentration-dependent manner, so that the physiological activity of isoxanthohumol in beer beverages was clarified.

**Table 56**

| Isoxanthohumol | | | | |
|---|---|---|---|---|
| Sample Concentration (µM) | 0 | 50 | 100 | 200 |
| Amount of NGF Produced (%) | 100 | 110.7 | 121.4 | 198.0 |
| (Here, the amount of NGF produced in the control was 0.181 ng/ml.) | | | | |

### Example 36

(1) Each of 3 brand names of beer (marketed product A, marketed product B and marketed product C), and a nonalcoholic beer beverage (marketed product D) in an amount of 150 ml each was concentrated to a volume of 60 ml with a rotary evaporator, to give each concentrate.
(2) The enhancing activity for NGF production of the above-mentioned concentrate was assayed in the same manner as in Example 13. The concentrate of each of 3 brand names of beer was added to the medium so as to have a concentration of 2.5, 5 or 10% (volume ratio). The concentrate of the nonalcoholic beverage was added to the medium so as to have a concentration of 10% (volume ratio). The results are shown in Table 57. Each of the concentrate of each of 3 brand names of beer and the concentrate of the nonalcoholic beverage enhanced NGF production of L-M cells in a concentration-dependent manner.

**Table 57**

| Concentrate of Marketed Product A | | | | |
|---|---|---|---|---|
| Sample Concentration (%) | 0 | 2.5 | 5 | 10 |
| Amount of NGF Produced (%) | 100 | 115.5 | 109.3 | 209.9 |

| Concentrate of Marketed Product B | | | | |
|---|---|---|---|---|
| Sample Concentration (%) | 0 | 2.5 | 5 | 10 |
| Amount of NGF Produced (%) | 100 | 86.3 | 126.2 | 272.4 |

| Concentrate of Marketed Product C | | | | |
|---|---|---|---|---|
| Sample Concentration (%) | 0 | 2.5 | 5 | 10 |
| Amount of NGF Produced (%) | 100 | 111.1 | 153.2 | 365.1 |

| Concentrate of Marketed Product D | | | | |
|---|---|---|---|---|
| Sample Concentration (%) | 0 | | | 10 |
| Amount of NGF Produced (%) | 100 | | | 127.0 |
| (Here, the amount of NGF produced in the control was 0.183 ng/ml.) | | | | |

In addition, the enhancing activity for HGF production of each of the concentrate of the marketed product B and the concentrate of the marketed product D was assayed in the same manner as in item (1) of Example 4. The concentrate of the marketed product B was added to the medium so as to have a concentration of 1% (volume ratio). The concentrate of the marketed product D was added to the medium so as to have a concentration of 1 and 5% (volume ratio). The results are shown in Table 58. Each of the concentrates enhanced HGF production of MRC-5 cells.

**Table 58**

| Concentrate of Marketed Product B | | | |
|---|---|---|---|
| Sample Concentration (%) | 0 | 1 | |
| Amount of HGF Produced (%) | 100 | 218 | |

| Concentrate of Marketed Product D | | | |
|---|---|---|---|
| Sample Concentration (%) | 0 | 1 | 5 |
| Amount of HGF Produced (%) | 100 | 160 | 158 |
| (Here, the amount of HGF produced in the control was 4.04 ng/ml.) | | | |

(3) The contents of xanthohumol and isoxanthohumol in each of the marketed products A to D, and beer marketed products E to I, and low-malt beer marketed products J to O were determined by mass spectrometer (see *Journal of Chromatography A*, **832**, 97-107 (1997)). The results are shown in Table 59. Each marketed product contained xanthohumol within the range of 0.0005 to 0.32 µg/ml. In addition, each marketed product contained isoxanthohumol within the range of 0.0082 to 1.27 µg/ml. The larger the content of these compounds, especially the content of xanthohumol, the better the beer flavor.

**Table 59**

| Marketed Product | Xanthohumol (µg/ml) | Isoxanthohumol (µg/ml) |
|---|---|---|
| A | 0.021 | 0.70 |
| B | 0.0073 | 0.58 |
| C | 0.0070 | 0.57 |
| D | 0.00060 | 0.034 |
| E | 0.0060 | 0.57 |
| F | 0.0073 | 0.53 |
| G | 0.017 | 0.48 |
| H | 0.021 | 1.27 |
| I | 0.00054 | 0.0082 |
| J | 0.0036 | 0.59 |
| K | 0.0041 | 0.16 |
| L | 0.0087 | 0.77 |
| M | 0.010 | 0.23 |
| N | 0.030 | 0.62 |
| O | 0.032 | 1.10 |

### Example 37

(1) Fifty grams of a dry product of *Humulus lupulus* was powdered, and the powder was extracted with 1000 ml of distilled water at 60°C for 1 hour. A water-extracted liquid of *Humulus lupulus* resulting from removal of the residue by filtration was concentrated to a volume of 150 ml with a rotary evaporator. The amount 225 ml of chloroform was added to the concentrate, and the mixture was shaken, and then fractionated into an aqueous layer, a chloroform layer and an intermediate layer. The solvent was distilled off from the chloroform layer fraction with a rotary evaporator, and thereafter the residue was concentrated to give a fraction migrated to chloroform from water-extracted product of *Humulus lupulus.*
(2) The enhancing activity for NGF production of the above-mentioned fraction migrated to chloroform from water-extracted product of *Humulus lupulus* was assayed in the same manner as in Example 13. The fraction migrated to chloroform from water-extracted product of *Humulus lupulus* was added to the medium so as to have a final concentration of 0.02 or 0.04 mg/ml. The results are shown in Table 60. The fraction migrated to chloroform from water-extracted product of *Humulus lupulus* enhanced NGF production of L-M cells in a concentration-dependent manner.

**Table 60**

| Fraction Migrated to Chloroform from Water-Extracted Product of *Humulus lupulus* (mg/ml) | Amount of NGF Produced (%) |
|---|---|
| 0 | 100 |
| 0.02 | 159.6 |
| 0.04 | 253.4 |
| (Here, the amount of NGF produced in the control was 0.120 ng/ml.) | |

(3) Fifty grams of a dry product of *Humulus lupulus* was powdered, and the powder was extracted with 1000 ml of ethanol at 4°C for 18 hours. An ethanol extract of *Humulus lupulus* resulting from removal of the residue by filtration was concentrated with a rotary evaporator. A mixed solution of chloroform : water = 3:2 was added to the concentrate, and the mixture was shaken, and then fractionated into an aqueous layer and a chloroform layer. The solvent was distilled off from the chloroform layer fraction with a rotary evaporator, and thereafter the residue was concentrated to give a fraction migrated to chloroform from ethanol-extracted product of *Humulus lupulus.*
(4) The enhancing activity for NGF production of the fraction migrated to chloroform from ethanol-extracted product of *Humulus lupulus* obtained in item (3) of Example 37 was assayed in the same manner as in Example 13. The fraction migrated to chloroform from ethanol-extracted product *of Humulus lupulus* was added to the medium so as to have a final concentration of 0.05 or 0.1 mg/ml. The results are shown in Table 61. The fraction migrated to chloroform from ethanol-extracted product of *Humulus lupulus* enhanced NGF production of L-M cells.

**Table 61**

| Fraction Migrated to Chloroform from Ethanol-Extracted Product of *Humulus lupulus* (mg/ml) | Amount of NGF Produced (%) |
|---|---|
| 0 | 100 |
| 0.05 | 245.2 |
| 0.1 | 240.7 |
| (Here, the amount of NGF produced 0.120 ng/ml.) in the control was | |

### Example 38

(1) Five grams of *Curcuma zedoaeia Roscoe* was lyophilized, and thereafter cut into thin pieces, and the thinly cut pieces were extracted with 200 ml of chloroform at room temperature. This extraction procedure was repeated twice against the residue after the suction filtration. A liquid portion after the filtration was collected and concentrated to dryness with a rotary evaporator, to give a chloroform-extracted fraction. Next, the residue after the extraction with chloroform was extracted with 200 ml of ethanol at room temperature. This procedure was repeated twice against the residue after the suction filtration. A liquid portion after the filtration was collected and concentrated to dryness with a rotary evaporator, and the dried product was dissolved in 4 ml of ethanol. A portion dissolved in ethanol was collected and concentrated to dryness with a rotary evaporator, to give an ethanol-extracted fraction-1. In addition, the part not dissolved in ethanol but dissolved in distilled water was collected and concentrated to dryness with a rotary evaporator, to give an ethanol-extracted fraction-2. Next, the residue after the extraction with ethanol was extracted with 25 ml of distilled water at 60°C for 2 hours. The 2.5-fold amount of ethanol was added to a liquid portion resulting from removal of the residue, and the mixture was allowed to stand at -20°C for 1 hour. Thereafter, the mixture was centrifuged at 10000 G to fractionate the mixture into precipitates and a liquid portion. The liquid portion was concentrated to dryness with a rotary evaporator, to give a water-extracted fraction-1. On the other hand, the precipitates were re-dissolved in distilled water, to give a water-extracted fraction-2.
(2) The enhancing activity for NGF production of each of the chloroform-extracted fraction, the ethanol-extracted fraction-1, the water-extracted fraction-1 and the water-extracted fraction-2 of *Curcuma zedoaeia* *Roscoe* prepared in item (1) of Example 38 was assayed in the same manner as in Example 13. The chloroform-extracted fraction of *Curcuma zedoaeia Roscoe* was added to the medium so as to have a concentration of 0.108 or 0.215 mg/ml. The ethanol-extracted fraction-1 of *Curcuma zedoaeia Roscoe* was added to the medium so as to have a concentration of 0.02 or 0.04 mg/ml. The water-extracted fraction-1 of *Curcuma zedoaeia Roscoe* was added to the medium so as to have a concentration of 0.825, 1.65 or 3.3 mg/ml. The water-extracted fraction-2 of *Curcuma zedoaeia Roscoe* was added to the medium so as to have a concentration of 0.55 mg/ml. The results are shown in Table 62. Each of the chloroform-extracted fraction, the ethanol-extracted fraction-1, the water-extracted fraction-1 and the water-extracted fraction-2 of *Curcuma zedoaeia Roscoe* enhanced NGF production of L-M cells in a concentration-dependent manner.

**Table 62**

| Sample | Concentration (mg/ml) | Amount of NGF Produced (%) |
|---|---|---|
| Chloroform-Extracted Fraction of *Curcuma zedoaeia Roscoe* | 0 | 100 |
| | 0.108 | 172.4 |
| | 0.215 | 296.8 |
| Ethanol-Extracted Fraction-1 of *Curcuma zedoaeia Roscoe* | 0 | 100 |
| | 0.02 | 149.8 |
| | 0.04 | 214.3 |
| Water-Extracted Fraction-1 of *Curcuma zedoaeia Roscoe* | 0 | 100 |
| | 0.825 | 458.6 |
| | 1.65 | 500.5 |
| | 3.3 | 559.3 |
| Water-Extracted Fraction-2 of *Curcuma zedoaeia Roscoe* | 0 | 100 |
| | 0.55 | 212.0 |
| (Here, the amount of NGF produced in the control was 0.122 ng/ml.) | | |

(3) One-hundred and fifty grams of *Curcuma zedoaeia Roscoe* was lyophilized, and thereafter cut into thin pieces, and the thinly cut pieces were extracted with 300 ml of chloroform at room temperature. This extraction procedure was repeated twice against the residue after the suction filtration. The residue after the extraction with chloroform was extracted with 300 ml of ethanol at room temperature. This procedure was repeated twice against the residue after the suction filtration. The residue after the extraction with ethanol was extracted with 900 ml of distilled water at 60°C for 2 hours. This procedure was repeated against the residue after the suction filtration. A liquid portion resulting from removal of the residue by the suction filtration was concentrated to a volume of 500 ml, and the 2.5-fold amount of ethanol was added thereto, and the mixture was allowed to stand at -20°C for 1 hour. Thereafter, a liquid portion resulting from removal of the precipitated portion by centrifugation at 10000 G was concentrated to dryness with a rotary evaporator, to give a water-extracted fraction-3 of *Curcuma zedoaeia Roscoe.*
Next, the active component of the water-extracted fraction-3 of *Curcuma zedoaeia Roscoe* was fractionated using a synthetic adsorbent.
The conditions therefor are given below.
The resin used was Amberlite XAD-2 (manufactured by Organo, amount of resin: 70 ml). The amount 3.4 g of the water-extracted fraction-3 of *Curcuma zedoaeia Roscoe* was applied to XAD-2, and non-adsorbed substances were sufficiently washed off with 140 ml of distilled water, and then the adsorbed substances were eluted with 350 ml of methanol. The methanol eluate was concentrated with a rotary evaporator, to give an XAD-2-treated, water-extracted low-molecular fraction-3 of *Curcuma zedoaeia Roscoe.*
(4) The enhancing activity for NGF production of the above-mentioned XAD-2-treated, water-extracted fraction-3 of *Curcuma zedoaeia Roscoe* was assayed in the same manner as in Example 13. The XAD-2-treated, water-extracted fraction-3 of *Curcuma zedoaeia Roscoe* was added to the medium so as to have a concentration of 0.5, 1 or 2 mg/ml. The results are shown in Table 63. The XAD-2-treated, water-extracted fraction-3 of *Curcuma zedoaeia Roscoe* enhanced NGF production of L-M cells in a concentration-dependent manner.

**Table 63**

| | | | | |
|---|---|---|---|---|
| Sample Concentration (mg/ml) | 0 | 0.5 | 1.0 | 2.0 |
| Amount of NGF Produced (%) | 100 | 340.2 | 526.6 | 607.2 |
| (Here, the amount for the control was 0.328 ng/ml.) | | | | |

In addition, the enhancing activity for HGF production of the treated fraction was assayed in the same manner as in item (1) of Example 4. The XAD-2-treated, water-extracted fraction-3 of *Curcuma zedoaeia Roscoe* was added to the medium so as to have a concentration of 2000, 1000, 500 or 250 µg/ml. The results are shown in Table 64. The XAD-2-treated, water-extracted fraction-3 of *Curcuma zedoaeia Roscoe* enhanced HGF production of MRC-5 cells in a concentration-dependent manner.

**Table 64**

| | | | | | |
|---|---|---|---|---|---|
| Sample Concentration (µg/ml) | 0 | 250 | 500 | 1000 | 2000 |
| Amount of HGF Produced (%) | 100 | 112 | 128 | 149 | 192 |
| (Here, the amount of HGF produced in the control was 8.02 ng/ml.) | | | | | |

(5) The active component of the XAD-2-treated, water-extracted fraction-3 of *Curcuma zedoaeia Roscoe* was fractionated using reverse phase chromatography.
The conditions therefor are given below.
The resin used was Cosmosil 140 C₁₈-OPN (manufactured by nakalaitesque, amount of resin: 70 ml). The amount 0.444 g of the XAD-2-treated, water-extracted fraction-3 of *Curcuma zedoaeia Roscoe* was applied thereto, and the elution was carried out using as the developing solvents 140 ml each of distilled water, a 5% aqueous acetonitrile solution, a 10% aqueous acetonitrile solution, a 20% aqueous acetonitrile solution, a 40% aqueous acetonitrile solution, and acetone, in this order, and each eluted fraction was concentrated under reduced pressure.
(6) The enhancing activity for NGF production of the reverse phase chromatography fraction derived from the XAD-2-treated, water-extracted fraction-3 of *Curcuma zedoaeia Roscoe* was assayed in the same manner as in Example 13. The results are shown in Table 65. As shown in the table, it was clarified that each of the distilled water-eluted fraction, the fraction eluted with a 10% aqueous acetonitrile solution, the fraction eluted with a 20% aqueous acetonitrile solution, the fraction eluted with a 40% aqueous acetonitrile solution, the acetonitrile-eluted fraction and the acetone-eluted fraction has enhancing activity for NGF production.

**Table 65**

| | Concentration (mg/ml) | Amount of NGF Produced (%) |
|---|---|---|
| Distilled Water-Eluted Fraction | 0.25 | 103.2 |
| | 0.5 | 133.0 |
| | 1.0 | 147.1 |
| Fraction Eluted with 10% Aqueous Acetonitrile Solution | 0.25 | 117.7 |
| | 0.5 | 130.6 |
| | 1.0 | 157.1 |
| Fraction Eluted with 20% Aqueous Acetonitrile Solution | 0.25 | 154.6 |
| | 0.5 | 192.7 |
| | 1.0 | 229.9 |
| Fraction Eluted with 40% Aqueous Acetonitrile Solution | 0.25 | 185.1 |
| | 0.5 | 255.4 |
| | 1.0 | 373.8 |
| Acetonitrile-Eluted Fraction | 0.025 | 135.6 |
| | 0.05 | 163.6 |
| | 0.1 | 170.0 |
| Acetone-Eluted Fraction | 0.025 | 110.4 |
| | 0.05 | 121.0 |
| | 0.1 | 140.1 |
| (Here, the amount of NGF produced in the control was 0.307 ng/ml.) | | |

In addition, the enhancing activity for HGF production of the reverse phase chromatography fraction derived from the XAD-2-treated, water-extracted fraction-3 of *Curcuma zedoaeia Roscoe* was assayed in the same manner as in item (1) of Example 4. As a result, it was clarified that each of the distilled water-eluted fraction, the fraction eluted with a 5% aqueous acetonitrile solution, the fraction eluted with a 10% aqueous acetonitrile solution and the fraction eluted with a 20% aqueous acetonitrile solution has enhancing activity for HGF production. The results are shown in Table 66.

**Table 66**

| Fraction | Concentration (µg/ml) | Amount of HGF Produced (%) |
|---|---|---|
| Distilled Water-Eluted Fraction | 20 | 127 |
| | 2 | 106 |
| Fraction Eluted with 5% Aqueous Acetonitrile Solution | 200 | 119 |
| | 20 | 121 |
| Fraction Eluted with 10% Aqueous Acetonitrile Solution | 2000 | 264 |
| | 200 | 126 |
| Fraction Eluted with 20% Aqueous Acetonitrile Solution | 2000 | 169 |
| | 200 | 111 |
| (Here, the amount of HGF produced in the control for the distilled water-eluted fraction and the fraction eluted with a 5% aqueous acetonitrile solution was 8.10 ng/ml, the amount of HGF produced in the control for the fraction eluted with a 10% aqueous acetonitrile solution was 9.00 ng/ml and the amount of HGF produced in the control for the fraction eluted with a 20% aqueous acetonitrile solution was 9.56 ng/ml.) | | |

### Example 39

(1) Ten grams of a product obtained by cutting soybean embryo into thin pieces was extracted with 200 ml of chloroform at room temperature. This extraction procedure was repeated twice against the residue after the suction filtration. The residue after the extraction with chloroform was extracted with 200 ml of ethanol at room temperature. This procedure was repeated twice against the residue after the suction filtration. A liquid portion after the filtration was collected and concentrated to dryness with a rotary evaporator, and the dried product was dissolved in 5 ml of ethanol. A part dissolved in ethanol was collected and concentrated to dryness with a rotary evaporator, to give an ethanol-extracted fraction-1. In addition, the part not dissolved in ethanol but dissolved in distilled water was collected and concentrated to dryness with a rotary evaporator, to give an ethanol-extracted fraction-2. Next, the residue after the extraction with ethanol was extracted with 200 ml of distilled water at 60°C for 2 hours. The 2.5-fold amount of ethanol was added to a liquid portion resulting from removal of the residue by centrifugation at 10000 G, and the mixture was allowed to stand at -20°C for 1 hour. Thereafter, the mixture was centrifuged at 10000 G to fractionate the mixture into precipitates and a liquid portion. The liquid portion was concentrated to dryness with a rotary evaporator, to give a water-extracted fraction-1. On the other hand, the precipitates were dissolved in distilled water, to give a water-extracted fraction-2.
(2) The enhancing activity for NGF production of each of the ethanol-extracted fraction-2, the water-extracted fraction-1 and the water-extracted fraction-2 of soybean embryo mentioned above was assayed in the same manner as in Example 13. The ethanol-extracted fraction-2 of soybean embryo was added to the medium so as to have a concentration of 0.8875, 1.775 or 3.55 mg/ml. The water-extracted fraction-1 of soybean embryo was added to the medium so as to have a concentration of 4.998, 9.975 or 19.95 mg/ml. The water-extracted fraction-2 of soybean embryo was added to the medium so as to have a concentration of 3.36, 6.72 or 13.44 mg/ml. The results are shown in Tables 67 and 68. Each of the ethanol-extracted fraction-2, the water-extracted fraction-1 and the water-extracted fraction-2 of soybean embryo enhanced NGF production of L-M cells in a concentration-dependent manner.

**Table 67**

| Ethanol-Extracted Fraction-2 | | | | |
|---|---|---|---|---|
| Sample Concentration (mg/ml) | 0 | 0.8875 | 1.775 | 3.55 |
| Amount of NGF Produced (%) | 100 | 543.9 | 523.3 | 589.2 |

| Water-Extracted Fraction-1 | | | | |
|---|---|---|---|---|
| Sample Concentration (mg/ml) | 0 | 4.988 | 9.975 | 19.95 |
| Amount of NGF Produced (%) | 100 | 287.4 | 465.6 | 1215.3 |
| (Here, the amount of NGF produced in the control was 0.251 ng/ml.) | | | | |

**Table 68**

| Water-Extracted Fraction-2 | | | | |
|---|---|---|---|---|
| Sample Concentration (mg/ml) | 0 | 3.36 | 6.72 | 13.44 |
| Amount of NGF Produced (%) | 100 | 250.1 | 259.1 | 299.8 |
| (Here, the amount of NGF produced in the control was 0.223 ng/ml.) | | | | |

In addition, the enhancing activity for HGF production of each of the water-extracted fraction-1 and the water-extracted fraction-2 of soybean embryo was assayed in the same manner as in item (1) of Example 4. The water-extracted fraction-1 of soybean embryo was added to the medium so as to have a concentration of 1995 or 199.5 µg/ml. The water-extracted fraction-2 of soybean embryo was added to the medium so as to have a concentration of 1344 or 134.4 µg/ml. The results are shown in Table 69. Each of the water-extracted fraction-1 and the water-extracted fraction-2 of soybean embryo enhanced HGF production of MRC-5 cells in a concentration-dependent manner.

**Table 69**

| Water-Extracted Fraction-1 of Soybean Embryo | | | |
|---|---|---|---|
| Sample Concentration (µg/ml) | 0 | 199.5 | 1995 |
| Amount of HGF Produced (%) | 100 | 125 | 138 |

| Water-Extracted Fraction-2 of Soybean Embryo | | | |
|---|---|---|---|
| Sample Concentration (µg/ml) | 0 | 134.4 | 1344 |
| Amount of HGF Produced (%) | 100 | 107 | 116 |
| (Here, the amount of HGF produced in the control was 14.92 ng/ml.) | | | |

(3) Ten grams of seed coat of soybean was cut into thin pieces, and the thinly cut pieces were extracted with 200 ml of chloroform at room temperature. This extraction procedure was repeated twice against the residue after the suction filtration. The residue after the extraction with chloroform was extracted with 200 ml of ethanol at room temperature. This procedure was repeated twice against the residue after the suction filtration. A liquid portion after the filtration was collected and concentrated to dryness with a rotary evaporator, and the dried product was dissolved in 3 ml of ethanol. A part dissolved in ethanol was collected and concentrated to dryness with a rotary evaporator, to give an ethanol-extracted fraction-1 of seed coat. In addition, the part not dissolved in ethanol but dissolved in distilled water was collected and concentrated to dryness with a rotary evaporator, to give an ethanol-extracted fraction-2 of seed coat. Next, the residue after the extraction with ethanol was extracted with 70 ml of distilled water at 60°C for 2 hours. The 2.5-fold amount of ethanol was added to a liquid portion resulting from removal of the residue by suction filtration, and the mixture was allowed to stand at -20°C for 1 hour. Thereafter, the mixture was centrifuged at 10000 G to fractionate the mixture into precipitates and a liquid portion. The liquid portion was concentrated to dryness with a rotary evaporator, to give a water-extracted fraction-1 of seed coat. On the other hand, the precipitates were dissolved in distilled water, to give a water-extracted fraction-2 of seed coat.
(4) The enhancing activity for NGF production of each of the ethanol-extracted fraction-2 of seed coat, the water-extracted fraction-1 of seed coat and the water-extracted fraction-2 of seed coat of soybean mentioned above was assayed in the same manner as in Example 13. The ethanol-extracted fraction-2 of seed coat of soybean was added to the medium so as to have a concentration of 0.275 or 0.55 mg/ml. The water-extracted fraction-1 of seed coat of soybean was added to the medium so as to have a concentration of 3.125, 6.25 or 12.5 mg/ml. The water-extracted fraction-2 of seed coat of soybean was added to the medium so as to have a concentration of 0.654, 1.308 or 2.616 mg/ml. The results are shown in Tables 70 and 71. Each of the ethanol-extracted fraction-2 of seed coat, the water-extracted fraction-1 of seed coat and the water-extracted fraction-2 of seed coat of soybean enhanced NGF production of L-M cells in a concentration-dependent manner.

**Table 70**

| Ethanol-Extracted Fraction-2 of Seed Coat | | | | |
|---|---|---|---|---|
| Sample Concentration (mg/ml) | | 0 | 0.275 | 0.55 |
| Amount of NGF Produced (%) | | 100 | 379.1 | 462.5 |
| (Here, the amount of NGF produced in the control was 0.251 ng/ml.) | | | | |

**Table 71**

| Water-Extracted Fraction-1 of Seed Coat | | | | |
|---|---|---|---|---|
| Sample Concentration (mg/ml) | 0 | 3.125 | *6.25* | 12.5 |
| Amount of NGF Produced (%) | 100 | 131.4 | 144.1 | 307.9 |

| Water-Extracted Fraction-2 of Seed Coat | | | | |
|---|---|---|---|---|
| Sample Concentration (mg/ml) | 0 | 0.654 | 1.308 | 2.616 |
| Amount of NGF Produced (%) | 100 | 310.2 | 436.8 | 512.3 |
| (Here, the amount of NGF produced in the control was 0.223 ng/ml.) | | | | |

In addition, the enhancing activity for HGF production of each of the water-extracted fraction-1 and the water-extracted fraction-2 of seed coat of soybean was assayed in the same manner as in item (1) of Example 4. The water-extracted fraction-1 of seed coat of soybean was added to the medium so as to have a concentration of 125 or 1250 µg/ml. The water-extracted fraction-2 of seed coat of soybean was added to the medium so as to have a concentration of 26.16 or 261.6 µg/ml. The results are shown in Tables 72 and 73. Each of the water-extracted fraction-1 and the water-extracted fraction-2 of seed coat of soybean enhanced HGF production of MRC-5 cells in a concentration-dependent manner.

**Table 72**

| Water-Extracted Fraction-1 of Seed Coat of Soybean | | | |
|---|---|---|---|
| Sample Concentration (µg/ml) | 0 | 125 | 1250 |
| Amount of HGF Produced (%) | 100 | 134 | 218 |
| (Here, the amount of HGF produced in the control was 14.92 ng/ml.) | | | |

**Table 73**

| Water-Extracted Fraction-2 of Seed Coat of Soybean | | | |
|---|---|---|---|
| Sample Concentration (µg/ml) | 0 | 26.16 | 261.6 |
| Amount of HGF Produced (%) | 100 | 115 | 218 |
| (Here, the amount of HGF produced in the control was 8.24 ng/mL.) | | | |

(5) Ten grams of a product obtained by cutting soyameal into thin pieces was extracted with 200 ml of chloroform at room temperature. This extraction procedure was repeated twice against the residue after the suction filtration. The residue after the extraction with chloroform was extracted with 200 ml of ethanol at room temperature. This procedure was repeated twice against the residue after the suction filtration. A liquid portion after the filtration was collected and concentrated to dryness with a rotary evaporator, and the dried product was dissolved in 3 ml of ethanol. A part dissolved in ethanol was collected and concentrated to dryness with a rotary evaporator, to give an ethanol-extracted fraction-1 of soyameal. In addition, the part not dissolved in ethanol but dissolved in distilled water was collected and concentrated to dryness with a rotary evaporator, to give an ethanol-extracted fraction-2 of soyameal. Next, the residue after the extraction with ethanol was extracted with 100 ml of distilled water at 60°C for 2 hours. The 2.5-fold amount of ethanol was added to a liquid portion resulting from removal of the residue by centrifugation at 10000 G, and the mixture was allowed to stand at -20°C for 1 hour. Thereafter, the mixture was centrifuged at 10000 G to fractionate the mixture into precipitates and a liquid portion. The liquid portion was concentrated to dryness with a rotary evaporator, to give a water-extracted fraction-1 of soyameal. On the other hand, the precipitates were dissolved in distilled water, to give a water-extracted fraction-2 of soyameal.
(6) The enhancing activity for NGF production of each of the ethanol-extracted fraction-2 of soyameal and the water-extracted fraction-1 of soyameal mentioned above was assayed in the same manner as in Example 13. The ethanol-extracted fraction-2 of soyameal was added to the medium so as to have a concentration of 0.488 or 0.975 mg/ml. The water-extracted fraction-1 of soyameal was added to the medium so as to have a concentration of 2.625 or 5.25 mg/ml. The results are shown in Tables 74 and 75. Each of the ethanol-extracted fraction-2 of soyameal and the water-extracted fraction-1 of soyameal enhanced NGF production of L-M cells in a concentration-dependent manner.

**Table 74**

| Ethanol-Extracted Fraction-2 of Soyameal | | | |
|---|---|---|---|
| Sample Concentration (mg/ml) | 0 | 0.488 | 0.975 |
| Amount of NGF Produced (%) | 100 | 426.5 | 400.7 |
| (Here, the amount of NGF produced in the control was 0.251 ng/ml.) | | | |

**Table 75**

| Water-Extracted Fraction-1 of Soyameal | | | |
|---|---|---|---|
| Sample Concentration (mg/ml) | 0 | 2.625 | 5.25 |
| Amount of NGF Produced (%) | 100 | 284.7 | 283.5 |
| (Here, the amount of NGF produced in the control was 0.223 ng/ml.) | | | |

In addition, the enhancing activity for HGF production of each of the water-extracted fraction-1 and the water-extracted fraction-2 of soyameal was assayed in the same manner as in item (1) of Example 4. The water-extracted fraction-1 of soyameal was added to the medium so as to have a concentration of 105 or 1050 µg/ml. The water-extracted fraction-2 of soyameal was added to the medium so as to have a concentration of 56.5 or 565 µg/ml. The results are shown in Table 76. Each of the water-extracted fraction-1 and the water-extracted fraction-2 of soyameal enhanced HGF production of MRC-5 cells in a concentration-dependent manner.

**Table 76**

| Water-Extracted Fraction-1 of Soyameal | | | |
|---|---|---|---|
| Sample Concentration (µg/ml) | 0 | 105 | 1050 |
| Amount of HGF Produced (%) | 100 | 142 | 131 |

| Water-Extracted Fraction-2 of Soyameal | | | |
|---|---|---|---|
| Sample Concentration (µg/ml) | 0 | 56.5 | 565 |
| Amount of HGF Produced (%) | 100 | 127 | 171 |
| (Here, the amount of HGF produced in the control was 14.47 ng/ml.) | | | |

### Example 40

(1) Five grams of a powder of mulukhiya leaves (manufactured by Kokan Yakuhin Kenkyusho) was extracted with 200 ml of chloroform at room temperature. This extraction procedure was repeated twice against the residue after the suction filtration. The residue after the extraction with chloroform was extracted with 200 ml of ethanol at room temperature. This procedure was repeated twice against the residue after the suction filtration. A liquid portion after the filtration was collected and concentrated to dryness with a rotary evaporator, and the dried product was dissolved in 17 ml of ethanol. A part dissolved in ethanol was collected and concentrated to dryness with a rotary evaporator, to give an ethanol-extracted fraction-1. In addition, the part not dissolved in ethanol but dissolved in distilled water was collected and concentrated to dryness with a rotary evaporator, to give an ethanol-extracted fraction-2. Next, the residue after the extraction with ethanol was extracted with 80 ml of distilled water at 60°C for 2 hours. The 2.5-fold amount of ethanol was added to a liquid portion resulting from removal of the residue by centrifugation at 10000 G, and the mixture was allowed to stand at -20°C for 1 hour. Thereafter, the mixture was centrifuged at 10000 G to fractionate the mixture into precipitates and a liquid portion. The liquid portion was concentrated to dryness with a rotary evaporator, to give a water-extracted fraction-1. On the other hand, the precipitates were re-dissolved in distilled water, and the solution was dialyzed against distilled water (dialysis membrane: Seamless Cellulose Tubing UC36-32-100, manufactured by Sanko Junyaku). The 2.5-fold amount of ethanol was added to a solution inside the dialysis membrane, and the mixture was allowed to stand at -20°C for 1 hour. Thereafter, the mixture was centrifuged at 10000 G to fractionate the mixture into precipitates and a liquid portion. The precipitates were dissolved in distilled water, to give a water-extracted fraction-2.
(2) The enhancing activity for NGF production of each of the ethanol-extracted fraction-2, the water-extracted fraction-1 and the water-extracted fraction-2 of mulukhiya leaves mentioned above was assayed in the same manner as in Example 13. The ethanol-extracted fraction-2 of mulukhiya leaves was added to the medium so as to have a concentration of 0.15 or 0.3 mg/ml. The water-extracted fraction-1 of mulukhiya leaves was added to the medium so as to have a concentration of 1.41 mg/ml. The water-extracted fraction-2 of mulukhiya leaves was added to the medium so as to have a concentration of 0.01, 0.02 or 0.04 mg/ml. The results are shown in Table 77. Each of the ethanol-extracted fraction-2, the water-extracted fraction-1 and the water-extracted fraction-2 of mulukhiya leaves enhanced NGF production of L-M cells in a concentration-dependent manner.

**Table 77**

| Ethanol-Extracted Fraction-2 | | | | |
|---|---|---|---|---|
| Sample Concentration (mg/ml) | 0 | 0.15 | 0.3 | |
| Amount of NGF Produced (%) | 100 | 256.1 | 295.7 | |

| Water-Extracted Fraction-1 | | | | |
|---|---|---|---|---|
| Sample Concentration (mg/ml) | 0 | 1.41 | | |
| Amount of NGF Produced (%) | 100 | 843.4 | | |

| Water-Extracted Fraction-2 | | | | |
|---|---|---|---|---|
| Sample Concentration (mg/ml) | 0 | 0.01 | 0.02 | 0.04 |
| Amount of NGF Produced (%) | 100 | 250.5 | 300.2 | 372.6 |
| (Here, the amount of NGF produced in the control was 0.122 ng/ml.) | | | | |

In addition, the enhancing activity for HGF production of each of the water-extracted fraction-1 and the water-extracted fraction-2 of mulukhiya leaves was assayed in the same manner as in item (1) of Example 4. The water-extracted fraction-1 of mulukhiya leaves was added to the medium so as to have a concentration of 225.5, 22.55 or 2.255 µg/ml. The water-extracted fraction-2 of mulukhiya leaves was added to the medium so as to have a concentration of 20, 8, 4, 0.4 or 0.04 µg/ml. The results are shown in Tables 78 and 79. Each of the water-extracted fraction-1 and the water-extracted fraction-2 of mulukhiya leaves enhanced HGF production of MRC-5 cells in a concentration-dependent manner.

**Table 78**

| Water-Extracted Fraction-1 of Mulukhiya Leaves | | | | |
|---|---|---|---|---|
| Sample Concentration (µg/ml) | 0 | 2.255 | 22.55 | 225.5 |
| Amount of HGF Produced (%) | 100 | 232 | 406 | 612 |
| (Here, the amount of HGF produced in the control was 5.96 ng/ml.) | | | | |

**Table 79**

| Water-Extracted Fraction-2 of Mulukhiya Leaves | | | | | | |
|---|---|---|---|---|---|---|
| Sample Concentration (µg/ml) | 0 | 0.04 | 0.4 | 4 | 8 | 20 |
| Amount of HGF Produced (%) | 100 | 117 | 129 | 188 | 266 | 449 |
| (Here, the amount of HGF produced in the control was 9.56 ng/ml.) | | | | | | |

### Example 41

(1) Ten grams of rice bran was extracted with 200 ml of chloroform at room temperature. This extraction procedure was repeated twice against the residue after the suction filtration. A liquid portion after the filtration was collected and concentrated to dryness with a rotary evaporator, to give a chloroform-extracted fraction. The residue after the extraction with chloroform was extracted with 200 ml of ethanol at room temperature. This procedure was repeated twice against the residue. A liquid portion after the filtration was collected and concentrated to dryness with a rotary evaporator, and the dried product was dissolved in 35 ml of ethanol. A part dissolved in ethanol was collected and concentrated to dryness with a rotary evaporator, to give an ethanol-extracted fraction-1. In addition, the part not dissolved in ethanol but dissolved in distilled water was collected and concentrated to dryness with a rotary evaporator, to give an ethanol-extracted fraction-2. Next, the residue after the extraction with ethanol was extracted with 100 ml of distilled water at 60°C for 2 hours. The 2.5-fold amount of ethanol was added to a liquid portion resulting from removal of the residue by centrifugation at 10000 G, and the mixture was allowed to stand at -20°C for 1 hour. Thereafter, the mixture was centrifuged at 10000 G to fractionate the mixture into precipitates and a liquid portion. The liquid portion was concentrated to dryness with a rotary evaporator, to give a water-extracted fraction-1. On the other hand, the precipitates were dissolved in distilled water, to give a water-extracted fraction-2.
(2) The enhancing activity for NGF production of each of the chloroform-extracted fraction, the ethanol-extracted fraction-1, the ethanol-extracted fraction-2, the water-extracted fraction-1 and the water-extracted fraction-2 of rice bran prepared above was assayed in the same manner as in Example 13. The chloroform-extracted fraction of rice bran was added to the medium so as to have a concentration of 4.4 or 8.8 mg/ml. The ethanol-extracted fraction-1 of rice bran was added to the medium so as to have a concentration of 0.125, 0.25, 0.5 or 1.0 mg/ml. The ethanol-extracted fraction-2 of rice bran was added to the medium so as to have a concentration of 0.625, 1.25, 2.5 or 5.0 mg/ml. The water-extracted fraction-1 of rice bran was added to the medium so as to have a concentration of 0.625, 1.25, 2.5 or 5.0 mg/ml. The water-extracted fraction-2 of rice bran was added to the medium so as to have a concentration of 0.625, 1.25, 2.5 or 5.0 mg/ml. The results are shown in Table 80. Each of the chloroform-extracted fraction, the ethanol-extracted fraction-1, the ethanol-extracted fraction-2, the water-extracted fraction-1 and the water-extracted fraction-2 of rice bran enhanced NGF production of L-M cells in a concentration-dependent manner.

**Table 80**

| Chloroform-Extracted Fraction | | | | | |
|---|---|---|---|---|---|
| Sample Concentration (mg/ml) | 0 | 4.4 | 8.8 | | |
| Amount of NGF Produced (%) | 100 | 125.8 | 181.8 | | |

| Ethanol-Extracted Fraction-1 | | | | | |
|---|---|---|---|---|---|
| Sample Concentration (mg/ml) | 0 | 0.125 | 0.25 | 0.5 | 1.0 |
| Amount of NGF Produced (%) | 100 | 239.6 | 318.6 | 357.3 | 373.0 |

| Ethanol-Extracted Fraction-2 | | | | | |
|---|---|---|---|---|---|
| Sample Concentration (mg/ml) | 0 | 0.625 | 1.25 | 2.5 | 5.0 |
| Amount of NGF Produced (%) | 100 | 313.0 | 393.7 | 445.9 | 685.8 |

| Water-Extracted Fraction-1 | | | | | |
|---|---|---|---|---|---|
| Sample Concentration (mg/ml) | 0 0 | 0.625 | 1.25 1.25 | 2.5 2.5 | 5.0 5.0 |
| Amount of NGF Produced (%) | 100 | 151.0 | 177.4 | 291.1 | 559.1 |

| Water-Extracted Fraction-2 | | | | | |
|---|---|---|---|---|---|
| Sample Concentration (mg/ml) | 0 | 0.625 | 1.25 | 2.5 | 5.0 |
| Amount of NGF Produced (%) | 100 | 138.1 | 185.8 | 248.0 | 428.5 |
| (Here, the amount of NGF produced in the control was 0.172 ng/ml.) | | | | | |

In addition, the enhancing activity for HGF production of the water-extracted fraction-1 was assayed in the same manner as in item (1) of Example 4. The water-extracted fraction-1 of rice bran was added to the medium so as to have a concentration of 1000 or 100 µg/ml. The results are shown in Table 81. The water-extracted fraction-1 of rice bran enhanced HGF production of MRC-5 cells in a concentration-dependent manner.

**Table 81**

| Water-Extracted Fraction | | | |
|---|---|---|---|
| Sample Concentration (µg/ml) | 0 | 100 | 1000 |
| Amount of HGF Produced (%) | 100 | 107 | 157 |
| (Here, the amount of HGF produced in the control was 4.04 ng/ml.) | | | |

### Example 42

(1) Commercially available oolong tea leaves were extracted in a 2-fold concentration of ordinary tea to give a 500 g extract. The extract was formulated into the composition as shown in Table 82, to give a 1000 ml alcohol-containing oolong tea.

**Table 82**

| | |
|---|---|
| Oolong Tea | 50% |
| 1/5 Lemon fruit juice | 0.1% |
| Citric acid or sodium citrate | as appropriate |
| Vitamin C | 0.02% |
| Sugar (canc) | 4.5% |
| Ethyl alcohol (v/v) | 4.0% |
| pH | 3.7 |
| Purified Water | Balance |

(2) Seven grams of powder of bamboo blade leaves was suspended in 100 ml of purified water, and extracted at 60°C for 1 hour, and the extract was lyophilized, to give a lyophilized product. An alcoholic beverage was prepared by adding the lyophilized product to the above-mentioned alcohol-containing oolong tea so as to have a concentration of the lyophilized product of 0.1 µg/ml. An alcoholic beverage was prepared by adding the lyophilized product to the above-mentioned alcohol-containing oolong tea so as to have a concentration of isoorientin contained in the lyophilized product of 100 µg/ml.
(3) Five grams of onionskin was suspended in 100 ml of purified water, and extracted at 100°C for 15 minutes, to give an extract. An alcoholic beverage was prepared by adding the extract to the above-mentioned alcohol-containing oolong tea so that the extract is made into a 100-fold dilution.
(4) Three grams of biloba tea leaves were suspended in 200 ml of purified water, and extracted at 100°C for 15 minutes, and the extract was lyophilized, to give a lyophilized product. An alcoholic beverage was prepared by dissolving the lyophilized product in 8 ml of purified water, and thereafter adding the solution to the above-mentioned alcohol-containing oolong tea so that the solution is made into a 100-fold dilution.
(5) Ten grams of a dry product of *Artemisia princeps pampan* was extracted with 150 ml of purified water at 60°C for 1 hour. An alcohol-containing beverage was prepared by adding the extract to the above-mentioned alcohol-containing oolong tea so as to have a concentration of 3,5-dicaffeoyl-quinic acid derived from the extract of 5 µg/ml. In addition, an alcohol-containing beverage was prepared by adding the extract to the above-mentioned alcohol-containing oolong tea so as to have a concentration of chlorogenic acid derived from the extract of 40 µg/ml.
(6) Four-hundred-and-eighty grams of leaf-and-stem portions of dry *Angelica keiskei* koidz. were suspended in 2 liters of purified water, and extracted at 60°C for 1 hour, to give an extract. An alcohol-containing beverage was prepared by adding the extract to the above-mentioned alcohol-containing oolong tea so as to have a concentration of chlorogenic acid contained in the extract of 10 µg/ml.
(7) Fifteen grams of dried flowers of Chrysanthemum were suspended in 150 ml of purified water, and extracted at 60°C for 2 hours. An alcohol-containing beverage was prepared by adding the extract to the above-mentioned alcohol-containing oolong tea so that the extract is made into a 100-fold dilution.
(8) Five grams of a dry product of *Curcuma zedoaeia Roscoe* was suspended in 25 ml of purified water, and extracted at 60°C for 2 hours, and the extract was lyophilized, to give a lyophilized product. An alcohol-containing beverage was prepared by adding the lyophilized product to the above-mentioned alcohol-containing oolong tea so as to have a concentration of the lyophilized product of 10 µg/ml.
(9) Three-hundred grams of plum was homogenized together with 500 ml of ethanol, and the homogenate was filtered, to give a filtrate. An alcohol-containing beverage was prepared by adding the filtrate to the above-mentioned alcohol-containing oolong tea so that the filtrate is a 100-fold dilution. Using the filtrate, alcohol-containing beverages each having a concentration of 5-caffeoyl-quinic acid and 4-caffeoyl-quinic acid of 100 µg/ml were prepared.
(10) Three-hundred grams of broccoli was homogenized together with 350 ml of a 50%-aqueous ethanol solution, and the homogenate was filtered, to obtain its filtrate, and the filtrate was concentrated to a volume of 150 ml. An alcohol-containing beverage was prepared by adding the concentrate to the above-mentioned alcohol-containing oolong tea so that the concentrate is made into a 1000-fold dilution.
(11) Ten grams of a lyophilized product of *Chrysanthemum coronarium* was powdered, and the powder was washed with ethanol. Thereafter, the powder was extracted with 100 ml of water, with heating the mixture at 60°C for 1 hour to give an extract. An alcohol-containing beverage was prepared by adding the extract to the above-mentioned alcohol-containing oolong tea so that the concentrate is made into a 1000-fold dilution.
(12) Nine-hundred grams of *Chrysanthemum coronarium* was homogenized together with 2 liters of an 80%-aqueous ethanol solution, and the homogenate was filtered, to give a filtrate, and the filtrate was concentrated to a volume of 50 ml. An alcohol-containing beverage was prepared by adding the concentrate to the above-mentioned alcohol-containing oolong tea so that the concentrate is made into a 1000-fold dilution. In addition, another alcohol-containing beverage was prepared using the concentrate so as to have a concentration of 3,5-dicaffeoyl-quinic acid of 10 µg/ml.
(13) Fifteen liters of a commercially available beer (previously mentioned Marketed Product B) was concentrated to a volume of 6 liters. An alcohol-containing beverage was prepared by adding the concentrate to the above-mentioned alcohol-containing oolong tea so that the concentrate is made into a 100-fold dilution.
(14) Fifteen liters of a commercially available nonalcoholic beverage (previously mentioned Marketed Product D) was concentrated to a volume of 6 liters. An alcohol-containing beverage was prepared by adding the concentrate to the above-mentioned alcohol-containing oolong tea so that the concentrate is made into a 100-fold dilution.
(15) Ten grams of thinly cut pieces of soybean embryo were washed with 200 ml of ethanol, and thereafter extracted with 200 ml of water, with heating at 60°C for 2 hours, to give an extract. The extract was filtered, to obtain its filtrate, and thereafter the 2.5-fold amount of ethanol was added to the filtrate, and a dry product of each of an ethanol-soluble fraction and an ethanol-insoluble fraction was prepared. An alcohol-containing beverage was prepared by adding the dry product of the ethanol-soluble fraction to the above-mentioned alcohol-containing oolong tea so as to have a concentration of the dry product of the ethanol-soluble fraction of 200 µg/ml.
In addition, an alcohol-containing beverage was prepared by adding the dry product of the ethanol-insoluble fraction to the above-mentioned alcohol-containing oolong tea so as to have a concentration of the dry product of the ethanol-insoluble fraction of 130 µg/ml.
(16) Ten grams of thinly cut pieces of seed coat of soybean were washed with 200 ml of ethanol, and thereafter extracted with 70 ml of water, i with heating at 60°C for 2 hours, to give an extract. The extract was filtered, to obtain its filtrate, and the 2.5-fold amount of ethanol was added to the filtrate, and a dry product of each of an ethanol-soluble fraction and an ethanol-insoluble fraction was prepared. An alcohol-containing beverage was prepared by adding the dry product of the ethanol-soluble fraction to the above-mentioned alcohol-containing oolong tea so as to have a concentration of the dry product of the ethanol-soluble fraction of 130 µg/ml.
In addition, an alcohol-containing beverage was prepared by adding the dry product of the ethanol-insoluble fraction to the above-mentioned alcohol-containing oolong tea so as to have a concentration of the dry product of the ethanol-insoluble fraction of 30 µg/ml.
(17) Ten grams of thinly cut pieces of soyameal were washed with 200 ml of ethanol, and thereafter extracted with 100 ml of water, with heating at 60°C for 2 hours, to give an extract. The extract was filtered, to obtain its filtrate, and the 2.5-fold amount of ethanol was added to the filtrate, and a dry product of each of an ethanol-soluble fraction and an ethanol-insoluble fraction was prepared. An alcohol-containing beverage was prepared by adding the dry product of the ethanol-soluble fraction to the above-mentioned alcohol-containing oolong tea so as to have a concentration of the dry product of the ethanol-soluble fraction of 100 µg/ml.
In addition, an alcohol-containing beverage was prepared by adding the dry product of the ethanol-insoluble fraction to the above-mentioned alcohol-containing oolong tea so as to have a concentration of the dry product of the ethanol-insoluble fraction of 60 µg/ml.
(18) Five grams of a powder of mulukhiya leaves was washed with 200 ml of ethanol, and thereafter extracted with 80 ml of water, with heating at 60°C for 2 hours, to give an extract. The extract was filtered, to obtain its filtrate, and the 2.5-fold amount of ethanol was added to the filtrate, and a dry product of each of an ethanol-soluble fraction and an ethanol-insoluble fraction was prepared. An alcohol-containing beverage was prepared by adding the dry product of the ethanol-soluble fraction to the above-mentioned alcohol-containing oolong tea so as to have a concentration of the dry product of the ethanol-soluble fraction of 2 µg/ml.
In addition, an alcohol-containing beverage was prepared by adding the dry product of the ethanol-insoluble fraction to the above-mentioned alcohol-containing oolong tea so as to have a concentration of the dry product of the ethanol-insoluble fraction of 4 µg/ml.
(19) Ten grams of rice bran was washed with 200 ml of ethanol, and thereafter extracted with 100 ml of water, with heating at 60°C for 2 hours, to give an extract. The extract was filtered, to obtain its filtrate, and the 2.5-fold amount of ethanol was added to the filtrate, and a dry product of an ethanol-soluble fraction was prepared. An alcohol-containing beverage was prepared by adding the dry product of the ethanol-soluble fraction to the above-mentioned alcohol-containing oolong tea so as to have a concentration of the dry product of the ethanol-soluble fraction of 1 mg/ml.
(20) Each of the alcohol-containing beverages of items (2) to (19) of Example 42 described above was a refreshing alcoholic beverage having good flavor and enhancing ability for HGF production.

Also, each of these beverages was carbonated, to give a carbonated beverage.

In addition, nonalcoholic beverages comprising an effective ingredient having enhancing ability for HGF production in a 20-fold amount of these beverages were prepared, respectively. Each of these beverages had good flavor and high enhancing ability for HGF production.

### Example 43

(1) An alcohol-containing fruit juice was prepared in accordance with the composition as shown in Table 83.

**Table 83**

| | |
|---|---|
| 1/6 Grapefruit fruit juice | 0.56% |
| Citric acid or sodium citrate | as appropriate |
| Vitamin C | 0.02% |
| Liquid saccharide of fructose and glucose | 1.5% |
| Syrup | 2.2% |
| Ethyl alcohol (v/v) | 3.0% |
| pH | 3.3 |
| Purified Water | Balance |

(2) An alcohol-containing beverage was prepared by adding a commercially available yellow pigment from *Carthamus tinctorius* to the above-mentioned alcohol-containing fruit juice so as to have a concentration of the pigment of 1.25 mg/ml. In the beverage, safflomin A was contained as its effective ingredient in a high concentration. In addition, 10 g of a dry product of *Carthamus tinctorius* was suspended in 150 ml of purified water and extracted therewith at 60°C for 2 hours, and the extract was lyophilized. An alcohol-containing beverage was prepared by adding the lyophilized product to the above-mentioned alcohol-containing fruit juice so as to have a concentration of safflomin A contained in the lyophilized product of 3 mg/ml.
(3) Fifteen grams of commercially available dried flowers of Chrysanthemum were suspended in 150 ml of purified water, and extracted therewith at 60°C for 2 hours, and the extract was lyophilized, to give a lyophilized product. An alcohol-containing beverage was prepared by adding the lyophilized product to the above-mentioned alcohol-containing fruit juice so as to have a concentration of the lyophilized product of 20 mg/ml.
In addition, an alcohol-containing beverage was prepared by adding the above-mentioned lyophilized product to the above-mentioned alcohol-containing fruit juice so as to have a concentration of the guaianolide of 20 µg/ml.
(4) Twenty grams of leaf-and-stem portions of dry *Angelica keiskei* koidz. were suspended in 360 ml of purified water, and extracted at 60°C for 2 hours, and the extract was lyophilized, to give a lyophilized product. An alcohol-containing beverage was prepared by adding the lyophilized product to the above-mentioned alcohol-containing fruit juice so as to have a concentration of the lyophilized product of 400 µg/ml.
In addition, 20 g of root portions of dry *Angelica keiskei* koidz. were suspended in 360 ml of purified water, and extracted at 60°C for 2 hours, and the extract was lyophilized. An alcohol-containing beverage was prepared by adding the lyophilized product to the above-mentioned alcohol-containing fruit juice so as to have a concentration of the lyophilized product of 600 µg/ml.
Further, an alcohol-containing beverage was prepared by adding the lyophilized product to the above-mentioned alcohol-containing fruit juice so as to have a concentration of xanthoangelol of 10 µg/ml.
Moreover, an alcohol-containing beverage was prepared by adding the lyophilized product to the above-mentioned alcohol-containing fruit juice so as to have a concentration of 3'-O-β-D-glucopyranoyl khellactone of 50 µg/ml.
Also, an alcohol-containing beverage was prepared by adding the lyophilized product to the above-mentioned alcohol-containing fruit juice so as to have a concentration of 7-O-β-D-glucopyranosyloxy-8-prenylcoumarin of 30 µg/ml.
Also, an alcohol-containing beverage was prepared by adding the lyophilized product to the above-mentioned alcohol-containing fruit juice so as to have a concentration of caffeic acid methyl ester of 20 µg/ml.
Also, an alcohol-containing beverage was prepared by adding the lyophilized product to the above-mentioned alcohol-containing fruit juice so as to have a concentration of 8-carboxyl-3-hydroxy-5-methoxyl-2-dimethylchroman of 3 µg/ml.
Also, an alcohol-containing beverage was prepared by adding the lyophilized product to the above-mentioned alcohol-containing fruit juice so as to have a concentration of 7-β-D-glucopyranosyloxy-6-prenylcoumarin of 300 µg/ml.
Also, an alcohol-containing beverage was prepared by adding the lyophilized product to the above-mentioned alcohol-containing fruit juice so as to have a concentration of 4'-O-angeloyl-3'-O-[6-O-(β-D-glucopyranosyl)-β-D-glucopyranosyl]-khellactone of 400 µg/ml.
Also, an alcohol-containing beverage was prepared by adding the lyophilized product to the above-mentioned alcohol-containing fruit juice so as to have a concentration of caffeic acid ethyl ester of 30 µg/ml.
(5) Twenty-five grams of onionskin was suspended in 500 ml of 95% v/v ethyl alcohol, and extracted therewith at room temperature, and the extract was dried. An alcohol-containing beverage was prepared by adding the dried product to the above-mentioned alcohol-containing fruit juice so as to have a concentration of the dried product of 100 µg/ml.
(6) Three grams of biloba tea leaves were suspended in 200 ml of purified water, and extracted therewith at 100°C for 1 hour, and the extract was lyophilized, to give a lyophilized product. An alcohol-containing beverage was prepared by adding the lyophilized product to the above-mentioned alcohol-containing fruit juice so as to have a concentration of the lyophilized product of 700 µg/ml.
(7) Two grams of commercially available rose flowers were suspended in 40 ml of purified water, and extracted therewith at 60°C for 1 hour, and the extract was lyophilized, to give a lyophilized product. An alcohol-containing beverage was prepared by adding the lyophilized product to the above-mentioned alcohol-containing fruit juice so as to have a concentration of the lyophilized product of 80 µg/ml.
(8) An alcohol-containing beverage was prepared by adding a commercially available extract from *Humulus lupulus* to the above-mentioned alcohol-containing fruit juice so that the content of xanthohumol is 3 µg/ml.
(9) An alcohol-containing beverage was prepared by adding a commercially available extract from *Humulus lupulus* to the above-mentioned alcohol-containing fruit juice so as to have a concentration of the extract of 10 µg/ml on a dry basis.
(10) An alcohol-containing beverage was prepared by adding a commercially available extract from *Humulus lupulus* to the above-mentioned alcohol-containing fruit juice so that a total amount of xanthohumol B and xanthohumol D is 20 µg/ml.
(11) An alcohol-containing beverage was prepared by adding a commercially available extract from *Humulus lupulus* to the above-mentioned alcohol-containing fruit juice so that the content of isoxanthohumol is 80 µg/ml.
(12) Fifteen liters of a commercially available beer (previously mentioned Marketed Product B) was concentrated to a volume of 6 liters. An alcohol-containing beverage was prepared by adding the concentrate to the above-mentioned alcohol-containing fruit juice so that the concentrate is made into a 10-fold dilution.
(13) Fifteen liters of a commercially available nonalcoholic beverage (previously mentioned Marketed Product D) was concentrated to a volume of 6 liters. An alcohol-containing beverage was prepared by adding the concentrate to the above-mentioned alcohol-containing fruit juice so that the concentrate is made into a 10-fold dilution.
(14) An alcohol-containing beverage was prepared by adding a commercially available extract from *Humulus lupulus* to the above-mentioned alcohol-containing fruit juice so that the content of xanthohumol is 0.04 µg/ml and the content of isoxanthohumol is 1.3 µg/ml.
In addition, an alcohol-containing beverage was prepared by adding a commercially available extract from *Humulus lupulus* to the above-mentioned alcohol-containing fruit juice so that the content of xanthohumol is 0.9 µg/ml and the content of isoxanthohumol is 4 µg/ml.
(15) Fifty grams of a dry product of *Humulus lupulus* was powdered, and the powder was extracted with 1 liter of ethanol, and the extract was concentrated. An alcohol-containing beverage was prepared by adding the concentrate to the above-mentioned alcohol-containing fruit juice so as to have a concentration of the concentrate of 20 µg/ml on a dry basis.
(16) Five grams of thinly cut pieces of *Curcuma zedoaeia Roscoe* were suspended in 25 ml of purified water, and extracted therewith at 60°C for 2 hours, and the extract was lyophilized, to give a lyophilized product. An alcohol-containing beverage was prepared by adding the lyophilized product to the above-mentioned alcohol-containing fruit juice so as to have a concentration of the lyophilized product of 600 µg/ml.
(17) Ten grams of thinly cut pieces of soybean embryo were washed with 200 ml of ethanol, and thereafter extracted with 200 ml of water, with heating at 60°C for 2 hours, to give an extract. The extract was filtered, to obtain its filtrate, and the 2.5-fold amount of ethanol was added to the filtrate, and a dry product of each of an ethanol-soluble fraction and an ethanol-insoluble fraction was prepared. An alcohol-containing beverage was prepared by adding the dry product of the ethanol-soluble fraction to the above-mentioned alcohol-containing fruit juice so as to have a concentration of the dry product of the ethanol-soluble fraction of 5 mg/ml.
In addition, an alcohol-containing beverage was prepared by adding the dry product of the ethanol-insoluble fraction to the above-mentioned alcohol-containing fruit juice so as to have a concentration of the dry product of the ethanol-insoluble fraction of 3 mg/ml.
(18) Ten grams of thinly cut pieces of seed coat of soybean were washed with 200 ml of ethanol, and thereafter extracted with 70 ml of water, with heating at 60°C for 2 hours, to give an extract. The extract was filtered, to obtain its filtrate, and the 2.5-fold amount of ethanol was added to the filtrate, and a dry product of each of an ethanol-soluble fraction and an ethanol-insoluble fraction was prepared. An alcohol-containing beverage was prepared by adding the dry product of the ethanol-soluble fraction to the above-mentioned alcohol-containing oolong tea so as to have a concentration of the dry product of the ethanol-soluble fraction of 3 mg/ml.
In addition, an alcohol-containing beverage was prepared by adding the dry product of the ethanol-insoluble fraction to the above-mentioned alcohol-containing fruit juice so as to have a concentration of the dry product of the ethanol-insoluble fraction of 600 µg/ml.
(19) Ten grams of thinly cut pieces of soyameal were washed with 200 ml of ethanol, and thereafter extracted with 100 ml of water, with heating at 60°C for 2 hours, to give an extract. The extract was filtered, to obtain its filtrate, and the 2.5-fold amount of ethanol was added to the filtrate, and a dry product of each of an ethanol-soluble fraction and an ethanol-insoluble fraction was prepared. An alcohol-containing beverage was prepared by adding the dry product of the ethanol-soluble fraction to the above-mentioned alcohol-containing fruit juice so as to have a concentration of the dry product of the ethanol-soluble fraction of 3 mg/ml.
In addition, an alcohol-containing beverage was prepared by adding the dry product of the ethanol-insoluble fraction to the above-mentioned alcohol-containing oolong tea so as to have a concentration of the dry product of the ethanol-insoluble fraction of 60 µg/ml.
(20) Five grams of a powder of mulukhiya leaves was washed with 200 ml of ethanol, and thereafter extracted with 80 ml of water, with heating at 60°C for 2 hours, to give an extract. The extract was filtered, to obtain its filtrate, and the 2.5-fold amount of ethanol was added to the filtrate, and a dry product of each of an ethanol-soluble fraction and an ethanol-insoluble fraction was prepared. An alcohol-containing beverage was prepared by adding the dry product of the ethanol-soluble fraction to the above-mentioned alcohol-containing fruit juice so as to have a concentration of the dry product of the ethanol-soluble fraction of 500 µg/ml.
In addition, an alcohol-containing beverage was prepared by adding the dry product of the ethanol-insoluble fraction to the above-mentioned alcohol-containing fruit juice so as to have a concentration of the dry product of the ethanol-insoluble fraction of 5 µg/ml.
(21) Ten grams of a powder of rice bran was washed with 200 ml of ethanol, and thereafter extracted with 100 ml of water, with heating at 60°C for 2 hours, to give an extract. The extract was filtered, to obtain its filtrate, and the 2.5-fold amount of ethanol was added to the filtrate, and a dry product of each of an ethanol-soluble fraction and an ethanol-insoluble fraction was prepared. An alcohol-containing beverage was prepared by adding the dry product of the ethanol-soluble fraction to the above-mentioned alcohol-containing fruit juice so as to have a concentration of the dry product of the ethanol-soluble fraction of 500 µg/ml. In addition, an alcohol-containing beverage was prepared by adding the dry product of the ethanol-insoluble fraction to the above-mentioned alcohol-containing fruit juice so as to have a concentration of the dry product of the ethanol-insoluble fraction of 500 µg/ml.
(22) Each of the alcohol-containing fruit juice beverages of items (2) to (21) of Example 43 described above was a refreshing alcoholic beverage having good flavor and enhancing ability for NGF production.

Also, each of these beverages was carbonated, to give a foamy beverage.

In addition, nonalcoholic beverages containing an effective ingredient having enhancing ability for NGF production in a 20-fold amount of these beverages were prepared, respectively. Each of these beverages had good flavor and high enhancing ability for NGF production.

### INDUSTRIAL APPLICABILITY

According to the present invention, there are provided a composition for enhancing production of a growth factor, comprising a plant-derived substance enhancing growth factor production, and a medicament, food, beverage or feed effective for a disease that requires enhancement of growth factor production, comprising the composition. The medicament has enhancing activity for growth factor production in a living body, and is useful as a therapeutic agent or prophylactic agent for a disease that requires enhancement for growth factor production, such as hepatic disorders including hepatitis, severe hepatitis, fulminant hepatitis, cirrhosis, cholestasia in the liver, kidney diseases caused by drugs and the like, gastrointestinal disorders, blood vessel disorders, chronic nephritis, pneumonia, wound, diabetes, cancers, dementia, or nerve disorders. In addition, by the daily intake as foodstuff, the food or beverage can ameliorate symptoms of a disease that require enhancement of growth factor production. Therefore, a functional foodstuff comprising as an effective ingredient a plant-derived substance having enhancing action for growth factor production is useful for sustaining homeostasis of a living body by the enhancing action for growth factor production. The feed has an effect of ameliorating body conditions and the like and is useful. Further, the present invention provides a plant-derived enhancer for growth factor production, and the enhancer is useful for functional studies for growth factors and screening for a medicament for a disease associated with the growth factor.

## Claims

1. A composition for enhancing growth factor production, **characterized in that** the composition comprises as an effective ingredient a plant-derived enhancer for growth factor production.

2. The composition according to claim 1, wherein the composition comprises as an effective ingredient one or more plant-derived enhancers for growth factor production, the plant being selected from the group consisting of plants belonging to Umbelliferae, Compositae, Liliaceae, Ginkgoaceae, Gramineae, Rosaceae, Moraceae, Leguminosae, Tiliaceae, Cruciferae and Zingiberaceae.

3. The composition according to claim 1 or 2, wherein the plant-derived enhancer for growth factor production is one or more compounds selected from the group consisting of chlorogenic acid, dicaffeoyl-quinic acid, caffeoyl-quinic acid, isoorientin, safflomin A, guaianolide, xanthoangelol, 3'-O-β-D-glucopyranoyl khellactone, 7-O-β-D-glucopyranosyloxy-8-prenylcoumarin, caffeic acid methyl ester, caffeic acid ethyl ester, 8-carboxyl-3-hydroxy-5-methoxyl-2-dimethylchroman, 7-β-D-glucopyranosyloxy-6-prenylcoumarin, 4'-O-angeloyl-3'-O-[6-O-(β-D-glucopyranosyl)-β-D-glucopyranosyl]-khellactone, isoxanthohumol, xanthohumol B, xanthohumol D, and xanthohumol.

4. The composition according to any one of claims 1 to 3, wherein the composition comprises a plant-derived extract or a fraction obtained from the plant-derived extract, comprising a plant-derived enhancer for growth factor production.

5. The composition according to any one of claims 1 to 4, wherein the composition comprises the plant-derived enhancer for growth factor production in a high concentration and/or at a high purity.

6. The composition according to any one of claims 1 to 5, wherein the growth factor is a hepatocyte growth factor or a nerve growth factor.

7. A therapeutic agent or prophylactic agent for a disease requiring enhancement of growth factor production, comprising the composition of any one of claims 1 to 6.

8. A food, beverage or feed for enhancing growth factor production, comprising the composition of any one of claims 1 to 6.

9. A food, beverage or feed, **characterized in that** the food, beverage or feed comprises a plant-derived enhancer for growth factor production in a high concentration and/or at a high purity.

10. The food, beverage or feed according to claim 9, wherein the plant-derived enhancer for growth factor production is one or more compounds selected from the group consisting of chlorogenic acid, dicaffeoyl-quinic acid, caffeoyl-quinic acid, isoorientin, safflomin A, guaianolide, xanthoangelol, 3'-O-β-D-glucopyranoyl khellactone, 7-O-β-D-glucopyranosyloxy-8-prenylcoumarin, caffeic acid methyl ester, caffeic acid ethyl ester, 8-carboxyl-3-hydroxy-5-methoxyl-2-dimethylchroman, 7-β-D-glucopyranosyloxy-6-prenylcoumarin, 4'-O-angeloyl-3'-O-[6-O-(β-D-glucopyranosyl)-β-D-glucopyranosyl]-khellactone, isoxanthohumol, xanthohumol B, xanthohumol D, and xanthohumol.

11. A food, beverage or feed, comprising the composition of any one of claims 1 to 6 in a high concentration and/or at a high purity.

12. The food, beverage or feed according to claim 11, wherein the composition is a composition comprising one or more compounds selected from the group consisting of chlorogenic acid, dicaffeoyl-quinic acid, caffeoyl-quinic acid, isoorientin, safflomin A, guaianolide, xanthoangelol, 3'-O-β-D-glucopyranoyl khellactone, 7-O-β-D-glucopyranosyloxy-8-prenylcoumarin, caffeic acid methyl ester, caffeic acid ethyl ester, 8-carboxyl-3-hydroxy-5-methoxyl-2-dimethylchroman, 7-β-D-glucopyranosyloxy-6-prenylcoumarin, 4'-O-angeloyl-3'-O-[6-O-(β-D-glucopyranosyl)-β-D-glucopyranosyl]-khellactone, isoxanthohumol, xanthohumol B, xanthohumol D, and xanthohumol.

13. A food, beverage or feed for enhancing nerve growth factor production, comprising xanthohumol in an amount of 0.00007% by weight or more.

14. A food, beverage or feed for enhancing growth factor production, comprising a food, beverage or feed comprising an enhancer for growth factor production, or a treated product thereof.

15. The food, beverage or feed according to claim 14, comprising a beer.

16. A food, beverage or feed for enhancing growth factor production, comprising an extract from *Humulus lupulus.*

17. A composition for enhancing growth factor production, **characterized in that** the composition comprises a guaianolide as an effective ingredient.

18. A therapeutic agent or prophylactic agent for a disease requiring enhancement of growth factor production, **characterized in that** the therapeutic agent or prophylactic agent comprises a guaianolide as an effective ingredient.

19. A food, beverage or feed for enhancing growth factor production, **characterized in that** the food, beverage or feed comprises a guaianolide as an effective ingredient.
